# EUROPEAN PATENT SPECIFICATION

(11) **EP 3 774 755 B1**
(45) Date of publication and mention of the grant of the patent: **18.05.2022**
(21) Application number: 19713804.3
(22) Date of filing: 29.03.2019
(51) Int. Cl.: C07D 307/94, C07D 491/048, C07D 405/12, C07D 209/96, C07D 405/08, A61P 31/00, A61P 25/00, A61P 17/06, A61P 35/00, A61K 31/343

(54) **SPIROCYCLIC COMPOUNDS AS MODULATORS OF INDOLEAMINE 2,3-DIOXYGENASE**
SPIROCYCLISCHE VERBINDUNGEN ALS MODULATOREN DER INDOLAMIN-2,3-DIOXYGENASE
COMPOSÉS SPIROCYCLIQUES UTILISÉS COMME MODULATEURS D'INDOLÉAMINE 2,3-DIOXYGÉNASE

(30) Priority: 29.03.2018 EP 18165090
(43) Date of publication of application: 17.02.2021
(73) Proprietor: Phenex Discovery Verwaltungs-GmbH, 69123 Heidelberg (DE)
(72) Inventor: KINZEL, Olaf, 69117 Heidelberg (DE); STEENECK, Christoph, 69120 Heidelberg (DE); ANDERHUB, Simon, 4125 Riehen (CH); HORNBERGER, Martin, 68526 Ladenburg (DE); PINTO, Sheena, 69115 Heidelberg (DE); MORSCHHÄUSER, Barbara, 55237 Bornheim (DE); HOFFMANN, Thomas, 67346 Speyer (DE)
(74) Representative: Grünecker Patent- und Rechtsanwälte PartG mbB
(86) International application number: PCT/EP2019/058017
(87) International publication number: WO 2019/185870

(56) References cited:
- WO-A1-2006/122150
- US-A1- 2008 146 624
- US-A1- 2016 022 619
- MICHAEL FICHTNER ET AL: "Discovery and evaluation of spirocyclic derivatives as antagonists of the neuropeptide Y5 receptor", BIOORGANIC & MEDICINAL CHEMISTRY LETTERS, vol. 22, no. 8, 6 March 2012 (2012-03-06), pages 2738-2743, XP055477264, AMSTERDAM, NL ISSN: 0960-894X, DOI: 10.1016/j.bmcl.2012.02.098
- HE SHUWEN ET AL: "Spiroindane based amides as potent and selective MC4R agonists for the treatment of obesity", BIOORGANIC & MEDICINAL CHEMISTRY LETTERS, vol. 20, no. 15, 15 June 2010 (2010-06-15) , pages 4399-4405, XP029121150, ISSN: 0960-894X, DOI: 10.1016/J.BMCL.2010.06.062

## Description

The present invention relates to novel compounds which act as modulators of indoleamine 2,3-dioxygenase (IDO1) and to the use of said compounds in the prophylaxis and/or treatment of diseases or conditions mediated by indoleamine 2,3-dioxygenase. The invention further relates to pharmaceutical compositions comprising the novel compounds.

Tryptophan is an essential amino acid and naturally serves as a building block for proteins. The majority of adult Tryptophan intake is not utilized for protein synthesis though, but channeled into two conversion pathways. The first pathway leading to the production of Serotonine degrades approximately 1% of ingested Tryptophan, whereas the majority of -90% of Tryptophan fuels the so called Kynurenine pathway (Le Floc'h et al.; Amino Acids. 2011; 41(5):1195-205).

The Kynurenine pathway of Tryptophan degradation is initialized by a specific set of enzymes, including Indoleamine 2,3-dioxygenase 1 (IDO1) and Tryptophan 2,3-dioxygenase (TDO2). The product of this reaction, N-Formylkynurenine is subsequently converted to Kynurenine, which can be further metabolized to such diverse products as Xanthurenic acid, Anthranilic acid or Nicotinamide to name a few (Stone, Darlington; Nat Rev Drug Discov. 2002; 1(8):609-20).

Under physiological conditions the expression of TDO is restricted to the liver (Bertazzo et al.; Biochim Biophys Acta. 2001; 15;1527(3):167-75) and the brain (Miller et al.; Neurobiol Dis. 2004 ;15(3):618-29). IDO1 in contrast is found in a variety of tissues such as lung, digestive tract, uterus and secondary lymphoid organs (Théate et al.; Cancer Immunol Res. 2015;3(2):161-72) and is readily (further) induced by pro inflammatory cytokines (Taylor, Feng; FASEB J. 1991;5(11):2516-22 1991).

Initially, IDO1 has been implicated in a protective role in fetal rejection. Mice, treated with the IDO1 inhibitor 1-Methyl-Tryptophan lost their allogeneic concepti in a T cell dependent manner (Munn et al.; Science. 1998;281(5380):1191-3).

It was then conceived that IDO1 creates an immunosuppressive environment by catabolizing Tryptophan, thereby locally depleting this amino acid and creating immune privilege sites. Tryptophan depletion is most likely sensed through the General Control Nonderepressable Kinase 2 (GCN2) and leads to activation of the integrated stress response of cells (Munn et al.; Immunity. 2005;22(5):633-42) with consecutive inhibition of T cell proliferation (Munn et al.; J Exp Med. 1999;189(9):1363-72). Additionally, a low Tryptophan environment also sensitizes activated T cells to apoptosis via Fas (Lee et al.; Immunology. 2002;107(4):452-60). More recently, the mechanism of how IDO1 can lead to immune suppression has been expanded, focusing on the catabolites of Tryptophan enzymatic conversion by IDO1, collectively called Kynurenines. It has been demonstrated that Kynurenine, 3-Hydroxykynurenine, 3-Hydroxyanthranilic acid and Quinolinic acid led to dose dependent inhibition of T cell proliferation (Terness et al.; J Exp Med. 2002;196(4):447-57). In part, this may be due to cell type specific apoptosis of Thymocytes in response to incubation with the aforementioned Tryptophan metabolites (Fallarino et al.; Cell Death Differ. 2002;9(10):1069-77).

The immune suppression observed concurrently with IDO1 expression is also associated with an increase in T cells displaying a regulatory phenotype (Treg). Treg cells are important to maintain immune homeostasis and induce immune tolerance to avoid inappropriate immune response as is the case in autoimmune disease (Sakaguchi et al.; Eur J Immunol. 2007;37 Suppl 1:S116-23). In mice, expression of the transcription factor FOXP3 is an important marker for regulatory T cells (Fontenot et al.; Nat Immunol. 2003;4(4):330-6) and co-cultivation of murine naive CD4+ T cells with IDO positive Dendritic cells led to a remarkable increase in FOXP3 expression of the CD4+ population. This polarization could be mimicked by incubation of naive CD4+ T cells in low Tryptophan medium supplemented with Kynurenines and was shown to be dependent on GCN2 (Fallarino et al.; Transpl Immunol. 2006;17(1):58-60). In humans, AML patients with elevated levels of IDO1 also displayed an increase in circulating Treg cells. Analogous to the situation in mice, human CD3+ cells were polarized towards a regulatory phenotype in an IDO1 dependent manner when co-cultivated with IDO1 positive cells derived from AML patients (Curti et al.; Blood. 2007;109(7):2871-7).

Several Kynurenines such as Kynurenine itself, 3-Hydroxykynurenine and Kynurenic acid also serve as ligands for the Aryl Hydrocarbon Receptor (AHR) albeit with differentially reported efficacies (DiNatale et al.; Toxicol Sci. 2010;115(1):89-97, Mezrich et al.; J Immunol. 2010;185(6):3190-8). This is of particular interest because firstly, the AHR has been implicated in the transcriptional regulation of IDO1 via a self-sustaining autocrine feed-forward loop with the AHR acting either directly on IDO1 transcription (Li et al.; J Immunol. 2016;197(3):962-70) or with IL-6 as mediator (Litzenburger et al.; Oncotarget. 2014;5(4):1038-51). Secondly, because the polarization of naive CD4+ T cells towards Treg cells by Kynurenines is dependent on the AHR (Kimura et al.; Proc Natl Acad Sci U S A. 2008 Jul 15;105(28):9721-6, Mezrich et al.; J Immunol. 2010;185(6):3190-8).

Whether the depletion of Tryptophan or the generation of Kynurenines or the combined action of both is the key in creating an immune suppressive environment needs to be further investigated. The net result though, is a key factor not only for immune homeostasis in healthy individuals but also for how tumors can escape immune surveillance.

The importance of IDO1 for cancer development is supported by several lines of evidence. IDO1 has been detected in most human tumors, such as prostate, pancreas, lung, ovarian, colorectal cancer, melanoma and leukemia (Uyttenhove et al.; Nat Med. 2003;9(10):1269-74; Hanagiri et al.; J Clin Cell Immunol 2014, 5:5, Okamoto et al.; Clin Cancer Res. 2005;11(16):6030-9; Ferdinande et al.; Br J Cancer. 2012;106(1):141-7, Brody et al.; Cell Cycle. 2009;8(12):1930-4, Chamuleau et al.; Haematologica. 2008;93(12):1894-8, Theáte et al.; Cancer Immunol Res. 2015;3(2):161-72). Interestingly, IDO1 positive cells were also often found in immune cells in the tumor stroma and adjoining tumor draining lymph nodes (Astigiano et al.; Neoplasia. 2005;7(4):390-6, Chen et al.; Breast Cancer Res. 2014;16(4):410, Polak et al.; Br J Cancer. 2007;96(12):1879-87, Theáte et al.; Cancer Immunol Res. 2015;3(2):161-72). A negative correlation of IDO1 expression either in tumor or in stromal cells with markers of disease progression has been observed in most of these cases.

Apart from these correlative analysis, elegant studies using mouse models underpinned the importance of IDO1 in tumor immune escape. When immunogenic mouse tumor cells lacking IDO1 were injected into immune competent mice, no tumor growth was observed. In contrast, if the cells constitutively expressed IDO1, tumors grew as expected. Pharmacologic inhibition of IDO1 in turn, resulted in a marked reduction of tumor outgrowth. As indicated above, this effect was dependent on the hosts' immune system, as immune compromised mice injected with the IDO1 positive and negative cell lines developed tumors to the same extent. Also, lower numbers of CD8+ T cells were found in mice injected with IDO1 positive cells in comparison to mice injected with IDO1 negative cells (Uyttenhove et al.; Nat Med. 2003;9(10):1269-74).

Although tumor derived IDO1 is a decisive factor for immune escape, research also investigated the role of IDO1 in immune cells. Munn et al. found a subset of plasmacytoid Dendritic cells in Tumor draining lymph nodes expressing IDO1. Although these cells comprised less than 1% of all lymph node cells they acted as potent and dominant suppressors of T cell proliferation (Munn et al.; J Clin Invest. 2004; 114(2): 280-290). The relative contribution of IDO1 from immune cells versus tumor derived IDO1 is still under debate. Koblish et al. observed that pharmacologic inhibition of IDO1 reduced tumor size, when IDO1 positive tumor cells were transplanted into immune competent IDO1 -/- mice (Koblish et al.; Mol Cancer Ther. 2010;9(2):489-98). In contrast, Banerjee et al. reported no effect on tumor size when using a syngeneic mouse tumor model in IDO1 negative mice and administration of an IDO1 inhibitor (Banerjee et al.; Oncogene. 2008;27(20):2851-7). Both studies though, were able to demonstrate the efficacy of IDO inhibitors in preclinical mouse models as single agents. Moreover, synergistic or additive effects were observed when IDO1 inhibitors where used in combination with chemotherapeutics, irradiation, tumor vaccines or immune checkpoint inhibitors (Muller et al.; Nat Med. 2005;11(3):312-9, Hou et al.; Cancer Res. 2007 Jan 15;67(2):792-801, Sharma et al.; Blood. 2009 Jun 11;113(24):6102-11, Spranger et al.; J Immunother Cancer. 2014;2:3).

The studies referenced herein did not report any potent toxicity of IDO1 inhibition and it is of interest to note that IDO knockout mice are viable and exhibit no major abnormal phenotype apart from defects in acquired tolerance (Mellor et al.; J Immunol. 2003;171(4):1652-5). Therefore it seems unlikely that IDO1 inhibition in humans will encounter profound dose limiting toxicities.

Apart from its relevance for tumor immune evasion, IDO1 is implicated in a plethora of other medical conditions.

Throughout HIV disease progression, an altered Th17/Treg balance has been observed, favoring the latter in later stages. Favre et al. were able to demonstrate a crucial role for the Kynurenine 3-Hydroxykynurenine in this process and it is therefore hypothesized that patients with HIV may benefit from IDO1 inhibition together with antiretroviral therapy (Favre et al.; Sci Transl Med. 2010 May 19;2(32):32ra36.).

IDO1 also seems to be involved in disorders of the central nervous system because its downstream products 3-Hydroxykynurenine and quinolinic acid act as neurotoxins (Okuda et al.; J Neurochem. 1998;70(1):299-307, Schwarcz et al.; Science. 1983;219(4582):316-8). Thereby, IDO1 is also implicated in the disease development of Huntington's disease, Amyotrophic lateral sclerosis, Alzheimer's disease, Parkinson's disease and Schizophrenia (Thevandavakkam et al.; CNS Neurol Disord Drug Targets. 2010;9(6):791-800; Chen et al.; Neurotox Res. 2010;18(2):132-42; Guillemin et al.; Neuropathol Appl Neurobiol. 2005;31(4):395-404; Lim et al.; Prog Neurobiol. 2016;pii: S0301-0082(15)30055-1, Kegel et al.; Int J Tryptophan Res. 2014; 7: 15-22).

IDO1 inhibitors may therefore be of high potential value for the treatment of HIV and CNS disorders and the reported preclinical data on efficacy against tumors either alone or in combination with other drugs validate the use of IDO1 inhibitors as a treatment option for antineoplastic therapies.

Compounds acting as IDO1 inhibitors are known in the art. WO 2006/122150 discloses compounds with a N-hydroxyamidino motif as potential modulators of IDO1. The efficacy of compounds having said motif is demonstrated e.g. in WO 2008/036642, WO 2008/036643, WO 2008/036652, WO 2008/036653 and WO 2008/051 78. US 2016/0022619 also discloses IDOl modulators.

The compounds detailed herein and compositions thereof as well as the methods described will serve to meet the future need for potent IDO1 inhibitors.

It is the object of the present invention to provide novel compounds which are suitable as potent IDO1 inhibitors.

Said object is solved by the compounds of formulae (1) and (2) 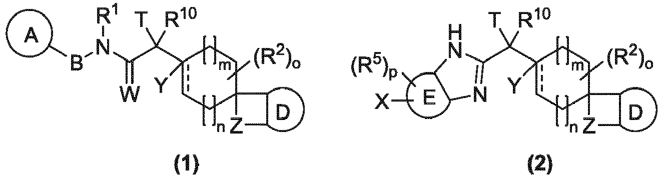 wherein A, B, D, E, T, W, X, Y, Z, R², R⁵, R¹⁰, m, n, o and p are defined as in the appended claims.

It is further an object of the present invention to provide compounds according to formulae (1) and (2) for use in the prophylaxis and/or treatment of diseases and conditions mediated by indoleamine 2,3-dioxygenase.

The present invention further relates to the use of the compounds according to formula (1) for the preparation of a medicament for the treatment and/or prophylaxis of a disease or condition mediated by indoleamine 2,3-dioxygenase.

Moreover, the present invention also relates to the compounds of the invention for use in a method for treating or preventing a disease or condition mediated by indoleamine 2,3-dioxygenase, the method comprising administering an effective amount of a compound according to formulae (1) and (2) to a patient in need thereof.

Accordingly, the present invention provides a compound represented by Formulae (1) or (2) 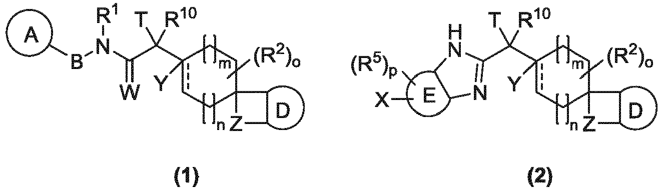 an enantiomer, diastereomer, tautomer or pharmaceutically acceptable salt thereof wherein

A represents C₃₋₁₀ cycloalkyl, which may be optionally fused with a phenyl ring being unsubstituted or substituted with 1 to 3 R^{a}, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo;
R^{a} represents halogen, CN, C₁₋₆-alkyl, halo-C₁₋₆-alkyl, hydroxy-C₁₋₆-alkyl, C₃₋₆-cycloalkyl or halo-C₃₋₆-cycloalkyl;
R^{x} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo C₁₋₆-alkyl, OR¹ and CN;
B represents a bond or C₁₋₂-alkylene, wherein alkylene is unsubstituted or substituted with one or two C₁₋₄-alkyl;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from the group consisting of halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl, C₃₋₆-cycloalkyl and halo C₁₋₆-alkyl, or
two substituents on the aryl or heteroaryl ring systems together with the carbon atoms to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
E represents a 6-membered aryl or 6-membered heteroaryl containing 1 to 2 nitrogen atoms;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl, or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
R¹⁰ represents hydrogen, halogen, C₁₋₆-alkyl, halo-C₁₋₆-alkyl; or
R¹⁰ and T form together a 3- to 10-membered mono- or bicyclic ring system which is saturated or partially unsaturated and wherein the ring system may further contain 1, 2 or 3 heteroatoms independently selected from N, O and S,
wherein the ring system is unsubstituted or substituted with 1 to 5 substituents independently selected from the group consisting of halogen, CN, OH, O-C₁₋₆-alkyl, oxo, C₁₋₆-alkyl and halo-C₁₋₆-alkyl;
W represents O, NOR⁴, NR¹, NCN, or NS(O)₂C₁₋₆-atkyt;
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
Y is absent or represents hydrogen, OR⁴, halogen, C₁₋₆-alkyl or halo-C₁₋₆-alkyl;
Z represents -C₂₋₃-alkylene-, -O-C₁₋₂-alkylene-, -C₁₋₂-alkylene-O-, -NR³C(O)-C₀₋₁-alkylene-, -C(O)NR³-C₀₋₁-alkylene-, -C₀₋₁-alkylene-NR³C(O)-, -C₀₋₁-alkylene C(O)NR³-, -S(O)ₜ-C₁₋₂-alkylene-, -C₁₋₂-alkylene-S(O)ₜ-, -NR⁹-C₁₋₂-alkylene- or -C₁₋₂-alkylene-NR⁹⁻, wherein alkylene is unsubstituted or substituted with 1 to 4 substituents independently selected from the group consisting of OR⁴, C₁₋₆-alkyl, halogen and halo-C₁₋₆-alkyl;
R⁹ is hydrogen, C₁₋₆-alkyl, halo-C₁₋₆-alkyl, C(O)-C₁₋₆-alkyl, or C(O)-halo-C₁₋₆-alkyl;
R¹ is hydrogen or C₁₋₆-alkyl;
R² is halogen, C₁₋₆-alkyl, C₃₋₆-cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, C(O)N(R¹)₂, CN, C(O)OR⁴ or oxo, or two R² on the same carbon atom form together with the carbon atom to which they are attached a C₃₋₁₀ cycloalkyl group, or
two R² at different carbon atoms form together a -CH₂-, -CH(CH₃)-, -C(CH₃)₂-, CH₂-CH(CH₃)-, -CH₂-CH₂- or -CH₂-CH₂-CH₂- group;
R³ is hydrogen or C₁₋₆-alkyl;
R⁴ is hydrogen or C₁₋₆-alkyl;
m is 0, 1 or 2;
n is 0, 1 or 2;
o is 0, 1, 2, 3 or 4;
p is 0, 1, 2 or 3; and
t is 0, 1 or 2.

In a preferred embodiment in combination with any of the embodiments above and below, the compound is represented by the following formulae (1) and (2) 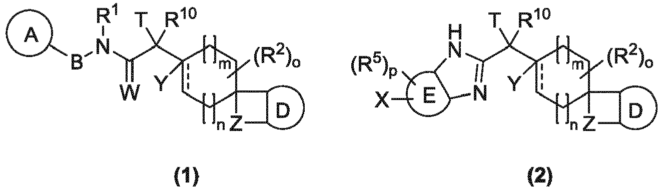 an enantiomer, diastereomer, tautomer or pharmaceutically acceptable salt thereof wherein
A represents C₃₋₁₀-cycloalkyl, which may be optionally fused with a phenyl ring, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl,C₃₋₆-cycloalkyl and halo-C₁₋₆-alkyl, or
two substituents on the same carbon atom or on two different carbon atoms form together with the carbon atom to which they are attached a C₃₋₁₀-cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo;
R^{x} represents C₁₋₆-alkyl, C₃₋₆-cycloalkyl or 3- to 6-membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo-C₁₋₆-alkyl, OR¹ and CN;
B represents a bond or C₁₋₂-alkylene, wherein alkylene is unsubstituted or substituted with one or two C₁₋₄-alkyl;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from the group consisting of halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S, and 6-membered aryl,
wherein aryl and heteroaryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the aryl or heteroaryl ring systems together with the carbon atoms to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
E represents a 6-membered aryl or 6-membered heteroaryl containing 1 to 2 nitrogen atoms;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl, or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkyleneS(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
R¹⁰ represents hydrogen, halogen, C₁₋₆-alkyl, halo-C₁₋₆-alkyl; or
R¹⁰ and T form together with the carbon atom to which they are attached a 3- to 10-membered mono- or bicyclic ring system which is saturated or partially unsaturated and wherein the ring system may further contain 1, 2 or 3 heteroatoms independently selected from N, O and S,
wherein the ring system is unsubstituted or substituted with 1 to 5 substituents independently selected from the group consisting of halogen, CN, OH, OC₁₋₆-alkyl, oxo, C₁₋₆-alkyl and halo-C₁₋₆-alkyl;
W represents O, NOR⁴, NR¹, NCN, or NS(O)₂C₁₋₆-alkyl;
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
Y is absent or represents hydrogen, OR⁴, halogen, C₁₋₆-alkyl or halo-C₁₋₆-alkyl;
Z represents -C₂₋₃-alkylene-, -O-C₁₋₂-alkylene-, -C₁₋₂-alkylene-O-, -NR³C(O)-C₀₋₁-alkylene-, -C(O)NR³-C₀₋₁-alkylene-, -C₀₋₁-alkylene-NR³C(O)-, -C₀₋₁-alkylene C(O)NR³-, -S(O)ₜ-C₁₋₂-alkylene-, -C₁₋₂-alkylene-S(O)ₜ-, -NR⁹-C₁-₂-alkylene- or -C₁-₂-alkylene-NR⁹-, wherein alkylene is unsubstituted or substituted with 1 to 4 substituents independently selected from the group consisting of OR⁴, C₁₋₆-alkyl, halogen and halo-C₁₋₆-alkyl;
R⁹ is hydrogen, C₁₋₆-alkyl, halo-C₁₋₆-alkyl, C(O)-C₁₋₆-alkyl, or C(O)-halo-C₁₋₆-alkyl;
R¹ is hydrogen or C₁₋₆-alkyl;
R² is C₁₋₆-alkyl, C₃₋₆-cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, C(O)N(R¹)₂, CN, C(O)OR⁴ or oxo, or
two R² on the same carbon atom form together with the carbon atom to which they are attached a C₃₋₁₀ cycloalkyl group, or
two R² at different carbon atoms form together a -CH₂-, -CH(CH₃)-, -C(CH₃)₂-, CH₂-CH(CH₃)-, -CH₂-CH₂- or -CH₂-CH₂-CH₂- group;
R³ is hydrogen or C₁₋₆-alkyl;
R⁴ is hydrogen or C₁₋₆-alkyl;
m is 0, 1 or 2;
n is 0, 1 or 2;
o is 0, 1, 2, 3 or 4;
p is 0, 1, 2 or 3; and
t is 0, 1 or 2.

In a preferred embodiment in combination with any of the embodiments above and below, the compound is represented by the following formulae (1-1) and (1-2) 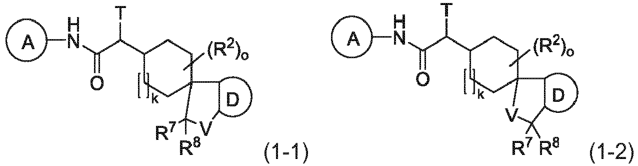 wherein
A represents C₃₋₁₀ cycloalkyl, which may be optionally fused with a phenyl ring being unsubstituted or substituted with 1 to 3 R^{a}, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S;
R^{a} represents halogen, CN, C₁₋₆-alkyl, halo-C₁₋₆-alkyl, or hydroxy-C₁₋₆-alkyl;
R^{x} represents C₁₋₆-alkyl,C₃₋₆-cycloalkyl or 3- to 6 membered heterocycloalkyl containing 1 to 2 heteroatoms independently selected from O, N and S, wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl;C₃₋₆-cycloalkyl, and halo-C₁₋₆-alkyl, or
two substituents on the aryl or heteroaryl ring systems together with the carbon atom to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
V is O or CR⁷R⁸;
R² is C₁₋₆ alkyl, C₃₋₆ cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, S(O)₂N(R¹)₂, C(O)N(R¹)₂, CN, C(O)OR⁴ or oxo, or two R² on the same carbon atom form together with the carbon atom to which they are attached a C₃₋₁₀ cycloalkyl group, or
two R² at different carbon atoms form together a -CH₂-, -CH(CH₃)-, -C(CH₃)₂-, CH₂-CH(CH₃)-, -CH₂-CH₂- or -CH₂-CH₂-CH₂- group;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-atkytene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -Co-₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
o is 0, 1, 2, 3 or 4; and
k is 1 or 2.

In a preferred embodiment in combination with any of the embodiments above and below, the compound is represented by the following formulae (1-3) and (1-4) 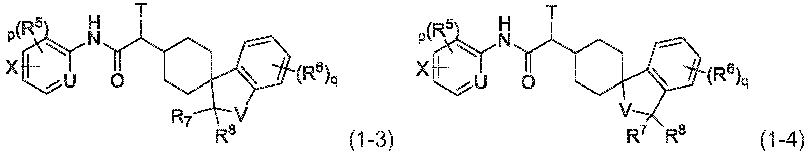
wherein X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
U is N or CR⁵_{;}
V is O or CR⁷R⁸;
p is 0, 1, 2 or 3; and
q is 0, 1, 2, 3 or 4.

In a further preferred embodiment in combination with any of the embodiments above and below, the compound is represented by the following formula (1-5) 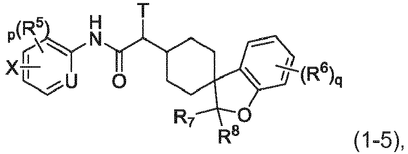 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
U is N or CR⁵_{;}
p is 0, 1, 2 or 3; and
q is 0, 1, 2, 3 or 4.

In an equally further preferred embodiment in combination with any of the embodiments above and below, the compound is represented by the following formula (1-6) 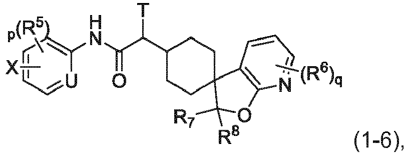 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-atkytene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R ¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
U is N or CR⁵_{;}
p is 0, 1, 2 or 3; and
q is 0, 1, 2, 3 or 4.

In a preferred embodiment in combination with any of the embodiments above and below, the compound is represented by the following formula(1-7) 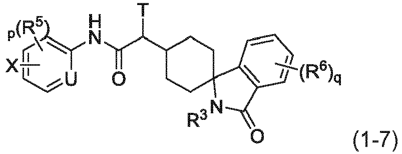 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R³ is hydrogen or C₁₋₆-alkyl;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkyleneS(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
U is N or CR⁵_{;}
p is 0, 1, 2 or 3; and
q is 0, 1, 2, 3 or 4.

In a preferred embodiment in combination with any of the embodiments above and below, the compound is represented by the following formulae (2-1) and (2-2) 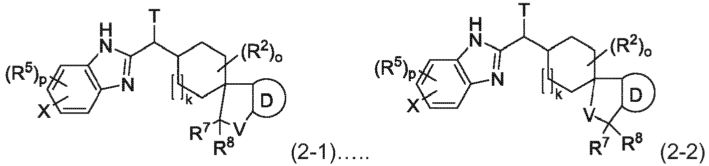
wherein X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
R^{x} represents C₁₋₆-alkyl, C₃₋₆-cycloalkyl or 3- to 6 membered heterocycloalkyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl, or
two substituents on the aryl or heteroaryl ring systems together with the carbon atom to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
V is O or CR⁷R⁸;
R² is C₁₋₆ alkyl, C₃₋₆ cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, S(O)₂N(R¹)₂, C(O)N(R¹)₂, CN, C(O)OR⁴ or oxo, or two R² on the same carbon atom form together with the carbon atom to which they are attached a C₃₋₁₀ cycloalkyl group, or
two R² at different carbon atoms form together a -CH₂-, -CH(CH₃)-, -C(CH₃)₂-, CH₂-CH(CH₃)-, -CH₂-CH₂- or -CH₂-CH₂-CH₂- group;
R⁷ and R⁸ are independently selected from the group consisting of hydrogen, halogen, C₁₋₆ alkyl, halo-C₁₋₆-alkyl and OR⁴;
o is 0, 1, 2, 3 or 4; and
k is 1 or 2.

In a preferred embodiment in combination with any of the embodiments above and below, the compound is represented by the following formulae (2-3) and (2-4) 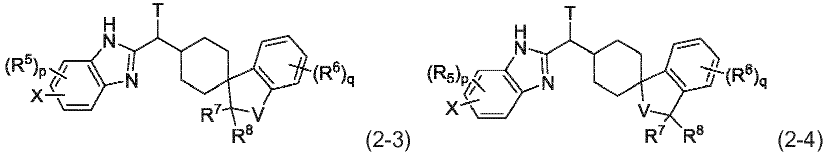
wherein X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl;C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-atkytene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
V is O or CR⁷R⁸;
p is 0, 1, 2 or 3; and
q is 0, 1, 2, 3 or 4.

In a more preferred embodiment in combination with any of the above and below embodiments, the compound is represented by the following formula (2-5) 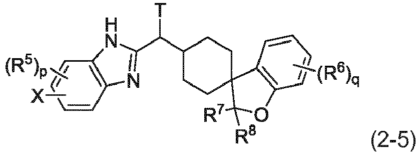 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S, wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl, wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
p is 0, 1, 2 or 3; and
q is 0, 1, 2, 3 or 4.

In a further preferred embodiment in combination with any of the above and below embodiments, T represents hydrogen, halogen, CN, C₁₋₆-alkyl, halo-C₁₋₆-alkyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-CN, -C₀₋₆-alkylene-R^{x} and -C₀₋₆-alkylene-OR^{x}.

In a more preferred embodiment in combination with any of the embodiments above and below, T represents hydrogen, C₁₋₆-alkyl, halo-C₁₋₆-alkyl, C₃₋₆-cycloalkyl, 3- to 6 membered cycloalkyl-C₁₋₆-alkyl,
wherein alkyl and cycloalkyl are unsubstituted or substituted with 1 to 5 substituents independently selected from the group consisting of C₁₋₆-alkyl, halo-C₁₋₆-alkyl, C₃₋₆-cycloalkyl, halogen, CN, OH and O-C₁₋₆-alkyl.

In a further preferred embodiment in combination with any of the above and below embodiments, the compound is represented by formula (1).

In a preferred embodiment of the compounds according to formula (1) in combination with any of the above and below embodiments, A represents C₃₋₆-cycloalkyl, 3- to 7-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo; and
R^{x} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo C₁₋₆-alkyl, OR¹ and CN.

In a more preferred embodiment of the compounds according to formula (1) in combination with any of the above and below embodiments, A represents 6- to 10-membered mono or bicyclic aryl or 5- to 10-membered mono or bicyclic heteroaryl containing 1 to 4 heteroatoms independently selected from O, N and S, wherein aryl and heteroaryl are unsubstituted or substituted with 1 to 5 substituents independently selected from the group consisting of halogen, OH, R^{x}, O-R^{x}, OC(O)-R^{x}, S-R^{x}, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo; and
R^{x} represents C₁₋₆-alkyl or C₃₋₆-cycloalkyl,
wherein alkyl and cycloalkyl are unsubstituted or substituted with 1 or 6 substituents independently selected from the group consisting of halogen, C₁₋₆-alkyl, halo C₁₋₆-alkyl, OR¹ and CN.

In a further preferred embodiment of the compounds according to formula (1) in combination with any of the above and below embodiments, A represents phenyl or 5- or 6-membered heteroaryl containing 1 to 4 heteroatoms independently selected from O, N and S, wherein phenyl and heteroaryl are substituted with 1 to 4 substituents independently selected from the group consisting of halogen, OH, R^{x}, O-R^{x}, CN, COOH and 5- or 6-membered heteroaryl containing 1 to 4 heteroatoms independently selected from O, N and S,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl; and
R^{x} represents C₁₋₆-alkyl or C₃₋₆-cycloalkyl,
wherein alkyl and cycloalkyl are unsubstituted or substituted with 1 or 6 substituents independently selected from the group consisting of halogen, C₁₋₆-alkyl, halo-C₁₋₆-alkyl, OR¹ and CN.

In a more preferred embodiment of the compounds according to formula (1) in combination with any of the above and below embodiments, A represents phenyl which is unsubstituted or substituted with 1 to 4 substituents independently selected from the group consisting of halogen, OH, R^{x}, O-R^{x}, OC(O)-R^{x}, S-R^{x}, S(O)2-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, and
R^{x} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo-C₁₋₆-alkyl, OR¹ and CN.

Also preferred is an embodiment of the compounds according to formula (1) in combination with any of the above and below embodiments, wherein A represents C₃₋₆-cycloalkyl, which may be optionally fused with a phenyl ring being unsubstituted or substituted by 1 to 3 R^{a}, or 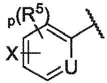 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S, wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
U is N or CR⁵;
p is 0, 1, 2 or 3; and
R^{a} represents halogen, CN, C₁₋₆-alkyl, halo-C₁₋₆-alkyl, hydroxy-C₁₋₆-alkyl, C₃₋₆-cycloalkyl or halo-C₃₋₆-cycloalkyl.

In a more preferred embodiment of the compounds according to formula (1) in combination with any of the above and below embodiments, A represents 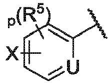 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S, wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
U is N or CR⁵; and
p is 0, 1, 2 or 3.

In an even more preferred embodiment of the compounds according to formula (1) in combination with any of the above and below embodiments, A represents 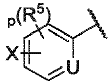 wherein
X is halogen, C₁₋₆-alkyl, C₃₋₆-cycloalkyl, or CN, wherein alkyl and cycloalkyl are unsubstituted or substituted with halogen or C₁₋₆-alkyl;
R⁵ is independently selected from hydrogen, halogen and C₁₋₆-alkyl;
U is N or CR⁵; and
p is 0, 1, 2 or 3.

In a further even more preferred embodiment of the compounds according to formula (1) in combination with any of the above and below embodiments, A represents 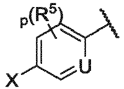 wherein
X is halogen, C₁₋₆-alkyl, C₃₋₆-cycloalkyl, or CN
wherein alkyl and cycloalkyl are unsubstituted or substituted with halogen or C₁₋₆-alkyl; R⁵ is independently selected from hydrogen, halogen and C₁₋₆-alkyl;
U is N or CR⁵; and
p is 0, 1, 2 or 3.

In a preferred embodiment in combination with any of the above and below embodiments, U is N.

In a further preferred embodiment in combination with any of the above and below embodiments, U is CR⁵ and R⁵ is independently selected from hydrogen, halogen and C₁₋₆-alkyl.

In a most preferred embodiment of the compounds according to formula (1) in combination with any of the above and below embodiments, A represents 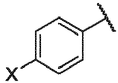 wherein
X is halogen, C₁₋₆-alkyl, C₃₋₆-cycloalkyl, or CN
wherein alkyl and cycloalkyl are unsubstituted or substituted with halogen or C₁₋₆-alkyl.

In an equally most preferred embodiment of the compounds according to formula (1) in combination with any of the above and below embodiments, A represents 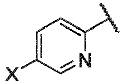 wherein
X is halogen, C₁₋₆-alkyl, C₃₋₆-cycloalkyl, or CN
wherein alkyl and cycloalkyl are unsubstituted or substituted with halogen or C₁₋₆-alkyl.

In a more preferred embodiment of the compounds according to formula (1) in combination with any of the above and below embodiments, A represents 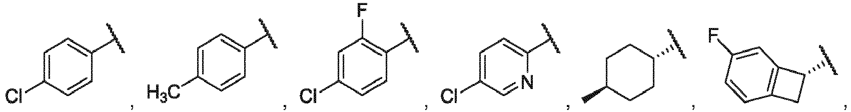 or 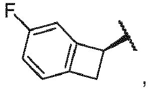 more preferably

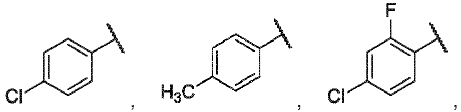

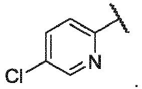

In an utmost preferred embodiment of the compounds according to formula (1) in combination with any of the above and below embodiments, A represents

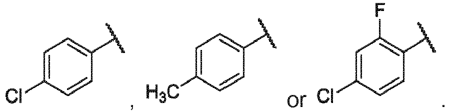

In an equally utmost preferred embodiment of the compounds according to formula (1) in combination with any of the above and below embodiments, A represents

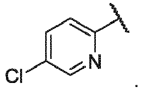

In a preferred embodiment of the compounds according to formula (1) in combination with any of the above and below embodiments, R^{a} represents halogen, CN, C₁₋₆-alkyl, halo-C₁₋₆-alkyl, or hydroxy-C₁₋₆-alkyl.

In a more preferred embodiment of the compounds according to formula (1) in combination with any of the above and below embodiments, R^{a} represents halogen, CN, or C₁₋₆-alkyl.

In a most preferred embodiment of the compounds according to formula (1) in combination with any of the above and below embodiments, R^{a} represents F or Cl, more preferably F.

In a preferred embodiment of the compounds according to formula (1) in combination with any of the above and below embodiments, R^{x} represents C₁₋₆-alkyl or C₃₋₆-cycloalkyl, wherein alkyl and cycloalkyl are unsubstituted or substituted with 1 or 4 substituents independently selected from the group consisting of halogen, C₁₋₆-alkyl, halo C₁₋₆-alkyl, OR¹ and CN.

In a further preferred embodiment of the compounds according to formula (1) in combination with any of the above and below embodiments, B represents a bond.

In a further preferred embodiment of the compounds according to formula (1) in combination with any of the above and below embodiments, R¹ is hydrogen.

In an equally preferred embodiment of the compounds according to formula (1) in combination with any of the above and below embodiments, R¹ is C₁₋₆ alkyl.

In a preferred embodiment of the compounds according to formula (1) in combination with any of the embodiments above and below, T represents halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl.

In an equally preferred embodiment of the compounds according to formula (1) in combination with any of the above and below embodiments, T is hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 3- to 6-membered heterocyclyl, 3- to 6-membered heterocyclyl-C₁₋₆-alkyl or 3- to 6-membered cycloalkyl-C₁₋₆-alkyl
wherein alkyl, cycloalkyl and heterocycloalkyl, are unsubstituted or substituted with 1 to 5 substituents independently selected from the group consisting of halogen, OH, R^{x}, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl.

In a more preferred embodiment of the compounds according to formula (1) in combination with any of the above and below embodiments, T is hydrogen, halogen, CN, OH, OR^{x}, S-R^{x}, C₁₋₆-alkyl, halo-C₁₋₆-alkyl, C₂₋₆-alkenyl, C₃₋₆-cycloalkyl, 3- to 6-membered heterocyclyl, 3- to 6-membered heterocyclyl-C₁₋₆-alkyl or 3- to 6-membered cycloalkyl-C₁₋₆-alkyl,
wherein alkyl, cycloalkyl and heterocycloalkyl, are unsubstituted or substituted with 1 to 5 substituents independently selected from the group consisting of halogen, OH, R^{x}, OR^{x}, S-R^{x}, CN and COOH.

In an even more preferred embodiment of the compounds according to formula (1) in combination with any of the above and below embodiments, T is hydrogen, C₁₋₆-alkyl, halo-C₁₋₆-alkyl, C₃₋₆-cycloalkyl, 3- to 6-membered heterocyclyl or 3- to 6-membered cycloalkyl-C₁₋₆-alkyl,
wherein alkyl, and cycloalkyl are unsubstituted or substituted with 1 to 5 substituents independently selected from the group consisting of halogen, OH, C₁₋₆-alkyl, halo-C₁₋₆-alkyl, C₃₋₆-cycloalkyl and O-C₁₋₆-alkyl.

In a most preferred embodiment of the compounds according to formula (1) in combination with any of the above and below embodiments, T is hydrogen, C₁₋₆-alkyl, or halo-C₁₋₆-alkyl, more preferably C₁₋₆-alkyl, or halo-C₁₋₆-alkyl, most preferably C₁₋₆-alkyl.

In a preferred embodiment of the compounds according to formula (1) in combination with any of the above and below embodiments, R² is C₁₋₆ alkyl, C₃₋₆ cycloalkyl, halo-C₁₋₆-alkyl, OR⁴, CN, oxo or two R² on the same carbon atom form together with the carbon atom to which they are attached a C₃₋₆ cycloalkyl group, or two R² at different carbon atoms form together a -CH₂-, -CH(CH₃)-, -C(CH₃)₂-, CH₂-CH(CH₃)-, -CH₂-CH₂- or -CH₂-CH₂-CH₂- and o is 0, 1 or 2.

In a more preferred embodiment of the compounds according to formula (1) in combination with any of the above and below embodiments, R² is C₁₋₆-alkyl, C₃₋₆-cycloalkyl, halo-C₁₋₆-alkyl, OR⁴ or oxo or two R² at different carbon atoms form together a -CH₂-, -CH(CH₃)-, -C(CH₃)₂-, CH₂-CH(CH₃)-, -CH₂-CH₂- or -CH₂-CH₂-CH₂- and o is 0, 1 or 2.

In a preferred embodiment of the compounds according to formula (1) in combination with any of the above and below embodiments, Z represents -ethylene-, -O-methylene-, -methylene-O-, -NR³C(O)-, -C(O)NR³-, -NR³C(O)-, -C(O)NR³-, -S(O)ₜ-methylene-, - methylene-S(O)ₜ-, -NR⁹-methylene- or -methylene-NR⁹-,
wherein methylene and ethylene are unsubstituted or substituted with 1 to 4 substituents independently selected from the group consisting of OR⁴, C₁₋₆-alkyl, halogen and halo-C₁₋₆-alkyl.

In a more preferred embodiment of the compounds according to formula (1) in combination with any of the above and below embodiments, Z represents an ethylene group which is unsubstituted or substituted with 1 to 4 substituents independently selected from the group consisting of OR⁴, C₁₋₆-alkyl, halogen and halo-C₁₋₆-alkyl.

In an equally more preferred embodiment of the compounds according to formula (1) in combination with any of the above and below embodiments, Z represents -CR⁷R⁸O-, wherein R⁷ and R⁸ are independently selected from the group consisting of hydrogen, halogen, C₁₋₆ alkyl, halo-C₁₋₆-alkyl and OR⁴.

In a more preferred embodiment of the compounds according to formula (1) in combination with any of the above and below embodiments, o is 0.

In a preferred embodiment of the compounds according to formula (1) in combination with any of the above and below embodiments, W represents O or NR¹.

In a more preferred embodiment of the compounds according to formula (1) in combination with any of the above and below embodiments, W represents O.

In a preferred embodiment of the compounds according to formula (1) in combination with any of the above and below embodiments, m is 0, 1 or 2 and n is 0 or 1.

In a more preferred embodiment of the compounds according to formula (1) in combination with any of the above and below embodiments, m is 1 or 2 and n is 1.

In a most preferred embodiment of the compounds according to formula (1) in combination with any of the above and below embodiments, m and n are both 1.

In a preferred embodiment of the compounds according to formula (1) in combination with any of the above and below embodiments, the compound is represented by the following formulae (1-1) or (1-2) 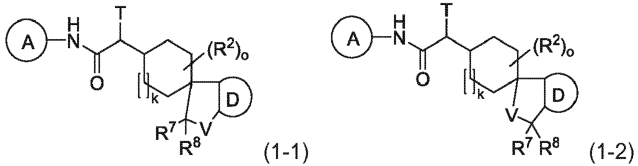
wherein V represents -C₀₋₂-alkylene-, -O-C₀₋₁-alkylene-, -C₀₋₁-alkylene-O-, -NR³C(O)-, -C(O)NR³-, -NR³C(O)-, -C(O)NR³-, -S(O)ₜ-C₀₋₁-alkylene-, -C₀₋₁-alkylene-S(O)ₜ-, -NR⁹-C₀₋₁-alkylene- or -C₀₋₁-alkylene-NR⁹-,
wherein alkylene is unsubstituted or substituted with 1 or 2 substituents independently selected from the group consisting of OR⁴, C₁₋₆-alkyl, halogen and halo-C₁₋₆-alkyl;
R⁷ and R⁸ are independently selected from the group consisting of hydrogen, halogen, C₁₋₆ alkyl, halo-C₁₋₆-alkyl and OR⁴;
R⁹ is hydrogen, C₁₋₆-alkyl, halo-C₁₋₆-alkyl, C(O)-C₁₋₆-alkyl, or C(O)-halo-C₁₋₆-alkyl and k is 1 or 2.

In a more preferred embodiment of the compounds according to formulae (1-1) or (1-2) in combination with any of the above and below embodiments, V represents -CR⁷R⁸-, -O-C₀₋₁-alkylene-, -C₀₋₁-alkylene-O-, -NR⁹-C₀₋₁-alkylene- or -C₀₋₁-alkylene-NR⁹-,
wherein alkylene is unsubstituted or substituted with 1 or 2 substituents independently selected from the group consisting of OR⁴, C₁₋₆-alkyl, halogen and halo-C₁₋₆-alkyl;
R⁷ and R⁸ are independently selected from the group consisting of hydrogen, halogen, C₁₋₆ alkyl, halo-C₁₋₆-alkyl and OR⁴;
R⁹ is hydrogen, C₁₋₆-alkyl, halo-C₁₋₆-alkyl, C(O)-C₁₋₆-alkyl, or C(O)-halo-C₁₋₆-alkyl; and
k is 1 or 2.

In an even more preferred embodiment of the compounds according to formulae (1-1) or (1-2) in combination with any of the above and below embodiments, V represents - CR⁷R⁸- or -O-;
R⁷ and R⁸ are independently selected from the group consisting of hydrogen, halogen, C₁₋₆ alkyl, halo-C₁₋₆-alkyl and OR⁴; and
k is 1 or 2.

In a most preferred embodiment of the compounds according to formulae (1-1) or (1-2) in combination with any of the above and below embodiments, V represents -CR⁷R⁸-;
R⁷ and R⁸ are independently selected from the group consisting of hydrogen, halogen, C₁₋₆ alkyl, halo-C₁₋₆-alkyl and OR⁴; and
k is 1 or 2.

In an equally most preferred embodiment of the compounds according to formulae (1-1) or (1-2) in combination with any of the above and below embodiments, V represents -O-;
R⁷ and R⁸ are independently selected from the group consisting of hydrogen, halogen, C₁₋₆ alkyl, halo-C₁₋₆-alkyl and OR⁴; and
k is 1 or 2.

In a preferred embodiment of the compounds according to formulae (1-1) or (1-2) in combination with any of the above and below embodiments, A represents C₃₋₁₀ cycloalkyl, which may be optionally fused with a phenyl ring, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl, or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S; and
R^{x} represents C₁₋₆-alkyl, C₃₋₆-cycloalkyl or 3- to 6 membered heterocycloalkyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN.

In a more preferred embodiment of the compounds according to formulae (1-1) or (1-2) in combination with any of the above and below embodiments, A represents C₃₋₁₀ cycloalkyl, 6- to 10-membered mono or bicyclic aryl or 5- to 10-membered mono or bicyclic heteroaryl containing 1 to 4 heteroatoms independently selected from O, N and S,
wherein cycloalkyl, aryl and heteroaryl are unsubstituted or substituted with 1 to 5 substituents independently selected from the group consisting of halogen, OH, R^{x}, O-R^{x}, S-R^{x}, S(O)₂-R^{x}, N(R¹)₂, CN, COOH, 5- or 6-membered heteroaryl containing 1 to 4 heteroatoms independently selected from O, N and S and 6-membered aryl.

In a more preferred embodiment of the compounds according to formulae (1-1) or (1-2) in combination with any of the above and below embodiments, the compound is represented by formulae (1-3) or (1-4) 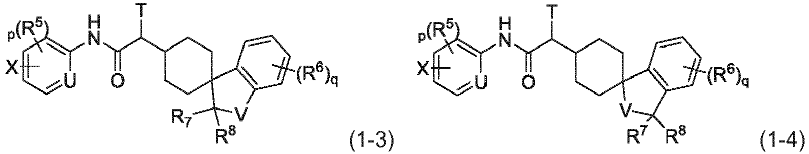 wherein U is N or CR⁵;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN; and
q is 0, 1, 2, 3, or 4.

In a preferred embodiment of the compounds according to formula (1) in combination with any of the above and below embodiments, the compound is represented by the following formula

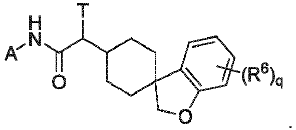

In an equally preferred embodiment of the compounds according to formula (1) in combination with any of the above and below embodiments, the compound is represented by the following formula

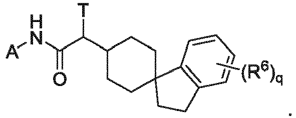

In a further equally preferred embodiment of the compounds according to formula (1) in combination with any of the above and below embodiments, the compound is represented by the following formula

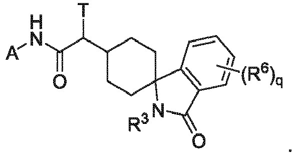

In a more preferred embodiment of the compounds according to formula (1) in combination with any of the above and below embodiments, the compound is represented by the following formula.

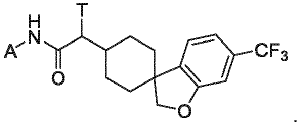

In an equally more preferred embodiment of the compounds according to formula (1) in combination with any of the above and below embodiments, the compound is represented by the following formula

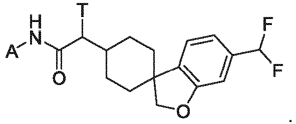

In a more preferred embodiment of the compounds according to formula (1) in combination with any of the above and below embodiments, the compound is represented by the following formula

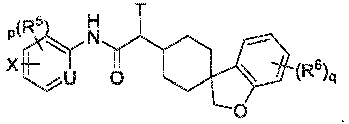

In an equally more preferred embodiment of the compounds according to formula (1) in combination with any of the above and below embodiments, the compound is represented by the following formula

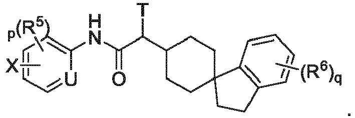

In a further equally more preferred embodiment of the compounds according to formula (1) in combination with any of the above and below embodiments, the compound is represented by the following formula

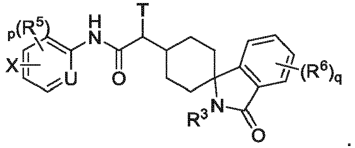

In an even more preferred embodiment of the compounds according to formula (1) in combination with any of the above and below embodiments, the compound is represented by the following formula

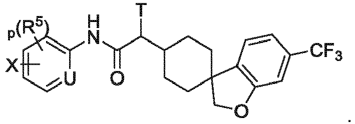

In a more preferred embodiment of the compounds according to formula (1) in combination with any of the above and below embodiments, the compound is represented by the following formula

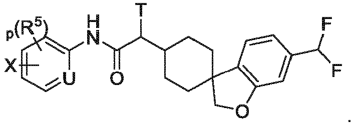

In a preferred embodiment of the compounds according to formula (1) in combination with any of the above and below embodiments, the compound is represented by the following formula

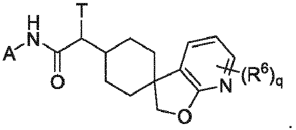

In a more preferred embodiment of the compounds according to formula (1) in combination with any of the above and below embodiments, the compound is represented by the following formula

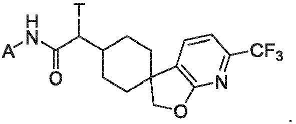

In an equally more preferred embodiment of the compounds according to formula (1) in combination with any of the above and below embodiments, the compound is represented by the following formula

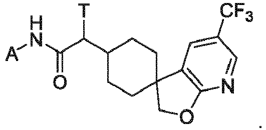

In a more preferred embodiment of the compounds according to formula (1) in combination with any of the above and below embodiments, the compound is represented by the following formula

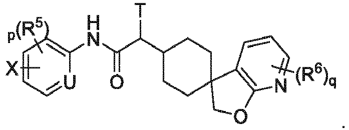

In an even more preferred embodiment of the compounds according to formula (1) in combination with any of the above and below embodiments, the compound is represented by the following formula

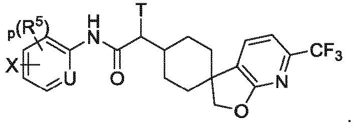

In a further more preferred embodiment of the compounds according to formula (1) in combination with any of the above and below embodiments, the compound is represented by the following formula

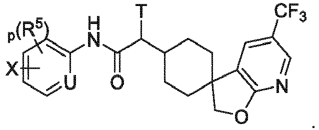

In a preferred embodiment of the compound according to formula (2) in combination with any of the above and below embodiments, the compound is represented by formulae (2-1) or (2-2) 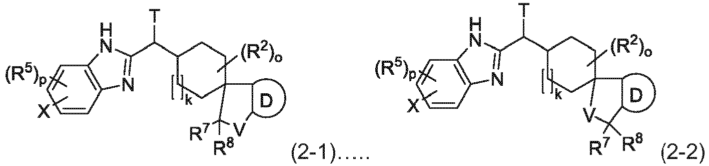
wherein V represents -C₀₋₂-alkylene-, -O-C₀₋₁-alkylene-, -C₀₋₁-alkylene-O-, -NR³C(O)-, - C(O)NR³-, -NR³C(O)-, -C(O)NR³-, -S(O)ₜ-C₀₋₁-alkylene-, -C₀₋₁-alkylene-S(O)ₜ-, -NR⁹-C₀₋₁-alkylene- or -C₀₋₁-alkylene-NR⁹-,
wherein alkylene is unsubstituted or substituted with 1 or 2 substituents independently selected from the group consisting of OR⁴, C₁₋₆-alkyl, halogen and halo-C₁₋₆-alkyl;
R⁷ and R⁸ are independently selected from the group consisting of hydrogen, halogen, C₁₋₆ alkyl, halo-C₁₋₆-alkyl and OR⁴;
R⁹ is hydrogen, C₁₋₆-alkyl, halo-C₁₋₆-alkyl, C(O)-C₁₋₆-alkyl, or C(O)-halo-C₁₋₆-alkyl and
k is 1 or 2.

In a more preferred embodiment of the compounds according to formulae (2-1) or (2-2) in combination with any of the above and below embodiments, V represents -CR⁷R⁸- - O-C₀₋₁-alkylene-, -C₀₋₁-alkylene-O-, -NR⁹-C₀₋₁-alkylene- or -C₀₋₁-alkylene-NR⁹-,
wherein alkylene is unsubstituted or substituted with 1 or 2 substituents independently selected from the group consisting of OR⁴, C₁₋₆-alkyl, halogen and halo-C₁₋₆-alkyl;
R⁷ and R⁸ are independently selected from the group consisting of hydrogen, halogen, C₁₋₆ alkyl, halo-C₁₋₆-alkyl and OR⁴;
R⁹ is hydrogen, C₁₋₆-alkyl, halo-C₁₋₆-alkyl, C(O)-C₁₋₆-alkyl, or C(O)-halo-C₁₋₆-alkyl; and
k is 1 or 2.

In an even more preferred embodiment of the compounds according to Formulae (2-1) or (2-2) in combination with any of the above and below embodiments, V represents -CR⁷R⁸- or -O-;
R⁷ and R⁸ are independently selected from the group consisting of hydrogen, halogen, C₁₋₆ alkyl, halo-C₁₋₆-alkyl and OR⁴; and
k is 1 or 2.

In a most preferred embodiment of the compounds according to formulae (2-1) or (2-2) in combination with any of the above and below embodiments, V represents -CR⁷R⁸-;
R⁷ and R⁸ are independently selected from the group consisting of hydrogen, halogen, C₁₋₆ alkyl, halo-C₁₋₆-alkyl and OR⁴; and
k is 1 or 2.

In a more preferred embodiment of the compound according to formulae (2-1) or (2-2) in combination with any of the above and below embodiments, the compound is represented by formulae (2-3) or (2-4)

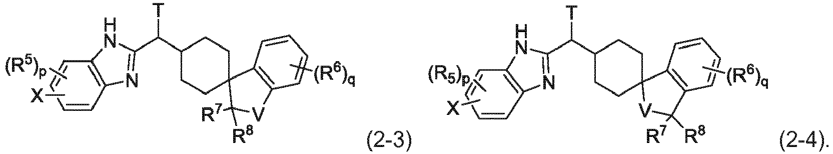

In a more preferred embodiment of the compound according to formulae (2-1), (2-2) (2-3) or (2-4) in combination with any of the above and below embodiments, X is halogen, C₁₋₆-alkyl, C₃₋₆-cycloalkyl, or CN, wherein alkyl and cycloalkyl are unsubstituted or substituted with halogen or C₁₋₆-alkyl; and
R⁵ is independently selected from hydrogen, halogen and C₁₋₆-alkyl.

In a preferred embodiment of the compounds according to formula (2) in combination with any of the embodiments above and below, T represents halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl.

In an equally preferred embodiment of the compounds according to formula (2) in combination with any of the above and below embodiments, T is hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 3- to 6-membered heterocyclyl, 3- to 6-membered heterocyclyl-C₁₋₆-alkyl or 3- to 6-membered cycloalkyl-C₁₋₆-alkyl
wherein alkyl, cycloalkyl and heterocycloalkyl, are unsubstituted or substituted with 1 to 5 substituents independently selected from the group consisting of halogen, OH, R^{x}, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl.

In a more preferred embodiment of the compounds according to formula (2) in combination with any of the above and below embodiments, T is hydrogen, halogen, CN, OH, OR^{x}, S-R^{x}, C₁₋₆-alkyl, halo-C₁₋₆-alkyl, C₂₋₆-alkenyl, C₃₋₆-cycloalkyl, 3- to 6-membered heterocyclyl, 3- to 6-membered heterocyclyl-C₁₋₆-alkyl or 3- to 6-membered cycloalkyl-C₁₋₆-alkyl,
wherein alkyl, cycloalkyl and heterocycloalkyl, are unsubstituted or substituted with 1 to 5 substituents independently selected from the group consisting of halogen, OH, R^{x}, OR^{x}, S-R^{x}, CN and COOH.

In an even more preferred embodiment of the compounds according to formula (2) in combination with any of the above and below embodiments, T is hydrogen, C₁₋₆-alkyl, halo-C₁₋₆-alkyl, C₃₋₆-cycloalkyl, 3- to 6-membered heterocyclyl or 3- to 6-membered cycloalkyl-C₁₋₆-alkyl,
wherein alkyl, and cycloalkyl are unsubstituted or substituted with 1 to 5 substituents independently selected from the group consisting of halogen, OH, C₁₋₆-alkyl, halo-C₁₋₆-alkyl, C₃₋₆-cycloalkyl and O-C₁₋₆-alkyl.

In a most preferred embodiment of the compounds according to formula (2) in combination with any of the above and below embodiments, T is hydrogen, C₁₋₆-alkyl or halo-C₁₋₆-alkyl, more preferably C₁₋₆-alkyl or halo-C₁₋₆-alkyl, most preferably C₁₋₆-alkyl.

In a preferred embodiment of the compounds according to formula (2) in combination with any of the embodiments above and below, the compound is represented by the following formula

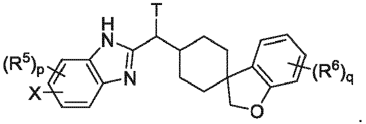

In a more preferred embodiment of the compounds according to formula (2) in combination with any of the embodiments above and below, the compound is represented by the following formula

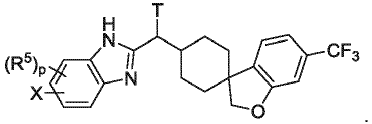

In a further preferred embodiment of the compound according to formula (1), the compound is selected from 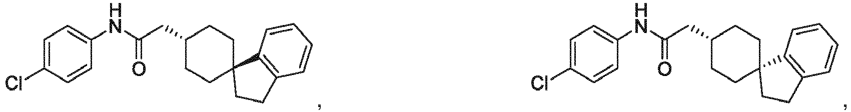 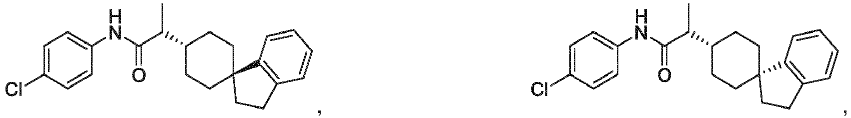 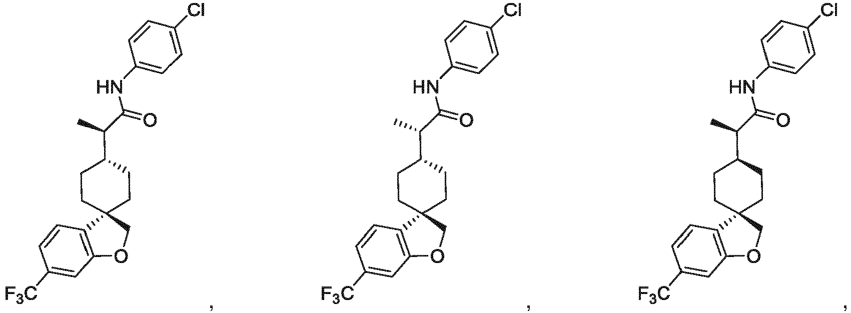 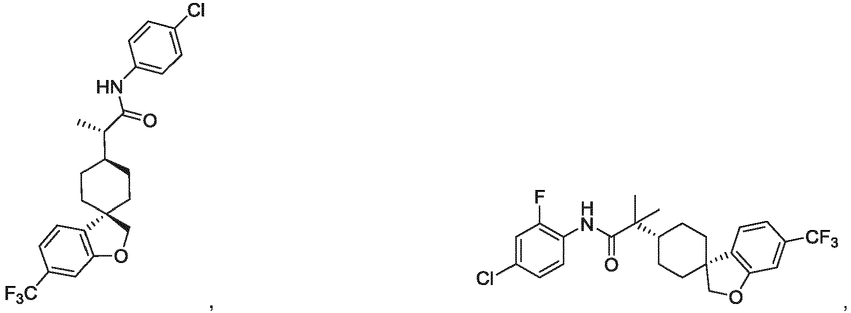 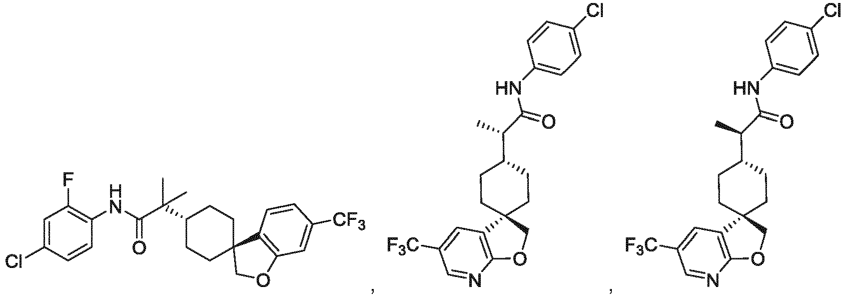 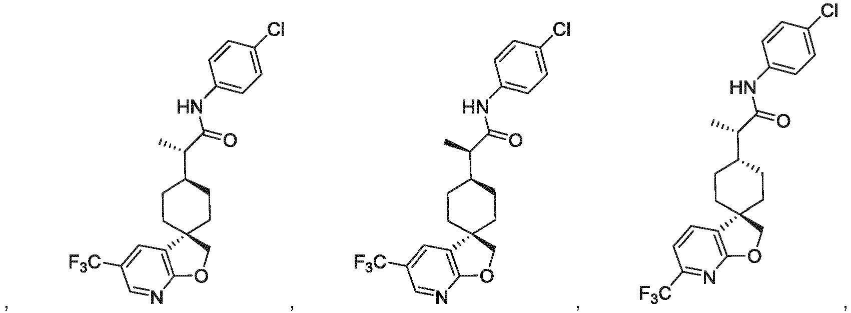 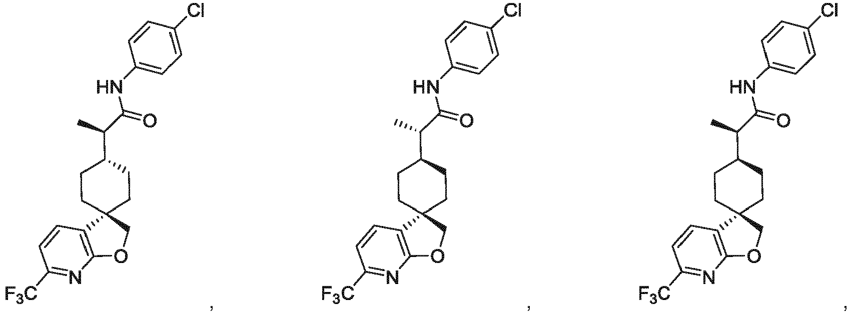 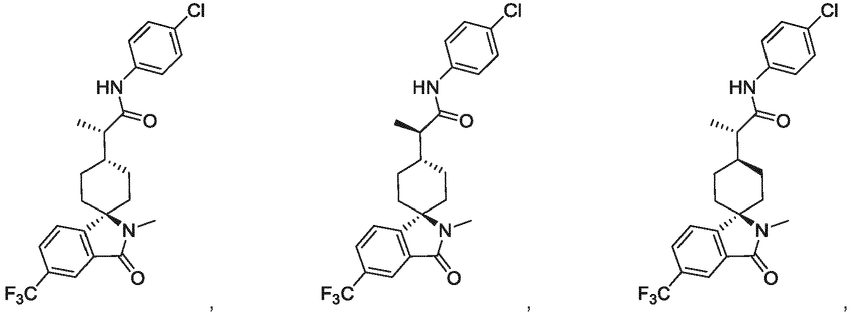 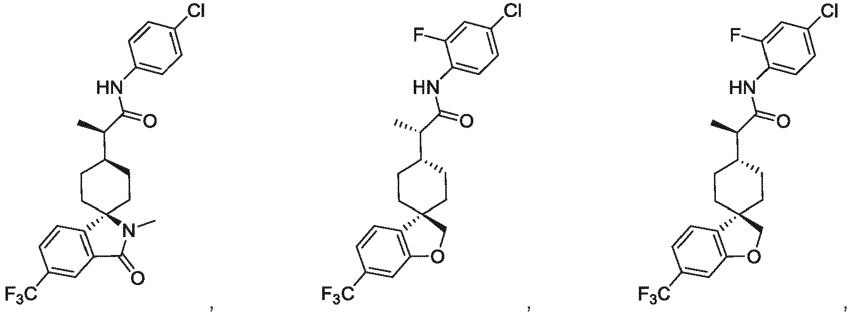 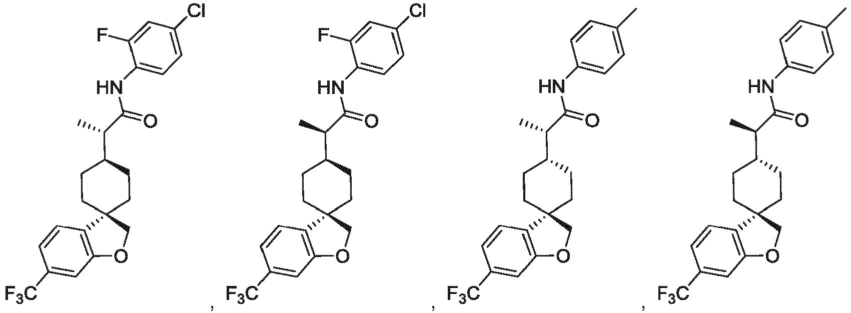 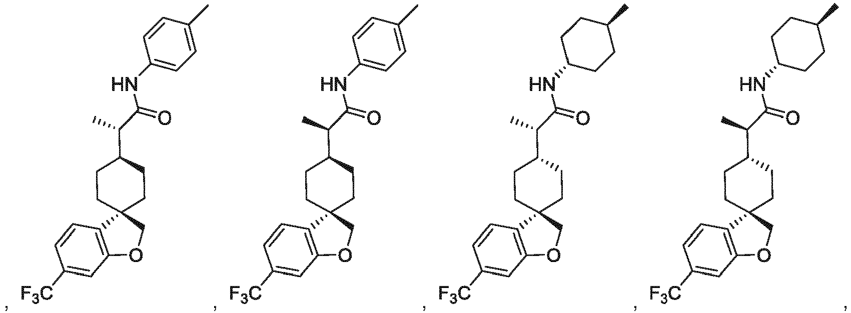 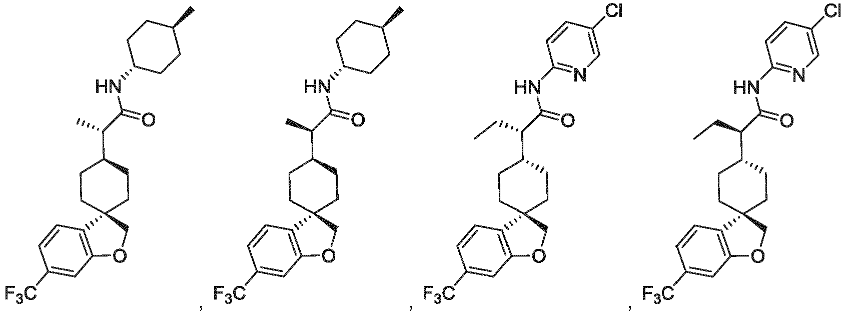 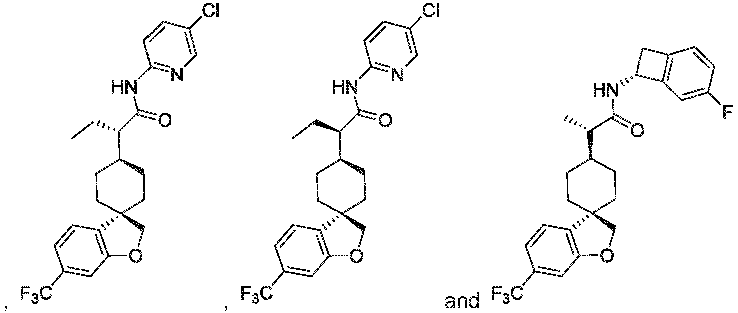 and their pharmaceutically acceptable salts thereof.

In a more preferred embodiment of the compound according to formula (1), the compound is selected from 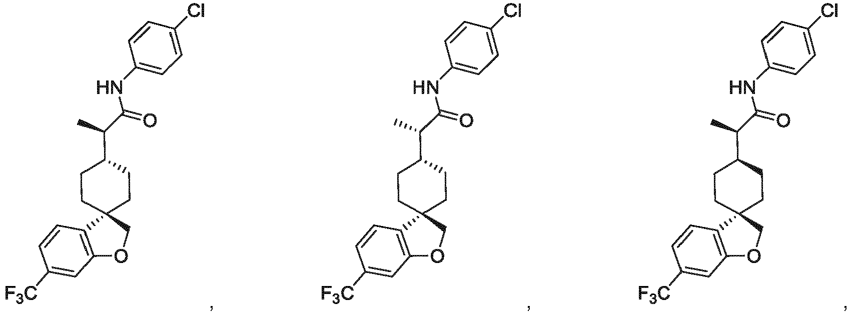 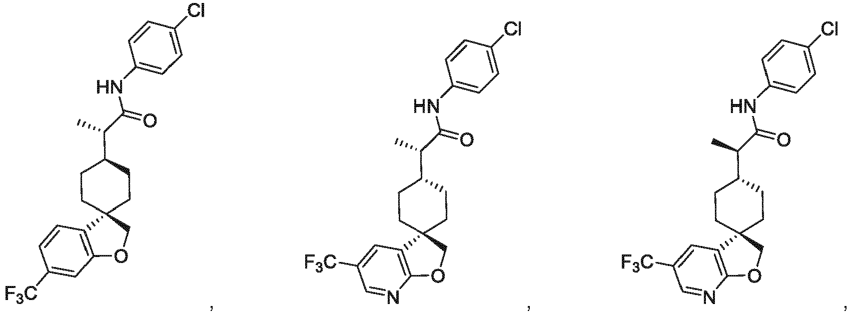 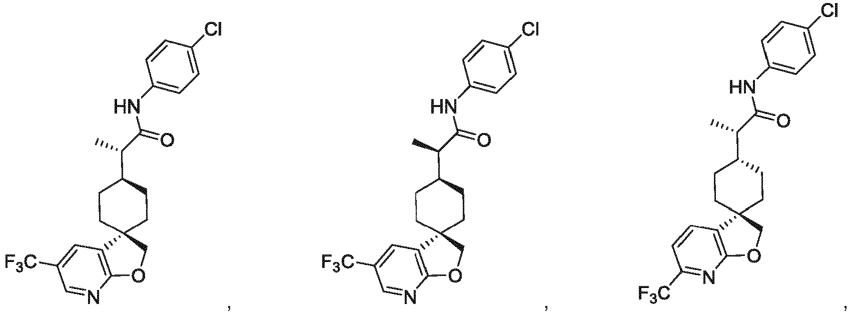 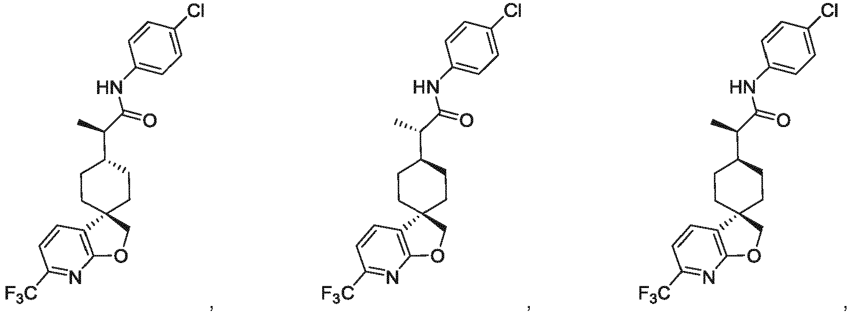 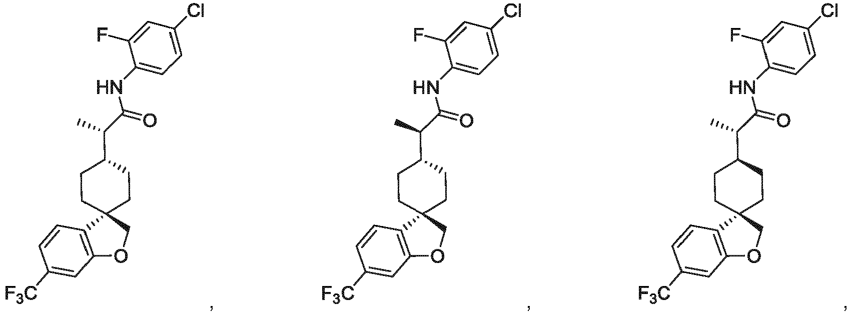 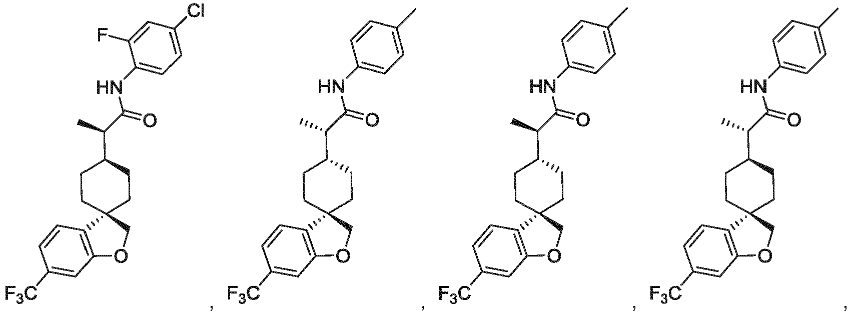 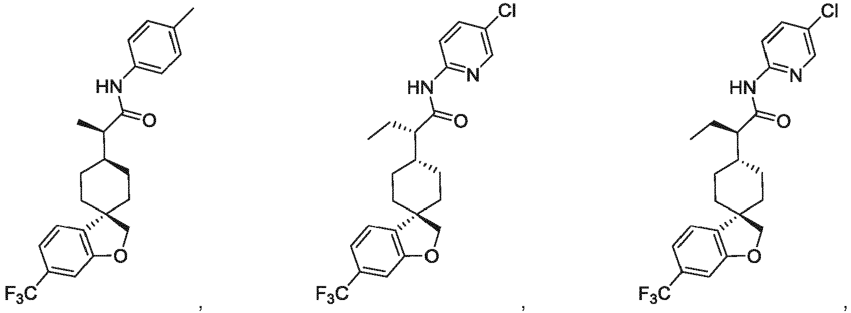 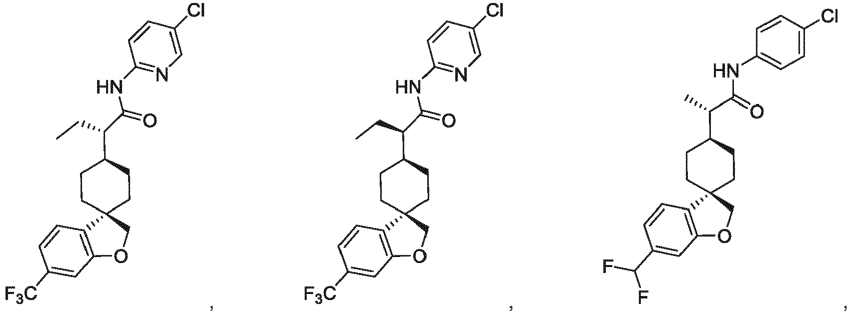 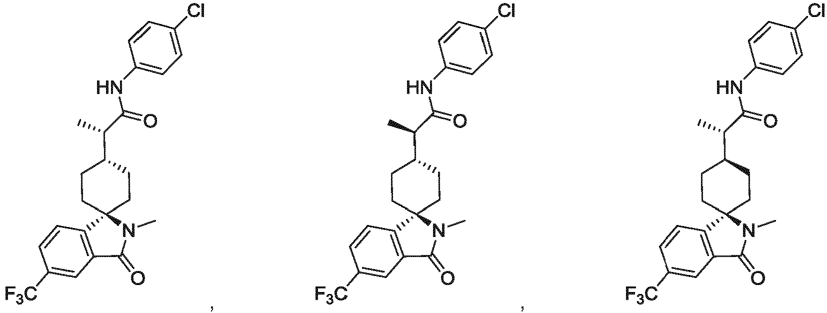 or 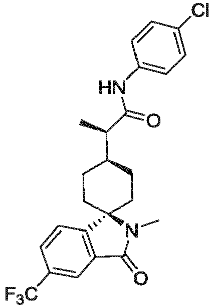 and their pharmaceutically acceptable salts thereof.

In a further more preferred embodiment of the compound according to formula (1), the compound is selected from 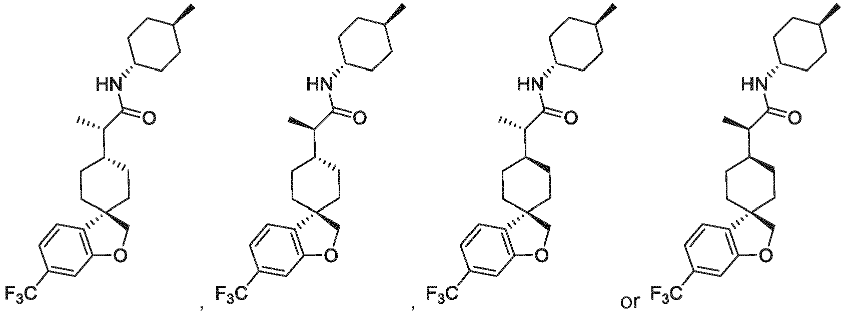 and their pharmaceutically acceptable salts thereof.

In a further more preferred embodiment of the compound according to formula (1), the compound is selected from 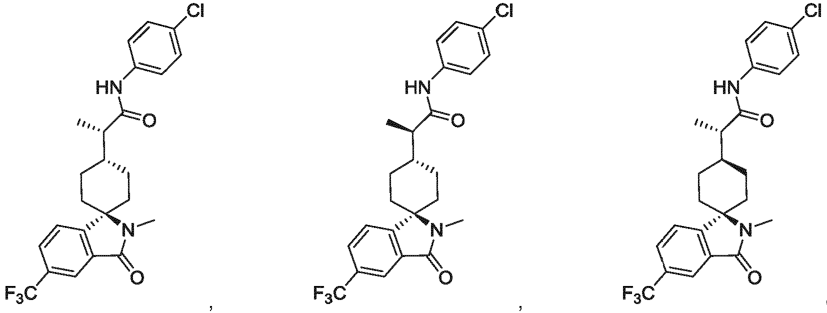 or 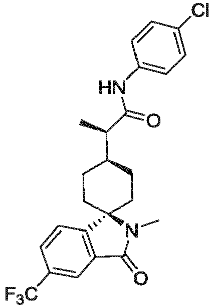 and their pharmaceutically acceptable salts thereof.

In a further preferred embodiment of the compound according to formula (2), the compound is selected from 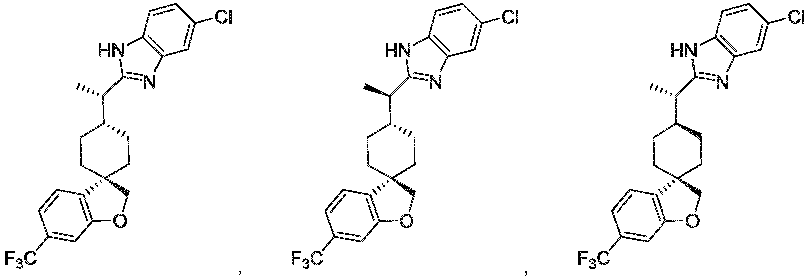 and 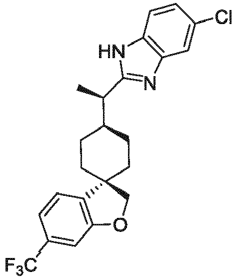 and their pharmaceutically acceptable salts thereof.

The present invention further relates to the compounds according to formulae (1) or (2) for use as a medicament.

The compounds according to formulae (1) or (2) are further for use in the prophylaxis and/or treatment of a disease or condition mediated by indoleamine 2,3-dioxygenase.

In a preferred embodiment in combination with any of the above and below embodiments, the disease or condition mediated by indoleamine 2,3-dioxygenase is selected from the group consisting of cancer, viral and bacterial infections such as HIV infection, hanta virus infection, tuberculosis, leprae, depression, epilepsy, schizophrenia, neurodegenerative diseases such as Parkinson's disease, Alzheimer's disease and Huntington's disease, trauma, age-related cataracts, organ transplantation, cardiovascular disease, endometriosis, type-2 diabetic nephropathy, chronic obstructive pulmonary disease (COPD), osteoporosis, asthma, rheumatoid arthritis, multiple sclerosis, inflammatory bowel disease, psoriasis, and systemic lupus erythematosus.

In a more preferred embodiment in combination with any of the above and below embodiments, the disease or condition mediated by indoleamine 2,3-dioxygenase is cancer.

In a further preferred embodiment in combination with any of the above and below embodiments, the compound according to formulae (1) or (2) can be administered with one or more therapeutic agents for cancer selected from the group consisting of PD-1 agent, PD-L1 agent, CTLA-4 agent, AhR modulator, chemotherapeutic agent, anticancer vaccine, onolytic viruses, TLR agonists, STING agonists,and cytokine therapy as well as other immuno oncology,, or wherein the compound is administered under irradiation therapy.

The invention furthermore relates to pharmaceutical composition comprising a compound according to formulae (1) or (2) and pharmaceutically acceptable excipients.

In the context of the present invention "C₁₋₆-alkyl" means a saturated alkyl chain having 1, 2, 3, 4, 5, or 6 carbon atoms which may be straight chained or branched. Examples thereof include methyl, ethyl, propyl, isopropyl, n-butyl, isobutyl, *tert*-butyl, n-pentyl, isopentyl, neopentyl and hexyl.

The term "halo-C₁₋₆-alkyl" means that one or more hydrogen atoms in the alkyl chain are replaced by a halogen atom which may be the same or different. Preferred example thereof include CHF₂, CF₃, CH₂CH₂Cl, and CH₂CF₃.

A "C_{x-y}-alkylene" means that the respective group is divalent and connects the attached residue with the remaining part of the molecule. X is an integer selected from 0, 1, and 2 and y is an integer selected from 0, 1, 2, and 3. Moreover, in the context of the present invention, "Co-alkylene" is meant to represent a bond. An alkylene group may be straight chained or branched.

A C₃₋₁₀-cycloalkyl group or C₃₋₁₀-carbocycle means a saturated or partially unsaturated mono-, bi-, spiro-, or multicyclic ring system comprising 3, 4, 5, 6, 7, 8, 9, or 10 carbon atoms. Examples include cyclopropyl, cyclobutyl, cyclopentyl, cyclohexyl, cyclohexenyl, bicyclo[2.2.2]octyl, bicyclo[2.2.1]heptyl, adamantyl, spiro[3.3]heptane and pentacyclo[4.2.0.0^{2,5}.0^{3,8}.0^{4,7}]octyl. As an example, a C₃₋₆-cycloalkyl group means a cycloalkyl ring having 3, 4, 5 or 6 carbon atoms. The C₃₋₁₀-cycloalkyl group can be connected to the remainder of the molecule via a bond or the cycloalkyl group may share a carbon at the attachment point with the remainder of the molecule. Illustrative examples of the attachment possibilities are shown below:

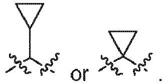

A 3- to 10-membered heterocycloalkyl group means a saturated or partially unsaturated mono-, bi-, tri-, spiro or multicyclic ring system having 3, 4, 5, 6, 7, 8, 9 or 10 ring members. Similarly, a 3- to 6-membered heterocycloalkyl group means a saturated or partially unsaturated mono-, bi-, spiro or multicyclic ring system having 3, 4, 5 or 6 ring members. The heterocycloalkyl comprises up to 5 heteroatoms, such as 1, 2, 3, 4 or 5 heteroatoms, preferably 1, 2 or 3 heteroatoms, more preferably 1 or 2 heteroatoms and most preferably 1 heteroatom, wherein the heteroatoms are independently selected from N, O, S, S(O) and S(O)₂, preferably N, O and S. Examples thereof include epoxidyl, azetidinyl, oxetanyl, pyrrolidinyl, tetrahydrofuranyl, piperidinyl, piperazinyl tetrahydropyranyl, 1,4-dioxanyl, morpholinyl, 4-quinuclidinyl, 1,4-dihydropyridinyl, 2-azaspiro[3.3]heptane and 3,6-dihydro-2H-thiopyranyl. The heterocycloalkyl group can be connected to the remainder of the molecule via a carbon atom or nitrogen atom.

A 5-14-membered mono-, bi- or tricyclic heteroaromatic ring system (within the application also referred to as heteroaryl) containing up to 4 heteroatoms means a monocyclic heteroaromatic ring such as pyrrolyl, imidazolyl, furanyl, thiophenyl, pyridinyl, pyrimidinyl, pyrazinyl, pyrazolyl, oxazolyl, isoxazolyl, triazolyl, oxadiazolyl and thiadiazolyl. It further means a bicyclic ring system wherein the heteroatom(s) may be present in one or both rings including the bridgehead atoms. Examples thereof include quinolinyl, isoquinolinyl, quinoxalinyl, benzimidazolyl, benzisoxazolyl, benzodioxanyl, benzofuranyl, benzoxazolyl, indolyl, indolizinyl, pyrazolo[1,5-a]pyrimidinyl and dibenzo[b,d]furanyl. The nitrogen or sulphur atom of the heteroaryl system may also be optionally oxidized to the corresponding *N*-oxide, S-oxide or S,S-dioxide. If not stated otherwise, the heteroaryl system can be connected via a carbon or nitrogen atom.

Examples for N-linked heterocycles are 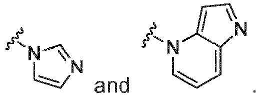 A 6-10-membered mono- or bicyclic aromatic ring system (within the application also referred to as aryl) means an aromatic carbon cycle such as phenyl or naphthyl.

Halogen is selected from fluorine, chlorine, bromine and iodine.

The compounds of the present invention are further intended to include all possible geometric isomers. Cis and trans geometric isomers of the compounds of the present invention are described and may be isolated as a mixture of isomers or as separated forms. A bond in a structure diagram represented by a wavy line " 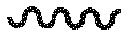 " is intended to indicate that the structure represents the cis or the trans isomer, or a mixture of the cis and trans isomers in any ratio.

Compounds of the present invention also include tautomeric forms. Tautomeric forms result from the swapping of a single bond with an adjacent double bond together with the concomitant migration of a proton.

Any formula or structure given herein, is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, fluorine and chlorine, such as, but not limited to ²H (deuterium, D), ³H (tritium), ¹¹C, ¹³C, ¹⁴C, ¹⁵N, ¹⁸F, ³⁵S, ³⁶Cl and ¹²⁵I. Various isotopically labeled compounds of the present disclosure, for example those into which radioactive isotopes such as ³H, ¹³C and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients. Isotopically labeled compounds of this disclosure and prodrugs thereof can generally be prepared by carrying out the procedures disclosed in the schemes or in the examples and preparations described below by substituting a readily available isotopically labeled reagent for a non-isotopically labeled reagent.

The disclosure also includes "deuterated analogs" of compounds of Formula (I) in which from 1 to n hydrogens attached to a carbon atom is/are replaced by deuterium, in which n is the number of hydrogens in the molecule. Such compounds may exhibit increased resistance to metabolism and thus be useful for increasing the half-life of any compound of Formula (I) when administered to a mammal, e.g. a human. See, for example, Foster in Trends Pharmacol. Sci. 1984:5;524. Such compounds are synthesized by means well known in the art, for example by employing starting materials in which one or more hydrogens have been replaced by deuterium.

Deuterium labelled or substituted therapeutic compounds of the disclosure may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index. An ¹⁸F labeled compound may be useful for PET or SPECT studies.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

The compounds of the present invention can be in the form of a prodrug compound (not part of the invention).

"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods. Other examples of the prodrug are compounds, wherein the carboxylate in a compound of the present invention is, for example, converted into an alkyl-, aryl-, choline-, amino, acyloxymethylester, linolenoylester.

Metabolites of compounds of the present invention are described, but not part of the invention.

Where tautomerism, like e.g. keto-enol tautomerism, of compounds of the present invention or their prodrugs may occur, the individual forms, like e.g. the keto and enol form, are each within the scope of the invention as well as their mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials. Another way to obtain pure enantiomers from racemic mixtures would use enantioselective crystallization with chiral counterions.

The compounds of the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids.

The compounds of the present invention which contain one or more basic groups, i.e. groups which can be protonated can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Further the compounds of the present invention may be present in the form of solvates, such as those which include as solvate water, or pharmaceutically acceptable solvates, such as alcohols, in particular ethanol.

The compounds of the present invention are useful as inhibitors of IDO1. Hence, they are potential therapeutic agents for use in the prophylaxis and/or treatment of IDO1-mediated diseases or conditions such as cancer, viral and bacterial infections such as HIV infection, hanta virus infection, tuberculosis, leprae, depression, epilepsy, schizophrenia, neurodegenerative diseases such as Parkinson's disease, Alzheimer's disease and Huntington's disease, trauma, age-related cataracts, organ transplantation, cardiovascular disease, endometriosis, type-2 diabetic nephropathy, chronic obstructive pulmonary disease (COPD), osteoporosis, asthma, rheumatoid arthritis, multiple sclerosis, inflammatory bowel disease, psoriasis, and systemic lupus erythematosus.

In a preferred embodiment, the compounds are for use in the prophylaxis and/or treatment of cancer.

Examples of cancer types that may be treated using the compounds and compositions described herein include but are not limited to carcinomas, sarcomas, lymphomas and leukemias, germ cell tumors and blastomas, cancer of adrenal gland, bladder, brain, breast, bone, cervix, colorectum, colon, connective tissue, endometrium, esophagus, head, liver, lung, mesothelial lining, muscle, neck, ovary, pancreas, prostate, skin, stomach, testis, thyroid, white blood cell, or glioblastoma, mesothelioma, melanoma, renal cell carcinoma, gastric carcinoma, choriocarcinoma, cutaneous basocellular carcinoma, testicular seminoma and ovarian dysgerminoma. In a recent review by Hornyák et al. examples of such cancer types are given (Hornyák et al. Front Immunol. 2018 Jan 31;9:151).

Furthermore, the present invention provides pharmaceutical compositions comprising at least one compound of the present invention, or a pharmaceutically acceptable salt or solvate thereof as active ingredient together with a pharmaceutically acceptable carrier. "Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable excipient.

The pharmaceutical composition of the present invention may additionally comprise one or more other compounds as active ingredients like a prodrug compound or other therapeutic agents.

Additional therapeutic agents are preferably selected from known cancer therapeutics. Examples thereof include PD-1 agents, PD-L1 agents, CTLA-4 agents, AhR modulators, chemotherapeutic agents, anticancer vaccines, onolytic viruses, TLR agonists, STING agonists, and cytokine therapy as well as other immuno oncology, or wherein the compound is administered under irradiation therapy.

Examples of PD-1 agents include, but are not limited to, Pembrolizumab, Cemiplimab and Nivolumab.

Examples of PD-L1 agents include, but are not limited to, Atezolizumab, Avelumab and Durvalumab.

Examples of CTLA-4 agents include, but are not limited to, Ipilimumab.

Examples of chemotherapeutic agents include, but are not limited to, Cyclophosphamide, Busulfan, Carmustin, Temozolimide, Procarbazin,Trabectedin, Cisplatin, Carboplatin, Methotrexat, Pemetrexed, 6-Mercatopurine, 6-Thioguanine, Cladibine, Clofarabine, Nelarabine, Pentostatine, 5-Fluorouracil, Cytarabine, Gemcitabine, Azacitidine, Vincristine, Vinblastine, Vindesine, Paclitaxel, Docetaxel, Cabazitaxel, Ixabepilone, Eribulin, Estramustine phosphate, Topotecan, Irinotecan, Etoposide, Teniposide, Dactinomycin, Bleomycin, Doxorubicin, Daunorubicin, Epirubicin, Idarubicin, Mitoxantron, all-trans retinoic acid, Bexarotene, As₂O₃, Imatinib, Nilotinib, Dasatinib, Bosutinib, Ponatinib, Erlotinib, Gefitinib, Afatinib, Osimertinib, Lapatinib, Crizotinib, Ceritinib, Axitinib, Cabozantinib, Lanvatinib, Nintedanib, Pazopanib, Regorafenib, Sorafenib, Sunitinib, Ruxolitinib, Dovitinib, Ibrutinib, Idelalisib, Vemurafenib, Dabrafenib, Trametinib, Cobimetinib, Palbociclib, Temsirolismus, Everolimus, Bortezomib, Carfilzomib, Vismodegib, Panobinostat, Olaparib, Venetoclax, Rituximab, Trastuzumab, Pertuzumab, Cetuximab, Panitumumab, Necitumumab, Bevacizumab, Ramucirumab, Olaratumab, Mifamurtide, Elotuzumab, Catumaxomab, Blinatumomab, Rituximab, Daratumumab, Alemtuzumab, Prednisone, Buserelin, Goserelin, Leuprorelin, Histrelin, Triptorelin, Degarelix, Abarelix, Flutamide, Bicalutmide, Enzalutamide, Arbiraterone, Tamoxifen, Toremifen, Exemestane, Letrozole, Anastrozole, Fulvestrant, Thalidomide, Lenalidomide, Pomalidomide,

Examples of anticancer vaccines include, but are not limited to, Hepa-VAC-101 and Sipuleucel-T.

Examples of oncolytic viruses include, but are not limited to, H101, Talimogene laherparepvec.

Examples of Toll like receptor agonists include, but are not limited to, Imiquimod, Resiquimod, monophosphoryl lipid A, BCG , CpG ODNs, Motolimod, GSK1795091 and Telratolimod.

Examples of STING agonists include, but are not limited to, ADU-S100 and MK-1454.

Examples of cytokine therapy include, but are not limited to, IL-2, GM-CSF, IL-12 and IL-10.

Examples of other Immune-Oncology therapeutics that can be used in combination with the compounds of the present invention include, but are not limited to Chimeric antigen receptor, or CAR T-cell therapy, such as Tisagenlecleucel, Axicabtagen Ciloleucel, agents targeting T cell co-stimulatory (e.g. OX40) or co-inhibitory (e.g. LAG3) molecules and immune response modifying enzymes such as Asparaginase or Kynureninase.

The compositions are suitable for oral, rectal, topical, parenteral (including subcutaneous, intramuscular, and intravenous), ocular (ophthalmic), pulmonary (nasal or buccal inhalation) or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, emulsions and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The compounds of the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing IDO mediated conditions for which compounds of the present invention are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

### Abbreviations

Herein and throughout the application, the following abbreviations may be used.
- Ac: acetyl
- AIBN: 2,2'-azobis(2-methylpropionitrile)
- br: broad
- CAN: cerium(IV) ammonium nitrate
- CDI: 1,1'-carbonyldiimidazole
- d: doublet
- DAST: diethylaminosulfur trifluoride
- DEAD: diethyl azodicarboxylate
- DCM: dichloromethane
- DIBAL-H: diisobutylaluminum hydride
- DIPEA: *N,N*-diisopropylethylamine
- DMF: *N,N*-dimethylformamide
- DMSO: dimethyl sulfoxide
- EDC: 1-ethyl-3-(3-dimethylaminopropyl)carbodiimide
- Et: ethyl
- Et₂O: diethyl ether
- EtOAc: ethyl acetate
- HATU: *O*-(7-azabenzotriazol-1-yl)-*N,N,N',N'*-tetramethyluronium hexafluorophosphate
- HOBt: 1-hydroxybenzotriazole
- HPLC: high performance liquid chromatography
- *i*-PrOH: isopropyl alcohol
- LDA: lithium diisopropylamide
- LHMDS: lithium bis(trimethylsilyl)amide
- m: multiplet
- Me: methyl
- MCPBA: 3-chloroperoxybenzoic acid
- Ms: methanesulfonyl
- NCS: *N*-chlorosuccinimide
- PE: petroleum ether
- prep: preparative
- rt: room temperature
- RT: retention time
- SFC: supercritical fluid chromatography
- t: triplet
- TEA: triethylamine
- TFA: trifluoroacetic acid
- THF: tetrahydrofurane

### General Schemes

The compounds of the present invention can be prepared by a combination of methods known in the art including the procedures described in scheme 1 below. The following reaction schemes are only meant to represent examples of the invention and are in no way meant to be a limit of the invention.

Scheme **1** shows the synthesis of Examples of structure **A-5.** Treatment of intermediates of structure **A-1** with *n*-Bu₃SnH leads to spirocyclic intermediates of structure **A-2.** Deprotection with for example hydrochloric acid followed by Horner Wadsworth Emmons reaction of the corresponding ketone leads to intermediates of structure **A-3.** Double bond reduction with for example hydrogen and Pd catalysis affords ester intermediates of structure **A-4.** A sequence of ester saponification and amide coupling with the corresponding amines using for example EDCI/HOBt leads to compounds of structure **A-5.**

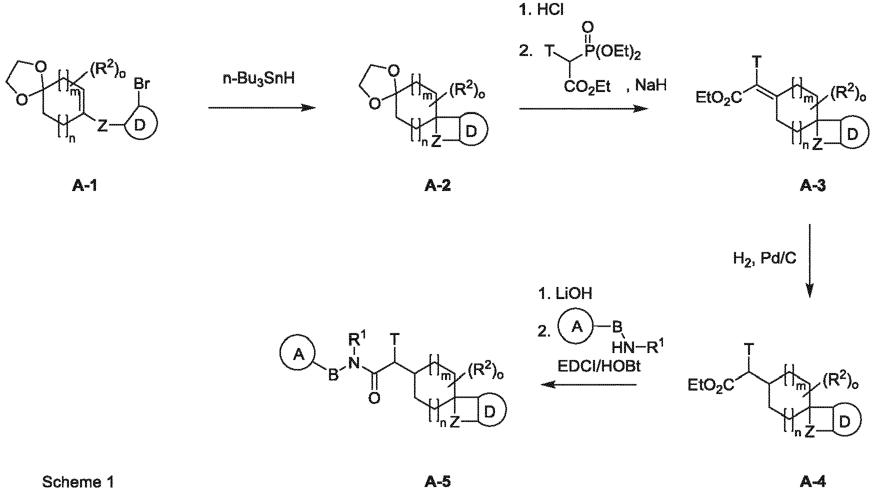

### Intermediate 1: 6-(Trifluoromethyl)-2H-spiro[benzofuran-3,1'-cyclohexan]-4'-one (Int 1)

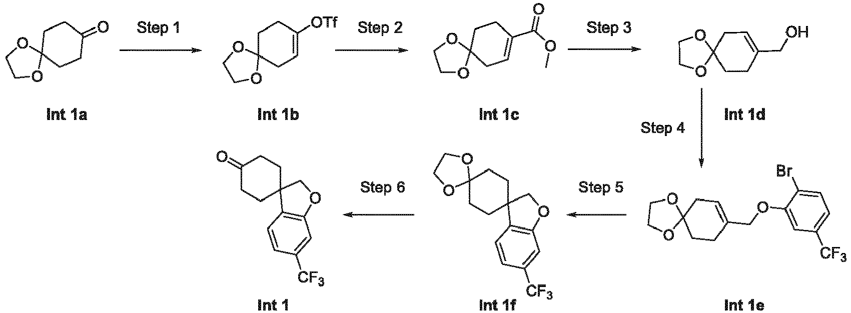

### Step 1: 1,4-Dioxaspiro[4.5]dec-7-en-8-yl trifluoromethanesulfonate (Int 1b)

To a solution of 1,4-dioxaspiro[4.5]decan-8-one **(Int 1a)** (1.20 g, 7.69 mmol) and 1,1,1-trifluoro-*N*-phenyl-*N*-((trifluoromethyl)sulfonyl)methanesulfonamide (3.57 g, 10.00 mmol) in THF (50 mL) under N₂ at -78 °C was added lithium bis(trimethylsilyl)amide in THF (1 M, 10.0 mL, 10.00 mmol). The mixture was stirred at rt overnight, quenched with H₂O (50 mL), and extracted with EtOAc (3 x 50 mL). The combined organic layers were dried over MgSO₄, filtered and concentrated to give the title compound as a brown oil, which was directly used in the next step without purification.

### Step 2: Methyl 1,4-dioxaspiro[4.5]dec-7-ene-8-carboxylate (Int 1c)

A mixture of 1,4-dioxaspiro[4.5]dec-7-en-8-yl trifluoromethanesulfonate **(Int 1b)** (7.69 mmol, crude), Pd(PPh₃)₂Cl₂ (291 mg, 1.11 mmol), and TEA (1.14 g, 11.04 mmol) in MeOH (20 mL) was stirred at rt overnight under a carbon monoxide atmosphere at 5 bar pressure. The reaction mixture was quenched with H₂O (100 mL) and extracted with EtOAc (2 x 100 mL). The combined organic layers were concentrated and the residue was purified by column chromatography on silica gel (PE: EtOAc = 10: 1) to give the title compound as a yellow oil.

### Step 3: (1,4-Dioxaspiro[4.5]dec-7-en-8-yl)methanol (Int 1d)

To a solution of methyl 1,4-dioxaspiro[4.5]dec-7-ene-8-carboxylate **(Int 1c)** (1.20 g, 6.06 mmol) in dry THF (50 mL) was added LiAlH₄ (230 mg, 6.06 mmol) at 0 °C, and the mixture was stirred at rt for 3 h. Then 40 % aqueous NaOH solution (1 mL) was added and the resulting mixture was stirred at rt for 2 h. The mixture was filtered and the filtrate was concentrated. The residue was purified by column chromatography on silica gel (PE: EtOAc = 2:1) to give the title compound as a light yellow oil.

### Step 4: 8-((2-Bromo-5-(trifluoromethyl)phenoxy)methyl)-1,4-dioxaspiro[4.5]dec-7-ene (Int 1e)

To a solution of (1,4-dioxaspiro[4.5]dec-7-en-8-yl)methanol **(Int 1d)** (340 mg, 2.00 mmol), 2-bromo-5-(trifluoromethyl)phenol (482 mg, 2.00 mmol) and PPh₃ (786 mg, 3.00 mmol) in THF (10 mL) was added DEAD (576 mg, 3.00 mmol) under Ar, and the mixture was stirred at rt overnight. The solvent was removed under vacuum and the residue was purified by column chromatography on silica gel (PE: EtOAc = 10: 1) to give the title compound as a yellow oil.

### Step 5: 6-(Trifluoromethyl)-2H-dispiro[benzofuran-3,1'-cyclohexane-4',2"-[1,3]dioxolane] (Int 1f)

A solution of 8-((2-bromo-5-(trifluoromethyl)phenoxy)methyl)-1,4-dioxaspiro[4.5]dec-7-ene **(Int 1e)** (393 mg, 1.00 mmol), *n*-Bu₃SnH (582 mg, 2.00 mmol) and AIBN (33 mg, 0.20 mmol) in toluene (10 mL) was heated to reflux for 3 h. The solvent was removed under vacuum and the residue was purified by column chromatography on silica gel (PE: EtOAc = 10: 1) to afford the title compound as a white solid.

### Step 6: 6-(Trifluoromethyl)-2H-spiro[benzofuran-3,1'-cyclohexan]-4'-one (Int 1)

To a solution of 6-(trifluoromethyl)-2H-dispiro[benzofuran-3,1'-cyclohexane-4',2"-[1,3]dioxolane] **(Int 1f)** (314 mg, 1.00 mmol) in THF (20 mL) was added concentrated aqueous HCI (1 mL, 12 M), and the mixture was stirred at rt overnight. The resulting mixture was extracted with EtOAc (3 x 30 mL), the combined organic layers were dried over MgSO₄, filtered and concentrated in vacuum. The residue was purified by column chromatography on silica gel (PE: EtOAc = 10: 1) to give the title compound as a yellow solid.

### Intermediate 1/1: 6-(Difluoromethyl)-2H-spiro[benzofuran-3,1'-cyclohexan]-4'-one (Int 1/1)

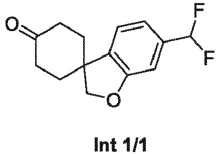

The title compound was prepared similar as described for intermediate **1,** steps 1-6, using in step 4 2-bromo-5-(difluoromethyl)phenol in place of 2-bromo-5-(trifluoromethyl)phenol.

### Intermediate 2: 2-(6-(Difluoromethyl)-2H-spiro[benzofuran-3,1'-cyclohexan]-4'-yl)acetic acid (Int 2)

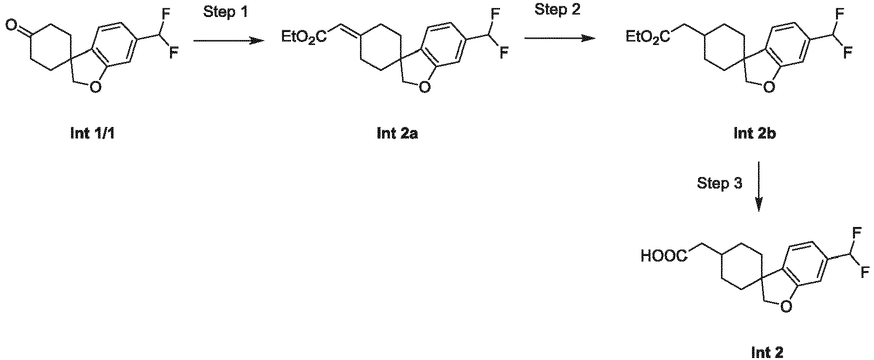

### Step 1: Ethyl 2-(6-(difluoromethyl)-2H-spiro[benzofuran-3,1'-cyclohexan]-4'-ylidene)acetate (Int 2a)

To a solution of ethyl 2-(diethoxyphosphoryl)acetate (1.40 g, 6.20 mmol) in dry THF (50 mL) was added NaH (384 mg, 9.60 mmol, 60% in oil) at 0 °C, and the mixture was stirred for 1 h. 6-(Difluoromethyl)-2*H*-spiro[benzofuran-3,1'-cyclohexan]-4'-one **(Int 1/1,** 1.20 g, 4.80 mmol) was added and the mixture was stirred at rt for 3 h. The mixture was poured into saturated NH₄Cl solution (100 mL) and extracted with EtOAc (3 x 50 mL). The combined organic layers were concentrated to dryness and the residue was purified by column chromatography on silica gel (PE:EtOAc = 10:1) to give the title compound as a colorless oil.

### Step 2: Ethyl 2-(6-(difluoromethyl)-2H-spiro[benzofuran-3,1'-cyclohexan]-4'-yl)acetate (Int 2b)

To a solution of ethyl 2-(6-(difluoromethyl)-2*H*-spiro[benzofuran-3,1'-cyclohexan]-4'-ylidene)acetate **(Int 2a)** (1.30 g, 4.00 mmol) and NiCl₂·6H₂O (2.00 g, 8.00 mmol) in MeOH (50 mL) was added NaBH₄ (1.50 g, 40.00 mmol) at 0 °C, and the mixture was stirred for 3 h. The mixture was poured into water (100 mL) and the mixture was extracted with EtOAc (3 x 30 mL). The combined organic layers were washed with brine (2 x 30 mL), dried over Na₂SO₄ and concentrated to dryness. The residue was purified by column chromatography on silica gel (PE: EtOAc = 20:1) to give the title compound as a colorless oil.

### Step 3: 2-(6-(Difluoromethyl)-2H-spiro[benzofuran-3,1'-cyclohexan]-4'-yl)acetic acid (Int 2)

A solution of ethyl 2-(6-(difluoromethyl)-2*H*-spiro[benzofuran-3,1'-cyclohexan]-4'-yl)acetate **(Int 2b)** (1.20 g, 3.70 mmol) and LiOH (10 mL, 2 M in water) in THF/MeOH (20 mL, 10:1) was refluxed for 2 h. After cooling to rt, the mixture was concentrated. HCI (1.0 mL, 1.0 M) was added. The precipitated solid was filtered and the filter residue was dried in vacuum to give the title compound as white solid.

### Intermediate 10: 2-(2',3'-Dihydrospiro[cyclohexane-1,1'-inden]-4-yl)acetic acid (Int 10)

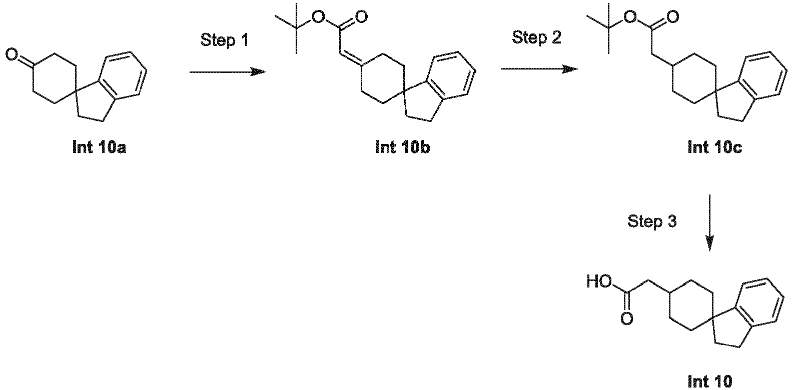

### Step1: tert-Butyl 2-(2',3'-dihydrospiro[cyclohexane-1,1'-inden]-4-ylidene)acetate (Int 10b)

To a solution of *tert*-butyl 2-(diethoxyphosphoryl)acetate (151 mg, 0.60 mmol) in THF (10 mL) was added 60% sodium hydride in mineral oil (24 mg, 0.60 mmol). The mixture was stirred at rt for 30 min. A solution of 2',3'-dihydrospiro[cyclohexane-1,1'-inden]-4-one **(Int 10a)** (100 mg, 0.50 mmol) in THF (5 mL) was added and the mixture was stirred at rt for 1 h. Saturated brine (15 mL) was added and the mixture was extracted with EtOAc (2 x 20 mL). The combined organic layers were dried over anhydrous Na₂SO₄, filtered and concentrated to dryness to give a brown oil. The crude product was triturated with isohexane to give the title compound as a white solid.

### Step 2: tert-Butyl 2-(2',3'-dihydrospiro[cyclohexane-1,1'-inden]-4-yl)acetate (Int 10c)

A mixture of *tert*-butyl 2-(2',3'-dihydrospiro[cyclohexane-1,1'-inden]-4-ylidene)acetate **(Int 10b)** (155 mg, 0.52 mmol) and Pd/C (20 mg) in MeOH (30 mL) was stirred under H₂ atmosphere at rt for 2 h. The mixture was filtered and concentrated to dryness to give the title compound as a white solid.

### Step 3: 2-(2',3'-Dihydrospiro[cyclohexane-1,1'-inden]-4-yl)acetic acid (Int 10)

To a mixture of *tert*-butyl 2-(2',3'-dihydrospiro[cyclohexane-1,1'-inden]-4-yl)acetate **(Int** 10c) (140 mg, 0.47 mmol) in DCM (5 mL) was added TFA (2 mL) and the mixture was stirred at rt for 30 min. The mixture was concentrated to dryness to give the title compound as a brown oil.

### Intermediate 11: 2-(2',3'-Dihydrospiro[cyclohexane-1,1'-inden]-4-yl)propanoic acid (Int 11)

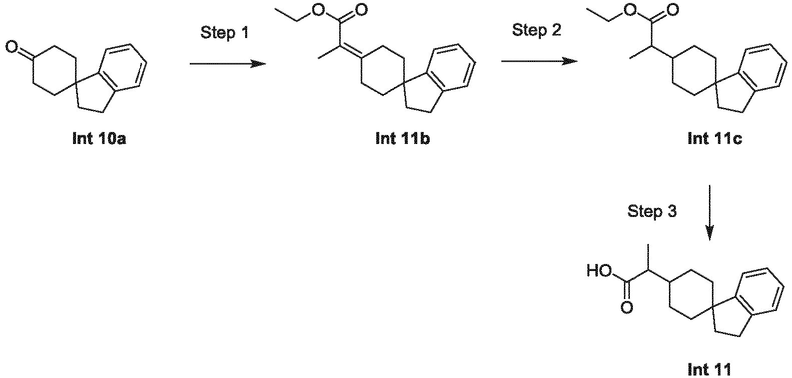

### Step 1: Ethyl 2-(2',3'-dihydrospiro[cyclohexane-1,1'-inden]-4-ylidene)propanoate (Int 11b)

To the solution of ethyl 2-(diethoxyphosphoryl)propanoate (1.40 g, 6.00 mmol) in THF (20 mL) was added NaH (180 mg, 7.50 mol) at 0 °C and the mixture was stirred at rt for 1 h. 2',3'-Dihydrospiro[cyclohexane-1,1'-inden]-4-one **(Int 10a)** (1.00 g, 5.00 mmol) was added and the mixture was stirred at rt for 2 h. The mixture was poured into saturated NH₄Cl solution (20 mL) and extracted with EtOAc (3 x 20 mL). The combined organic layers were concentrated to dryness and the residue was purified by column chromatography on silica gel (PE: EtOAc = 20:1) to give the title compound as a colorless oil.

### Step 2: Ethyl 2-(2',3'-dihydrospiro[cyclohexane-1,1'-inden]-4-yl)propanoate (Int 11c)

A mixture of ethyl 2-(2',3'-dihydrospiro[cyclohexane-1,1'-inden]-4-ylidene)propanoate (racemate) **(Int 11b)** (1.10 g, 3.80 mmol), Pd/C (82 mg, 0.80 mmol) and HCOONH₄ (178 mg, 3.80 mmol) in methanol (50 mL) was stirred at rt overnight. The mixture was filtered and the filtrate was concentrated to dryness to give the title compound as a colorless oil.

### Step 3: 2-(2',3'-Dihydrospiro[cyclohexane-1,1'-inden]-4-yl)propanoic acid (Int 11)

A mixture of ethyl 2-(2',3'-dihydrospiro[cyclohexane-1,1'-inden]-4-yl)propanoate **(Int 11c)** (978 mg, 3.40 mmol) and NaOH (273 mg, 6.80 mmol) in methanol (20 mL) was stirred at rt overnight. The mixture was concentrated to dryness and the residue was dissolved in water. The pH of the mixture was adjusted to pH 1-2 using aqueous HCI (1.0 M). The mixture was extracted with EtOAc (3 x 30 mL). The combined organic layers were concentrated to dryness to give the title compound as a yellow solid.

### Intermediate 12: 2-(6-(Trifluoromethyl)-2H-spiro[benzofuran-3,1'-cyclohexan]-4'-yl)propanoic acid (Int 12)

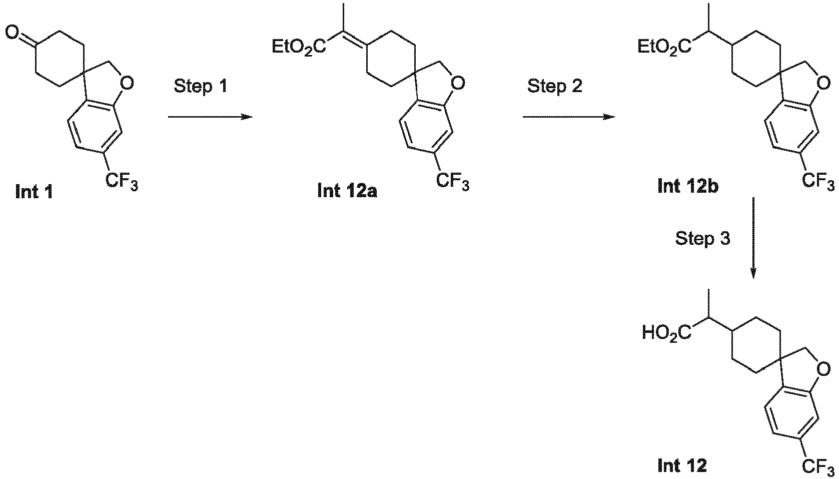

### Step 1: Ethyl 2-(6-(trifluoromethyl)-2H-spiro[benzofuran-3,1'-cyclohexan]-4'-ylidene)propanoate (Int 12a)

To a solution of ethyl 2-(diethoxyphosphoryl)propanoate (285 mg, 1.20 mmol) in dry THF (10 mL) was added NaH (60 mg, 1.50 mmol, 60% in mineral oil) at 0 °C, and the mixture was stirred for 1 h at this temperature. Then 6-(trifluoromethyl)-2*H*-spiro[benzofuran-3,1'-cyclohexan]-4'-one **(Int 1)** (270 mg, 1.00 mmol) was added and the mixture was stirred at rt for 3 h. The mixture was poured into saturated NH₄Cl solution (10 mL) and extracted by EtOAc (3 x 20 mL). The combined organic layers were concentrated and the residue was purified by column chromatography on silica gel (PE:EtOAc = 10:1) to give the title compound as a colorless oil.

### Step 2: Ethyl 2-(6-(triftuoromethyt)-2H-spiro[benzofuran-3,1'-cyctohexan]-4'-yl)propanoate (Int 12b)

To a solution of ethyl 2-(6-(trifluoromethyl)-2*H*-spiro[benzofuran-3,1'-cyclohexan]-4'-ylidene)propanoate **(Int 12a)** (340 mg, 1.00 mmol) and NiCl₂•6H₂O (237 mg, 1.00 mmol) in MeOH (20 mL) was added NaBH₄ (190 mg, 5.00 mmol) at 0 °C, and the mixture was stirred for 3 h. The mixture was poured into water (30 mL) and the resulting mixture was extracted with EtOAc (3 x 30 mL). The combined organic layers were washed with brine (2 x 30 mL), dried over Na₂SO₄, and concentrated. The residue was purified by column chromatography on silica gel (PE: EtOAc = 20:1) to give the title compound as a colorless oil.

### Step 3: 2-(6-(Trifluoromethyl)-2H-spiro[benzofuran-3,1'-cyclohexan]-4'-yl)propanoic acid (Int 12)

A solution of ethyl 2-(6-(trifluoromethyl)-2*H*-spiro[benzofuran-3,1'-cyclohexan]-4'-yl)propanoate **(Int 12b)** (342 mg, 1.00 mmol) and LiOH (120 mg, 5.00 mmol) in a mixture of THF/MeOH (10 mL, 1:1) was heated to reflux for 2 h. After cooling to rt, aqueous HCI (1 mL, 1M) was added. The formed solid was filtered off and dried under vacuum to give the title compound as a white solid.

### Intermediate 13: 2-Methyl-2-(6-(trifluoromethyl)-2H-spiro[benzofuran-3,1'-cyclohexan]-4'-yl)propanoic acid (Int 13)

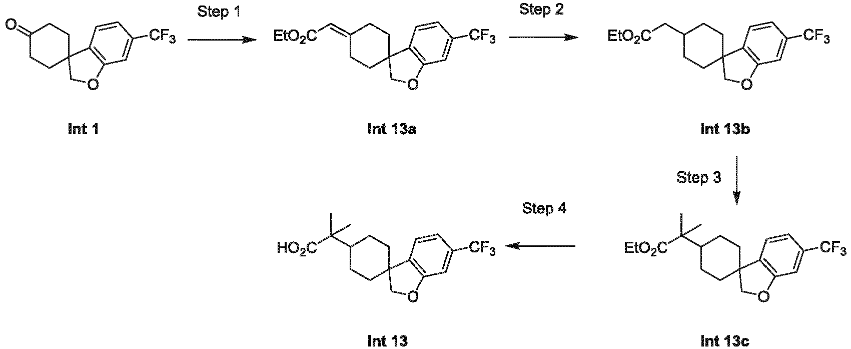

### Step1: Ethyl 2-(6-(trifluoromethyl)-2H-spiro[benzofuran-3,1 '-cyclohexan]-4'-ylidene)acetate (Int 13a)

To a solution of ethyl 2-(diethoxyphosphoryl)acetate (2.69 g, 12.00 mmol) in dry THF (100 mL) was added NaH (600 mg, 15.00 mmol, 60% in mineral oil) at 0 °C, and the mixture was stirred for 1 h at 0 °C. Then 6-(trifluoromethyl)-2*H*-spiro[benzofuran-3,1'-cyclohexan]-4'-one **(Int 1)** (2.70 g, 10.00 mmol) was added and the mixture was stirred at rt for 3 h. The mixture was poured into saturated NH₄Cl solution (100 mL) and extracted with EtOAc (3 x 50 mL). The combined organic layers were concentrated to dryness and the residue was purified by column chromatography on silica gel (PE:EtOAc = 10:1) to give the title compound as colorless oil.

### Step 2: Ethyl 2-(6-(trifluoromethyl)-2H-spiro[benzofuran-3,1'-cyclohexan]-4'-yl)acetate (Int 13b)

To a solution of ethyl 2-(6-(trifluoromethyl)-2*H*-spiro[benzofuran-3,1'-cyclohexan]-4'-ylidene)acetate **(Int 13a)** (2.90 g, 8.50 mmol) and NiCl₂·6H₂O (2.00 g, 8.50 mmol) in MeOH (100 mL) was added NaBH₄ (1.50 g, 40.00 mmol) at 0 °C, and the mixture was stirred for 3 h. Water (100 mL) was added and the mixture was extracted with EtOAc (3 x 30 mL). The combined organic layers were washed with brine (2 x 30 mL), dried over Na₂SO₄, filtered and concentrated to dryness. The residue was purified by column chromatography on silica gel (PE:EA = 20:1) to give the title compound as a colorless oil.

### Step 3: Ethyl 2-methyl-2-(6-(trifluoromethyl)-2H-spiro[benzofuran-3,1'-cyclohexan]-4'-yl)propanoate (Int 13c)

To a solution of ethyl 2-(6-(trifluoromethyl)-2*H*-spiro[benzofuran-3,1'-cyclohexan]-4'-yl)acetate **(Int 13b)** (1.00 g, 2.90 mmol) in THF (20 mL) was added LHMDS (6.4 mL, 1 M in THF) at -78 °C and the mixture was stirred at -78 °C for 1 h. lodomethane (1.00 g, 7.20 mmol) was added and the mixture was allowed to warm up to rt and stirred overnight. Water (100 mL) was added and the mixture was extracted with EtOAc (3 x 30 mL). The combined organic layers were washed by brine (2 x 30 mL), dried over Na₂SO₄, filtered and concentrated. The residue was purified by column chromatography on silica gel (PE:EA = 20:1) to give the title compound as a colorless oil.

### Step 4: 2-Methyl-2-(6-(trifluoromethyl)-2H-spiro[benzofuran-3,1'-cyclohexan]-4'-yl)propanoic acid (Int 13)

A solution of ethyl 2-methyl-2-(6-(trifluoromethyl)-2H-spiro[benzofuran-3,1'-cyclohexan]-4'-yl)propanoate **(Int 13c)** (370 mg, 1.00 mmol) and LiOH·H₂O (84 mg, 2.00 mmol) in a mixture of THF and MeOH (70 mL, 1:1) was heated to reflux for 2 h. After cooling to rt, HCI (25 mL, 1M) was added and the mixture was filtered. The filter cake was dried under high vacuum to give the title compound as a white solid.

### Intermediate 14: 5'-(Trifluoromethyl)-2'H-spiro[cyclohexane-1,3'-furo[2,3-b]pyridin]-4-one (Int 14)

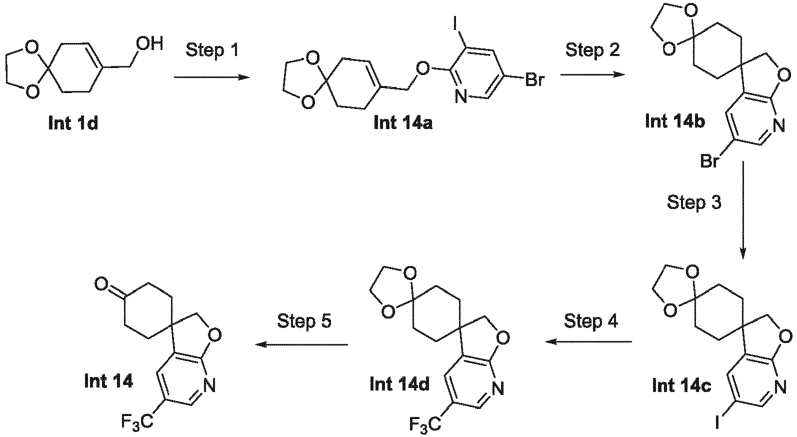

### Step 1: 2-((1,4-Dioxaspiro[4.5]dec-7-en-8-yl)methoxy)-5-bromo-3-iodopyridine (Int 14a)

To a solution of (1,4-dioxaspiro[4.5]dec-7-en-8-yl)methanol **(Int 1d)** (6.80 g, 40.00 mmol) in DMF (70 mL) was added NaH (3.20 g, 80.00 mmol, 60 % in oil) at 0 °C and the mixture was stirred at 0 °C for 1h. 5-Bromo-2-fluoro-3-iodopyridine (12.00 g, 40.00 mmol) was added and stirring was continued at rt for 2 h. The mixture was poured into water (1 L) and extracted by EtOAc (3 x 100 mL). The combined organic layers were washed with brine (100 mL), dried over Na₂SO₄, filtered and concentrated. The residue was purified by column chromatography on silica gel (PE:EtOAc = 5:1) to afford the title compound as a white solid.

### Step 2: 5-Bromo-2H-dispiro[furo[2,3-b]pyridine-3,1'-cyclohexane-4',2"-[1,3]dioxolane] (Int 14b)

A solution of 2-((1,4-dioxaspiro[4.5]dec-7-en-8-yl)methoxy)-5-bromo-3-iodopyridine **(Int 14a,**16.1 g, 35.7 mmol), *n*-Bu₃SnH (10.4 g, 35.7 mmol) and AIBN (590 mg, 3.6 mmol) in toluene (320 mL) was heated to reflux overnight. After cooling to rt the solvent was removed under reduced pressure and the residue was purified by column chromatography on silica gel (PE:EtOAc = 5:1) to afford the title compound as a white solid.

### Step 3: 5-Iodo-2H-dispiro[furo[2,3-b]pyridine-3,1'-cyclohexane-4',2"-[1,3]dioxolane] (Int 14c)

To a solution of 5-bromo-2*H*-dispiro[furo[2,3-b]pyridine-3,1'-cyclohexane-4',2"-[1,3]dioxolane] **(Int 14b,** 4.50 g, 13.80 mmol) in 1,4-dioxane (20 mL) was added KI (4.60 g, 27.60 mmol), Cul (266.00 mg, 1.40 mmol) and N¹,N²-dimethylethane-1,2-diamine (123 mg, 1.40 mmol). The mixture was heated to reflux overnight under N₂. Then the mixture was concentrated and the residue was purified by column chromatography on silica gel (PE:EtOAc = 5:1) to afford the title compound as a white solid.

### Step 4: 5-(Trifluoromethyl)-2H-dispiro[furo[2,3-b]pyridine-3,1'-cyclohexane-4',2"-[1,3]dioxolane] (Int 14d)

KF (1.50 g, 25.90 mmol) and Cul (4.90 g, 25.90 mmol) were placed in a flask and were flame-heated under gentle shaking and high vacuum until a greenish color appeared. After cooling to rt a solution of 5-iodo-2*H*-dispiro[furo[2,3-b]pyridine-3,1'-cyclohexane-4',2"-[1,3]dioxolane] **(Int 14c,** 3.70 g, 10.00 mmol) and (trifluoromethyl)trimethylsilane (3.70 g, 25.90 mmol) in DMF (50 mL) was added and the mixture was stirred at 80 °C for 2 h. The mixture was poured into water (500 mL) and extracted with EtOAc (3 x 100 mL). The combined organic layers were washed with brine (100 mL), dried over Na₂SO₄, filtered and concentrated. The residue was purified by column chromatography on silica gel (PE:EtOAc = 5:1) to afford the title compound as a white solid.

### Step 5: 5'-(Trifluoromethyl)-2'H-spiro[cyclohexane-1,3'-furo[2,3-b]pyridin]-4-one (Int 14)

To a solution of 5-(trifluoromethyl)-2*H*-dispiro[furo[2,3-b]pyridine-3,1'-cyclohexane-4',2"-[1,3]dioxolane] **(Int 14d,** 630 mg, 2.00 mmol) in THF (10 mL) was added concentrated aqueous HCI solution (2 mL, 12 N), and the mixture was stirred at rt overnight. Water (30 mL) was added and the mixture was extracted with EtOAc (3 x 30 mL). The combined organic layers were washed with brine (30 mL), dried over Na₂SO₄, filtered and concentrated. The residue was purified by column chromatography on silica gel (PE:EtOAc = 5:1) to afford the title compound as a white solid.

### Intermediate 15: 6'-(Trifluoromethyl)-2'H-spiro[cyclohexane-1,3'-furo[2,3-b]pyridin]-4-one (Int 15)

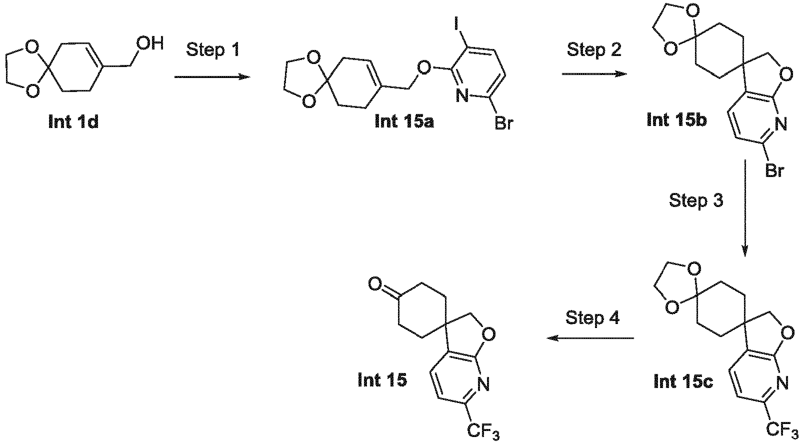

### Step 1: 2-((1,4-Dioxaspiro[4.5]dec-7-en-8-yl)methoxy)-6-bromo-3-iodopyridine (Int 15a)

To a solution of (1,4-dioxaspiro[4.5]dec-7-en-8-yl)methanol **(Int 1d,** 6.80 g, 40.00 mmol) in DMF (70 mL) was added NaH (3.20 g, 80.00 mmol, 60 % in oil) at 0 °C and the mixture was stirred at 0 °C for 1h, then 6-bromo-2-fluoro-3-iodopyridine (12.00 g, 40.00 mmol) was added and stirring was continued at rt for 2 h. The mixture was poured into water (1 L) and extracted by EtOAc (3 x 100 mL). The combined organic layers were washed with brine (100 mL), dried over Na₂SO₄, filtered and concentrated. The residue was purified by column chromatography on silica gel (PE:EtOAc = 5:1) to afford the title compound as a white solid.

### Step 2: 6-Bromo-2H-dispiro[furo[2,3-b]pyridine-3,1'-cyclohexane-4',2"-[1,3]dioxolane] (Int 15b)

A solution of 2-((1,4-dioxaspiro[4.5]dec-7-en-8-yl)methoxy)-6-bromo-3-iodopyridine **(Int 15a,** 16.1 g, 35.7 mmol), *n*-Bu₃SnH (10.4 g, 35.7 mmol) and AIBN (590 mg, 3.6 mmol) in toluene (320 mL) was heated to reflux overnight. After cooling to rt the solvent was removed under reduced pressure and the residue was purified by column chromatography on silica gel (PE:EtOAc = 5:1) to afford the title compound as a white solid.

### Step 3: 6-(Trifluoromethyl)-2H-dispiro[furo[2,3-b]pyridine-3,1'-cyclohexane-4',2"-[1,3]dioxolane] (Int 15c)

KF (1.50 g, 25.90 mmol) and Cul (4.90 g, 25.90 mmol) were placed in a flask and were flame-heated under gentle shaking and high vacuum until a greenish color appeared. After cooling to rt a solution of 5-iodo-2*H*-dispiro[furo[2,3-b]pyridine-3,1'-cyclohexane-4',2"-[1,3]dioxolane] **(Int 14c,** 3.70 g, 10.00 mmol) and (trifluoromethyl)trimethylsilane (3.70 g, 25.90 mmol) in DMF (50 mL) was added and the mixture was stirred at 80 °C for 2 h. The mixture was poured into water (500 mL) and extracted with EtOAc (3 x 100 mL). The combined organic layers were washed with brine (100 mL), dried over Na₂SO₄, filtered and concentrated. The residue was purified by column chromatography on silica gel (PE:EtOAc = 5:1) to afford the title compound as a white solid.

### Step 4: 6'-(Trifluoromethyl)-2'H-spiro[cyclohexane-1,3'-furo[2,3-b]pyridin]-4-one (Int 15)

To the solution of 6-(trifluoromethyl)-2*H*-dispiro[furo[2,3-b]pyridine-3,1'-cyclohexane-4',2"-[1,3]dioxolane] **(Int 15c,** 1.00 g, 3.10 mmol) in DMF (10 mL) was added Cul (4.90 g, 25.90 mmol) and methyl 2,2-difluoro-2-(fluorosulfonyl)acetate (1.20 g, 6.20 mmol). The mixture was stirred at 100 °C for 1 h under microwave irradiation. The mixture was poured into water (100 mL) and extracted by EtOAc (3 x 50 mL). The combined organic layers were washed with brine (50 mL), dried over Na₂SO₄, filtered and concentrated. The residue was purified by column chromatography on silica gel (PE:EtOAc = 5:1) to afford the title compound as a white solid.

### Intermediate 16: 2-(5'-(Trifluoromethyl)-2'H-spiro[cyclohexane-1,3'-furo[2,3-b]pyridin]-4-yl)propanoic acid (Int 16)

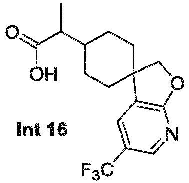

2-(5'-(Trifluoromethyl)-2'*H*-spiro[cyclohexane-1,3'-furo[2,3-b]pyridin]-4-yl)propanoic acid **(Int 16)** was prepared from 5'-(trifluoromethyl)-2'*H*-spiro[cyclohexane-1,3'-furo[2,3-b]pyridin]-4-one **(Int 14)** using a similar procedure as described for 2-(6-(trifluoromethyl)-2*H*-spiro[benzofuran-3,1'-cyclohexan]-4'-yl)propanoic acid **(Int 12).**

### Intermediate 17: 2-(6'-(Trifluoromethyl)-2'H-spiro[cyclohexane-1,3'-furo[2,3-b]pyridin]-4-yl)propanoic acid (Int 17)

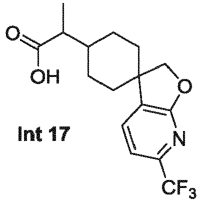

2-(6'-(Trifluoromethyl)-2'*H*-spiro[cyclohexane-1,3'-furo[2,3-b]pyridin]-4-yl)propanoic acid **(Int 17)** was prepared from 6'-(trifluoromethyl)-2'*H*-spiro[cyclohexane-1,3'-furo[2,3-b]pyridin]-4-one **(Int 15)** using a similar procedure as described for 2-(6-(trifluoromethyl)-2*H*-spiro[benzofuran-3,1'-cyclohexan]-4'-yl)propanoic acid **(Int 12).**

### Intermediate 18: 2'-Methyl-5'-(trifluoromethyl)spiro[cyclohexane-1,1'-isoindoline]-3',4-dione (Int 18)

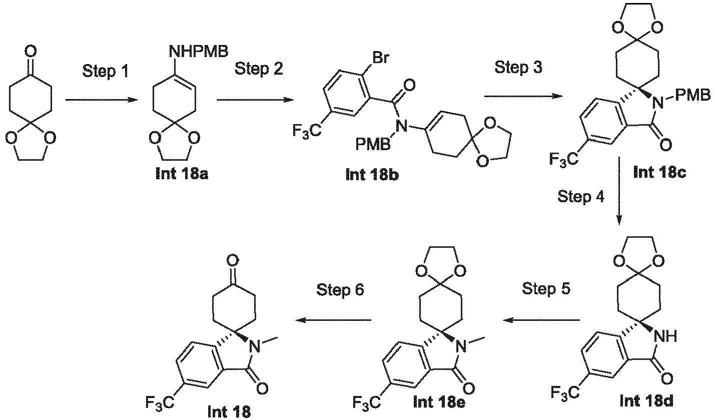

### Step 1: N-(4-Methoxybenzyl)-1,4-dioxaspiro[4.5]dec-7-en-8-amine (Int 18a)

A mixture of 1,4-dioxaspiro[4.5]decan-8-one (2.90 g, 18.60 mmol), (4-methoxyphenyl)methanamine (2.55 mg, 18.60 mmol) and 4A molecular sieves (20.00 g) in DCM (150 mL) was refluxed overnight. The mixture was used directly in the next step.

### Step 2: 2-Bromo-N-(4-methoxybenzyl)-N-(1,4-dioxaspiro[4.5]dec-7-en-8-yl)-5-(trifluoromethyl)benzamide (Int 18b)

To a mixture of crude *N*-(4-methoxybenzyl)-1,4-dioxaspiro[4.5]dec-7-en-8-amine **(Int 18a,** 18.60 mmol) above was added 2-bromo-5-(trifluoromethyl)benzoyl chloride (5.35 g, 18.60 mmol) and DIPEA (4.80 g, 37.2 mmol) in DCM at 0 °C. The mixture was stirred overnight and then filtered. The filtrate was concentrated and the residue was purified by column chromatography on silica gel (PE:EtOAc = 5:1) to give the title compound as a yellow oil.

### Step 3: 2-(4-Methoxybenzyl)-5-(trifluoromethyl)dispiro[isoindoline-1,1'-cyclohexane-4',2"-[1,3]dioxolan]-3-one (Int 18c)

A mixture of 2-bromo-*N*-(4-methoxybenzyl)-*N*-(1,4-dioxaspiro[4.5]dec-7-en-8-yl)-5-(trifluoromethyl)benzamide **(Int 18b,** 4.90 g, 9.32 mmol), *n*-Bu₃SnH (5.40 g, 18.64 mmol) and AIBN (305 mg, 1.86 mmol) in toluene (20 mL) was refluxed overnight. The resulting mixture was concentrated. The residue was purified by column chromatography on silica gel (PE:EtOAc= 5:1) to give the title compound as a yellow solid.

### Step 4: 5-(Trifluoromethyl)dispiro[isoindoline-1,1'-cyclohexane-4',2"-[1,3]dioxolan]-3-one (Int 18d)

To a solution of 2-(4-methoxybenzyl)-5-(trifluoromethyl)dispiro[isoindoline-1,1'-cyclohexane-4',2"-[1,3]dioxolan]-3-one **(Int 18c,** 4.47 g, 10.00 mmol) in acetonitrile (50 mL) was added CAN (16.50 g, 30.00 mmol) in water (20 mL) at 0 °C. The mixture was stirred for 3 h and water (100 mL) was added. The mixture was filtered and the filtrate was extracted with EtOAc (3 x 100 mL). The organic phase was concentrated and the residue was purified by column chromatography on silica gel (PE:EtOAc = 3:1) to afford the title compound as a yellow solid.

### Step 5: 2-Methyl-5-(trifluoromethyl)dispiro[isoindoline-1,1'-cyclohexane-4',2"-[1,3]dioxolan]-3-one (Int 18e)

To a solution of 5-(trifluoromethyl)dispiro[isoindoline-1,1'-cyclohexane-4',2"-[1,3]dioxolan]-3-one **(Int 18d,** 981 mg, 3.00 mmol) in THF (20 mL) was added NaH (180 mg, 4.5 mmol, 60 % dispersed in mineral oil) at 0 °C and then the mixture was stirred for 1 h. To the mixture above was added Mel (852 mg, 6.0 mmol) and the mixture was stirred at rt overnight. The mixture was poured into water (20 mL) and then extracted with EtOAc (3 x 20 mL). The organic phase was concentrated and the residue was purified by column chromatography on silica gel (PE:EtOAc = 3:1) to afford the title compound as a light yellow solid.

### Step 6: 2'-Methyl-5'-(trifluoromethyl)spiro[cyclohexane-1,1'-isoindoline]-3',4-dione (Int 18)

To a solution of HClO₄ (10 mL, 70% aqueous) was added 2-methyl-5-(trifluoromethyl)dispiro[isoindoline-1,1'-cyclohexane-4',2"-[1,3]dioxolan]-3-one **(Int 18e,** 716 mg, 2.10 mmol) at 0 °C and then the mixture was stirred for 3 h. The mixture was added to a solution of NaHCO₃ (30 mL) and extracted with EtOAc (3 x 30 mL). The organic phase was concentrated and the residue was purified by column chromatography on silica gel (PE:EtOAc = 3:1) to afford the title compound as a white solid.

### Intermediate 19: 2-(2'-Methyl-3'-oxo-5'-(trifluoromethyl)spiro[cyclohexane-1,1'-isoindolin]-4-yl)propanoic acid (Int 19)

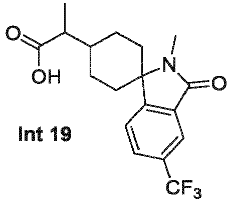

2-(2'-Methyl-3'-oxo-5'-(trifluoromethyl)spiro[cyclohexane-1,1'-isoindolin]-4-yl)propanoic acid **(Int 19)** was prepared from 2'-methyl-5'-(trifluoromethyl)spiro[cyclohexane-1,1'-isoindoline]-3',4-dione **(Int 18)** using a similar procedure as described for 2-(6-(trifluoromethyl)-2H-spiro[benzofuran-3,1'-cyclohexan]-4'-yl)propanoic acid **(Int 12).**

### Intermediate 20: 2-(6-(Trifluoromethyl)-2H-spiro[benzofuran-3,1'-cyclohexan]-4'-yl)butanoic acid (Int 20)

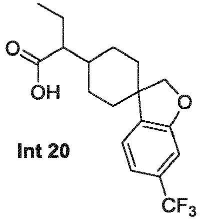

2-(6-(Trifluoromethyl)-2H-spiro[benzofuran-3,1'-cyclohexan]-4'-yl)butanoic acid **(Int 20)** was prepared from 6-(trifluoromethyl)-2*H*-spiro[benzofuran-3,1'-cyclohexan]-4'-one **(Int 1)** by a similar procedure as described for 2-(6-(trifluoromethyl)-2*H*-spiro[benzofuran-3,1'-cyclohexan]-4'-yl)propanoic acid **(Int 12),** using ethyl 2-(diethoxyphosphoryl)butanoate instead of ethyl 2-(diethoxyphosphoryl)propanoate.

### Examples 1b, 1c: Separate isomers of N-(4-chlorophenyl)-2-(2',3'-dihydrospiro[cyclohexane-1,1'-inden]-4-yl)acetamide (Examples 1b, 1c)

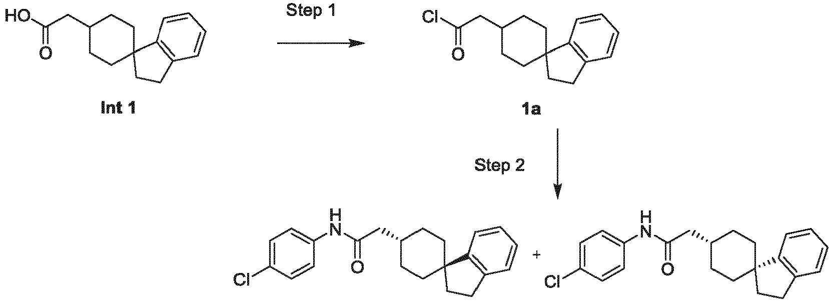

### Examples 1b, 1c, configurations not assigned

### Step 1: 2-(2',3'-Dihydrospiro[cyclohexane-1,1'-inden]-4-yl)acetyl chloride (mixture of isomers) (1a)

A mixture of 2-(2',3'-dihydrospiro[cyclohexane-1,1'-inden]-4-yl)acetic acid **(Int 1)** (114 mg, 0.47 mmol) in SOCl₂ (5 mL) was stirred at rt for 2 h. The mixture was concentrated to dryness to give the title compound as a yellow oil which was used in the next step without further purification.

### Step 2: Separate isomers of N-(4-chlorophenyl)-2-(2',3'-dihydrospiro[cyclohexane-1,1'-inden]-4-yl)acetamide (1b, 1c)

To a mixture of 2-(2',3'-dihydrospiro[cyclohexane-1,1'-inden]-4-yl)acetyl chloride (mixture of isomers) (1a) (122 mg, 0.46 mmol) in DCM (5 mL) and 4-chloroaniline (71 mg, 0.56 mmol), TEA (70 mg, 0.69 mmol) were added. The mixture was stirred at 70 °C for 5 h. The mixture was concentrated to dryness and the residue was purified by preparative HPLC (ODS C18, column 21.2 x 250 mm, 10 µm, 0.05% TFA in water/acetonitrile with gradient 65-95% acetonitrile, flow rate 30 mL/min) *to afford the two* cis/trans isomers of *N*-(4-chlorophenyl)-2-(2',3'-dihydrospiro[cyclohexane-1,1'-inden]-4-yl)acetamide as white solids.

First eluting isomer of *N*-(4-chlorophenyl)-2-(2',3'-dihydrospiro[cyclohexane-1,1'-inden]-4-yl)acetamide **(Example 1b)** (RT of 2.60 min with the following analytical HPLC conditions: column X-Bridge C18 (4.6 x 50 mm, 3.5 µm), mobile phase gradient 5% - 95% acetonitrile in 10 mM aqueous NH₄HCO₃, flow rate 2.0 mL/min, column temperature 40°C): ¹H NMR (500 MHz, DMSO-d₆): δ ppm 10.04 (s, 1H), 7.65-7.63 (m, 2H), 7.35-7.33 (m, 2H), 7.18-7.09 (m, 4H), 2.84-2.81 (m, 2H), 2.26-2.25 (m, 2H), 1.94-1.84 (m, 3H), 1.70-1.68 (m, 2H), 1.64-1.58 (m, 2H), 1.50-1.48 (m, 2H), 1.26-1.18 (m, 2H). MS (ESI): m/z 354.2 [M+H]⁺.

Second eluting isomer of *N*-(4-chlorophenyl)-2-(2',3'-dihydrospiro[cyclohexane-1,1'-inden]-4-yl)acetamide **(Example 1c)** (RT of 2.62 min with the following analytical HPLC conditions: column X-Bridge C18 (4.6 x 50 mm, 3.5 µm), mobile phase gradient 5% - 95% acetonitrile in 10 mM aqueous NH₄HCO₃, flow rate 2.0 mL/min, column temperature 40°C): ¹H NMR (500 MHz, DMSO-d₆): δ ppm 10.08 (s, 1H), 7.65-7.63 (m, 2H), 7.38-7.34 (m, 3H), 7.20-7.11 (m, 3H), 2.83-2.80 (m, 2H), 2.48-2.46 (m, 2H), 2.17-2.13 (m, 1H), 1.92-1.89 (m, 2H), 1.79-1.66 (m, 4H), 1.52-1.48 (m, 2H), 1.40-1.36 (m, 2H). MS 354.0 (ESI): m/z [M+H]⁺.

### Examples 1/1a, 1/1b: Separate isomers of N-(4-chloro-2-fluorophenyl)-2-methyl-2-(6-(trifluoromethyl)-2H-spiro[benzofuran-3,1'-cyclohexan]-4'-yl)propanamide (1/1 a, 1/1 b)

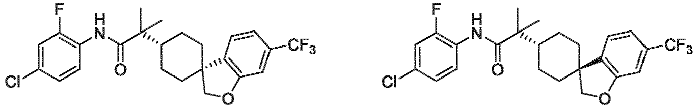

### Examples 1/1a, 1/1b, configurations not assigned

The title compounds were prepared simlar as described for Examples **1b** and **1c**, steps 1 and 2, using in step 1 2-methyl-2-(6-(trifluoromethyl)-2*H*-spiro[benzofuran-3,1'-cyclohexan]-4'-yl)propanoic acid **(Int 13)** in place of 2-(2',3'-dihydrospiro[cyclohexane-1,1'-inden]-4-yl)acetic acid **(Int 1).**

First eluting isomer of *N*-(4-chloro-2-fluorophenyl)-2-methyl-2-(6-(trifluoromethyl)-2*H-*spiro[benzofuran-3,1'-cyclohexan]-4'-yl)propanamide **(Example 1/1a)** (RT of 0.96 min with the following SFC conditions: column AD (Daicel) (20 x 250 mm), mobile phase CO₂/0.2% NH₃ in Methanol 50:50, flow rate 80 mL/min, column temperature 35°C): ¹H NMR (500 MHz, DMSO-d₆): δ ppm 9.15 (s, 1H), 7.60 (d, J = 7.8 Hz, 1H), 7.48 (dd, J = 10.2, 2.3 Hz, 1H), 7.44 (t, J = 8.5 Hz, 1H), 7.27 (dd, J₁ = 8.6, J₂ =1.5 Hz, 1H), 7.22 (d, J = 7.8 Hz, 1H), 7.12 (s, 1H), 4.25 (s, 2H), 1.95-1.76 (m, 3H), 1.67-1.49 (m, 6H), 1.23 (s, 6H). MS (ESI): m/z 468.1 [M-H]-.

Second eluting isomer of *N*-(4-chloro-2-fluorophenyl)-2-methyl-2-(6-(trifluoromethyl)-2*H-*spiro[benzofuran-3,1'-cyclohexan]-4'-yl)propanamide **(Example 1/1b)** (RT of 1.54 min with the following SFC conditions: column AD (Daicel) (20 x 250 mm), mobile phase CO₂/0.2% NH₃ in Methanol 50:50, flow rate 80 mL/min, column temperature 35°C): ¹H NMR (500 MHz, DMSO-d₆): δ ppm 9.13 (s, 1H), 7.47 (dd, J₁ = 10.2, J₂ = 2.2 Hz, 1H), 7.43 (t, J = 8.4 Hz, 1H), 7.37 (d, J = 7.7 Hz, 1H), 7.26 (dd, J₁ = 8.6, J₂ = 1.4 Hz, 1H), 7.21 (d, J = 7.6 Hz, 1H), 7.08 (s, 1H), 4.46 (s, 2H), 1.90-1.75 (m, 3H), 1.74-1.59 (m, 4H), 1.25-1.10 (m, 8H). MS (ESI): m/z 468.1 [M-H]-.

### Examples 2a, 2b: Separate racemic cis/trans isomers of N-(4-chlorophenyl)-2-(2',3'-dihydrospiro[cyclohexane-1,1'-inden]-4-yl)propanamide (2a, 2b)

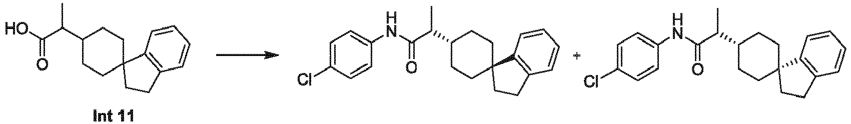

### Examples 2a, 2b, racemates, cis/trans configurations not assigned

A mixture of 2-(2',3'-dihydrospiro[cyclohexane-1,1'-inden]-4-yl)propanoic acid (mixture of cis/trans isomers, racemates) **(Int 11)** (789 mg, 3.00 mmol), 4-chloroaniline (466 mg, 3.60 mmol), HATU (1.70 g, 4.50 mmol) and DIPEA (774 mg, 6.00 mmol) in DMF (10 mL) was stirred at rt overnight. The mixture was poured into water (20 mL) and extracted with EtOAc (3 x 30 mL). The combined organic layers were concentrated to dryness and the residue was purified by column chromatography on silica gel (PE:EtOAc = 10:1)followed by preparative chiral-SFC (CHIRALCEL OD, column 30 x 250 mm, 5 µm, CO₂/MeOH-EtOH 1:1, flow rate 45 mL/min) to afford the two racemic cis/trans isomers of *N*-(4-Chlorophenyl)-2-(2',3'-dihydrospiro[cyclohexane-1,1'-inden]-4-yl)propanamide.

First eluting racemic cis/trans isomer of *N*-(4-chlorophenyl)-2-(2',3'-dihydrospiro[cyclohexane-1,1'-inden]-4-yl)acetamide **(Example 2a)** with RT = 3.87 min and 4.12 min, collected in one fraction. ¹H NMR (400 MHz, DMSO-d₆): δ ppm 10.07 (s, 1H), 7.67-7.64 (m, 2H), 7.42-7.40 (m, 1H), 7.36-7.33 (m, 2H), 7.19-7.12 (m, 3 H), 2.82-2.78 (m, 2H), 2.71-2.67 (m, 1H), 1.91-1.79 (m, 3H), 1.75-1.67 (m, 3H), 1.60-1.49 (m, 3H), 1.40-1.33 (m, 2H), 1.12 (d, J = 6.8 Hz, 3H). MS (ESI): 368.1 [M+H]⁺.

Second eluting racemic cis/trans isomer of *N*-(4-chlorophenyl)-2-(2',3'-dihydrospiro[cyclohexane-1,1'-inden]-4-yl)acetamide **(Example 2b)** with RT = 4.78 min and 5.99 min, collected in one fraction. ¹H NMR (400 MHz, DMSO-d₆): δ ppm 9.99 (s, 1H), 7.67-7.64 (m, 2H), 7.35-7.32 (m, 2H), 7.17-7.07 (m, 4H), 2.83-2.79 (m, 2H), 2.28-2.24 (m, 1H), 1.92-1.88 (m, 2H), 1.83-1.79 (m, 1H), 1.59-1.46 (m, 6H), 1.30-1.23 (m, 2H), 1.10-1.15 (m, 3H). MS (ESI): 368.3 [M+H]⁺.

### Examples 3b, 3c, 3d, 3e: Separate isomers of N-(4-chlorophenyl)-2-(6-(trifluoromethyl)-2H-spiro[benzofuran-3,1'-cyclohexan]-4'-yl)propanamide (3a), (3b, 3c, 3d, 3e)

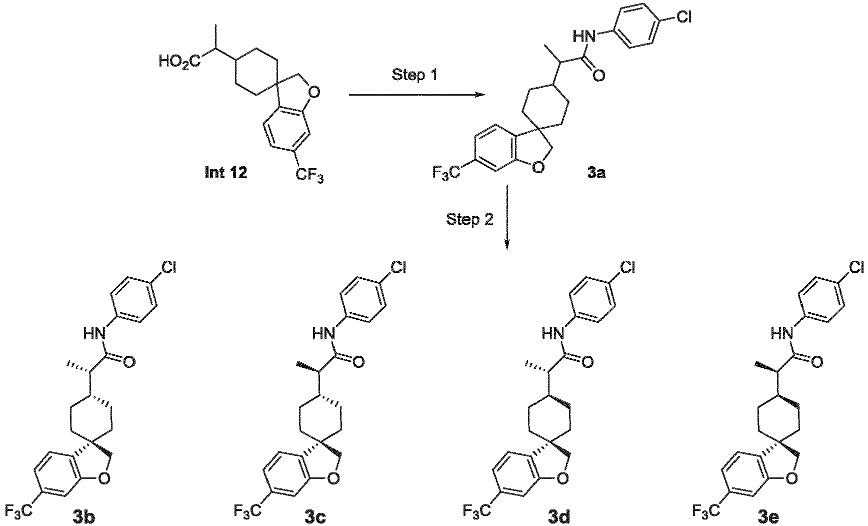

### Examples 3b, 3c, 3d, 3e

### Step 1: N-(4-Chlorophenyl)-2-(6-(trifluoromethyl)-2H-spiro[benzofuran-3,1'-cyclohexan]-4'-yl)propanamide, mixture of isomers (3a)

A mixture of 2-(6-(trifluoromethyl)-2*H*-spiro[benzofuran-3,1'-cyclohexan]-4'-yl)propanoic acid **(Int 12)** (328 mg, 1.00 mmol), 4-chloroaniline (127 mg, 1.00 mmol), EDCI (384 mg, 2.00 mmol), HOBt (270 mg, 2.00 mmol) and DIPEA (516 mg, 4.00 mmol) in DMF (10 mL) was stirred at rt overnight. The mixture was poured into water (20 mL) and extracted with DCM (3 x 20 mL). The combined organic layers were concentrated and the residue was purified by column chromatography on silica gel (PE:EtOAc = 10:1) to give the title compound as a white solid.

### Step 2: Separate isomers of N-(4-chlorophenyl)-2-(6-(trifluoromethyl)-2H-spiro[benzofuran-3,1'-cyclohexan]-4'-yl)propanamide (Examples 3b, 3c, 3d, 3e)

The mixture of isomers *N*-(4-Chlorophenyl)-2-(6-(trifluoromethyl)-2*H*-spiro[benzofuran-3,1'-cyclohexan]-4'-yl)propanamide **(3a)** was separated by preparative chiral-SFC (stationary phase CHIRALCEL AD (Daicel), mobile phase CO₂/MeOH) to afford the four separated isomers of *N*-(4-chlorophenyl)-2-(6-(trifluoromethyl)-2H-spiro[benzofuran-3,1'-cyclohexan]-4'-yl)propanamide **(Examples 3b, 3c, 3d, 3e).**

(S)-*N*-(4-Chlorophenyl)-2-((3*S*,4'*r*)-6-(trifluoromethyl)-2*H*-spiro[benzofuran-3,1'-cyclohexan]-4'-yl)propanamide **(Example 3b),** (first eluting isomer of the mixture **3a,** RT of 1.37 min with the following analytical SFC-Conditions: column CHIRALPAK AD-3 (4.6 x 100 mm, 3µm), mobile phase 70% CO₂/ 30% MeOH, flow rate 2 mL/min, column temperature 35°C): ¹H NMR (400 MHz, DMSO-d₆): δ ppm 10.1 (s, 1H), 7.68-7.63 (m, 2H), 7.60-7.57 (m, 1H), 7.38-7.32 (m, 2H), 7.25-7.21 (m, 1H), 7.11 (s, 1H), 4.37-4.29 (m, 2H), 2.64-2.57 (m, 1H), 1.90-1.67 (m, 4H), 1.61-1.47 (m, 5H), 1.14 (d, J=6.8 Hz, 3H). MS (ESI): 438.1 [M+H]+.

(R)-*N*-(4-Rhlorophenyl)-2-((3*R*,4'*r*)-6-(trifluoromethyl)-2*H*-spiro[benzofuran-3,1'-cyclohexan]-4'-yl)propanamide **(Example 3c),** (second eluting isomer of the mixture **3a,** RT of 1.90 min with the following analytical SFC-Conditions: column CHIRALPAK AD-3 (4.6 x 100 mm, 3µm), mobile phase 70% CO₂/ 30% MeOH, flow rate 2 mL/min, column temperature 35°C): ¹H NMR (400 MHz, DMSO-d₆): δ ppm 10.1 (s, 1H), 7.67-7.64 (m, 2H), 7.60-7.57 (m, 1H), 7.37-7.33 (m, 2H), 7.25-7.21 (m, 1H), 7.11 (s, 1H), 4.37-4.29 (m, 2H), 2.64-2.58 (m, 1H), 1.90-1.68 (m, 4H), 1.61-1.48 (m, 5H), 1.14 (d, J=6.8 Hz, 3H). MS (ESI): 438.1 [M+H]⁺.

(S)-*N*-(4-Chlorophenyl)-2-((3*R*,4'*s*)-6-(trifluoromethyl)-2*H*-spiro[benzofuran-3,1'-cyclohexan]-4'-yl)propanamide **(Example 3d),** (third eluting isomer of the mixture 3a, RT of 2.18 min with the following analytical SFC-Conditions: column CHIRALPAK AD-3 (4.6 x 100 mm, 3µm), mobile phase 70% CO₂/ 30% MeOH, flow rate 2 mL/min, column temperature 35°C): ¹H NMR (400 MHz, DMSO-d₆): δ ppm 10.0 (s, 1H), 7.67-7.63 (m, 2H), 7.42-7.39 (m, 1H), 7.36-7.32 (m, 2H), 7.22-7.18 (m, 1H), 7.07 (s, 1H), 4.48-4.41 (m, 2H), 2.27-2.22 (m, 1H), 1.87-1.82 (m, 1H), 1.75-1.58 (m, 6H), 1.18-0.97 (m, 5H). MS (ESI): 438.2 [M+H]⁺.

(*R*)-*N*-(4-chlorophenyl)-2-((3*S*,4'*s*)-6-(trifluoromethyl)-2*H*-spiro[benzofuran-3,1'-cyclohexan]-4'-yl)propanamide **(Example 3e),** (fourth eluting isomer of the mixture **3a,** RT of 2.72 min with the following analytical SFC-Conditions: column CHIRALPAK AD-3 (4.6 x 100 mm, 3µm), mobile phase 70% CO₂/ 30% MeOH, flow rate 2 mL/min, column temperature 35°C): ¹H NMR (400 MHz, DMSO-d₆): δ ppm 10.0 (s, 1H), 7.67-7.63 (m, 2H), 7.42-7.39 (m, 1H), 7.36-7.33 (m, 2H), 7.22-7.18 (m, 1H), 7.07 (s, 1H), 4.48-4.41 (m, 2H), 2.28-2.22 (m, 1H), 1.87-1.82 (m, 1H), 1.75-1.57 (m, 6H), 1.18-0.98 (m, 5H). MS (ESI): 438.2 [M+H]⁺. The absolute configuration of **Example 3e** was determined by X-ray diffraction analysis.

### Stereoselective route for (S)-N-(4-Chlorophenyl)-2-((3R,4's)-6-(trifluoromethyl)-2H-spiro[benzofuran-3,1'-cyclohexan]-4'-yl)propanamide (Example 3d) and (S)-N-(4-Chlorophenyl)-2-((3S,4'r)-6-(trifluoromethyl)-2H-spiro[benzofuran-3,1'-cyclohexan]-4'-yl)propanamide (Example 3b)

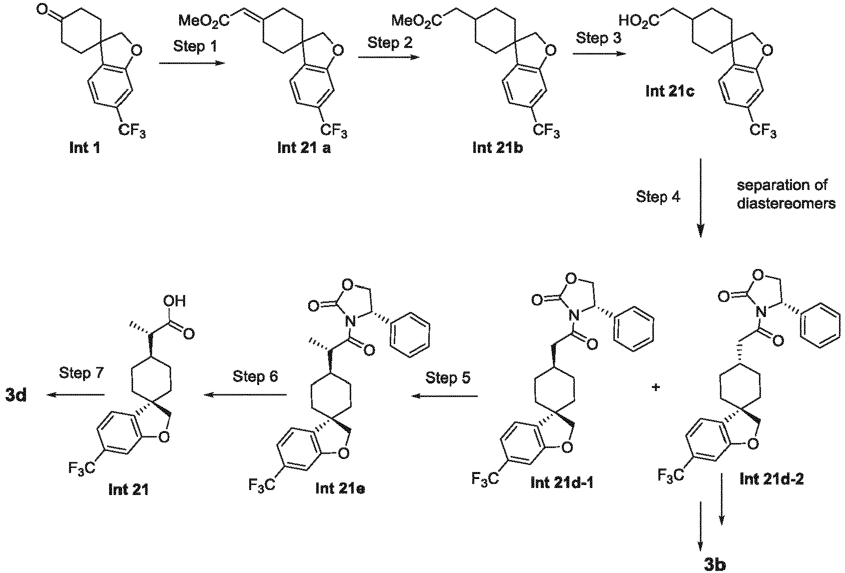

### Example 3d: (S)-N-(4-Chlorophenyl)-2-((3R,4's)-6-(trifluoromethyl)-2H-spiro[benzofuran-3,1'-cyclohexan]-4'-yl)propanamide (3d)

### Step 1: Methyl 2-(6-(trifluoromethyl)-2H-spiro[benzofuran-3,1'-cyclohexan]-4'-ylidene)acetate (Int 21a)

To a solution of 6-(trifluoromethyl)-2*H*-spiro[benzofuran-3,1'-cyclohexan]-4'-one **(Int 1,** 1.35 g, 5.00 mmol) in dry THF (25 mL) was added NaH (300 mg, 15.00 mmol, 60 % dispersion in mineral oil) at 0 °C, and the mixture was stirred for 1 h. Then methyl 2-(diethoxyphosphoryl)acetate (1.76 g, 12.00 mmol) was added and the mixture was stirred at rt for 3 h. The mixture was poured into saturated NH₄Cl solution (50 mL) and extracted with EtOAc (3 x 50 mL). The combined organic phase was concentrated and the residue was purified by column chromatography on silica gel (PE:EtOAc = 10:1) to afford the title compound as a colorless oil.

### Step 2: Methyl 2-(6-(trifluoromethyl)-2H-spiro[benzofuran-3,1'-cyclohexan]-4'-yl)acetate (Int 21b)

To a solution of methyl 2-(6-(trifluoromethyl)-2*H*-spiro[benzofuran-3,1'-cyclohexan]-4'-ylidene)acetate **(Int 21a,** 1.68 g, 5.00 mmol) and NiCl₂•6H₂O (1.18 g, 5.00 mmol) in MeOH (30 mL) was added NaBH₄ (950 mg, 25.00 mmol) at 0 °C, and the mixture was stirred for 3 h. Then it was poured into water (30 mL) and the resulting mixture was extracted with EtOAc (3 x 50 mL). The combined organic phase was washed by brine (2 x 30 mL), dried over Na₂SO₄ and concentrated. The residue was purified by column chromatography on silica gel (PE:EtOAc = 20:1) to afford the title compound as a colorless oil.

### Step 3: 2-(6-(Trifluoromethyl)-2H-spiro[benzofuran-3,1'-cyclohexan]-4'-yl)aceticacid (Int 21c)

A solution of methyl 2-(6-(trifluoromethyl)-2*H*-spiro[benzofuran-3,1'-cyclohexan]-4'-yl)acetate **(Int 21b**,1.64 g, 5.00 mmol) and LiOH (600 mg, 25.00 mmol) in a mixture of THF/MeOH (30 mL, 10:1) was refluxed for 2 h. After cooling to rt, aqueous HCl (1.0 mL, 1.0 N) was added, the formed solid was filtered and dried under vacuum to afford the title compound as a white solid.

### Step 4: (S)-4-Phenyl-3-(2-((3R,4's)-6-(trifluoromethyl)-2H-spiro[benzofuran-3,1'-cyclohexan]-4'-yl)acetyl)oxazolidin-2-one (Int 21d-1) and (S)-4-phenyl-3-(2-((3S,4'r)-6-(trifluoromethyl)-2H-spiro[benzofuran-3,1'-cyclohexan]-4'-yl)acetyl)oxazolidin-2-one (21d-2)

A mixture of 2-(6-(trifluoromethyl)-2*H*-spiro[benzofuran-3,1'-cyclohexan]-4'-yl)acetic acid **(Int 21c**,1.35 g, 4.30 mmol), EEDQ (1.06 g, 4.30 mmol), (S)-4-phenyloxazolidin-2-one (701 mg, 4.30 mmol) and LiCl (180 mg, 4.30 mmol) in EA (20 mL) was stirred and heated to reflux overnight. After cooling to rt the mixture was poured into aqueous HCl (20 mL, 1.0 N) and extracted by EtOAc (3 x 20 mL). The combined organic phase was concentrated and the residue was purified by column chromatography on silica gel (PE: EtOAc = 10: 1) to afford the title compounds as a white solid. This mixture was subjected to separation by chiral SFC (CHIRALCEL OD, CO₂/MeOH, 60/40%) to afford (S)-4-phenyl-3-(2-((3*R*,4'*s*)-6-(trifluoromethyl)-2*H*-spiro[benzofuran-3,1'-cyclohexan]-4'-yl)acetyl)oxazolidin-2-one **(Int 21d-1,** second eluting isomer) and (S)-4-phenyl-3-(2-((3*S*,4'*r*)-6-(trifluoromethyl)-2*H*-spiro[benzofuran-3,1'-cyclohexan]-4'-yl)acetyl)oxazolidin-2-one **(Int 21d-2,** first eluting isomer).

### Step 5: (S)-4-Phenyl-3-((S)-2-((3R,4's)-6-(trifluoromethyl)-2H-spiro[benzofuran-3,1'-cyclohexan]-4'-yl)propanoyl)oxazolidin-2-one (Int 21e)

To a solution of (*S*)-4-phenyl-3-(2-((3*R*,4'*s*)-6-(trifluoromethyl)-2*H*-spiro[benzofuran-3,1'-cyclohexan]-4'-yl)acetyl)oxazolidin-2-one **(Int 21d-1,** 502 mg, 1.09 mmol) in dry THF (15 mL) was added LHMDS (1.6 mL, 1.61 mmol, 1.0 M in THF) at -40 °C and the mixture was stirred for 0.5 h. Mel (774 mg, 5.45 mmol) was added and the mixture was stirred for further 2 h at -40 °C. Saturated aqueous NH₄Cl solution (3.0 mL) was added at -40 °C and the mixture was concentrated. The residue was purified by column chromatography on silica gel (PE:EtOAc = 10:1) to afford the title compound as a white solid.

### Step 6: (S)-2-((3R,4's)-6-(Trifluoromethyl)-2H-spiro[benzofuran-3,1'-cyclohexan]-4'-yl)propanoic acid (Int 21)

To a mixture of (*S*)-4-phenyl-3-((*S*)-2-((3*R*,4'*s*)-6-(trifluoromethyl)-2*H*-spiro[benzofuran-3,1'-cyclohexan]-4'-yl)propanoyl)oxazolidin-2-one **(Int 21e,** 206 mg, 0.44 mmol) and LiOH (36 mg, 0.88 mmol) in THF (10 mL) was added H₂O₂ (1.0 mL, 30 % in H₂O). The mixture was stirred at 0 °C for 2 h and then poured into saturated aqueous NaHSO₃ solution. The resulting mixture was extracted by EtOAc (3 x 10 mL) and the combined organic layers were washed with brine, dried over anhydrous Na₂SO₄, filtered and concentrated. The residue was purified by prep-HPLC to give the title compound as a white solid

### Step 7: (S)-N-(4-Chlorophenyl)-2-((3R,4's)-6-(trifluoromethyl)-2H-spiro[benzofuran-3,1 '-cyclohexan]-4'-yl)propanamide (Example 3d)

A mixture of (*S*)-2-((3*R*,4'*s*)-6-(trifluoromethyl)-2*H*-spiro[benzofuran-3,1'-cyclohexan]-4'-yl)propanoic acid **(Int 21,** 40 mg, 0.12 mmol), 4-chloroaniline (15 mg, 0.12 mmol), EDCI (29 mg, 0.15 mmol), HOBt (20 mg, 0.15 mmol) and DIEA (28 mg, 0.22 mmol) in DMF (10 mL) was stirred at rt overnight. The mixture was poured into water (20 mL) and extracted with DCM (3 x 20 mL). The combined organic phase was concentrated and the residue was purified by prep-HPLC to give the title compound as a white solid. ¹H NMR (400 MHz, DMSO-d₆): δ ppm 10.0 (s, 1H), 7.69-7.61 (m, 2H), 7.42-7.40 (m, 1H), 7.37 - 7.30 (m, 2H), 7.22-7.18 (m, 1H), 7.07 (s, 1H), 4.48-4.41 (m, 2H), 2.27-2.21 (m, 1H), 1.87-1.82 (m, 1H), 1.75-1.58 (m, 6H), 1.18-0.95 (m, 5H). MS (ESI): 438.2 [M+H]⁺.

### Example 3b: (S)-N-(4-Chlorophenyl)-2-((3S,4'r)-6-(trifluoromethyl)-2H-spiro[benzofuran-3,1'-cyclohexan]-4'-yl)propanamide (3b)

Starting from (*S*)-4-phenyl-3-(2-((3*S*,4'*r*)-6-(trifluoromethyl)-2*H*-spiro[benzofuran-3,1'-cyclohexan]-4'-yl)acetyl)oxazolidin-2-one (**21 d-2**), (*S*)-*N*-(4-chlorophenyl)-2-((3*S*,4'*r*)-6-(trifluoromethyl)-2*H*-spiro[benzofuran-3,1'-cyclohexan]-4'-yl)propanamide **(Example 3b)** was obtained following a similar procedure as described for (*S*)-*N*-(4-chlorophenyl)-2-((3*R*,4'*s*)-6-(trifluoromethyl)-2*H*-spiro[benzofuran-3,1'-cyclohexan]-4'-yl)propanamide **(Example 3d).** ¹H NMR (400 MHz, DMSO-d₆): δ ppm 10.1 (s, 1H), 7.70-7.62 (m, 2H), 7.60-7.57 (m, 1H), 7.40-7.30 (m, 2H), 7.25-7.21 (m, 1H), 7.11 (s, 1H), 4.37-4.29 (m, 2H), 2.65-2.55 (m, 1H), 1.90-1.67 (m, 4H), 1.62-1.47 (m, 5H), 1.14 (d, J = 6.8 Hz, 3H). MS (ESI): 438.1 [M+H]⁺.

### Stereoselective route for Examples 3e and 3c

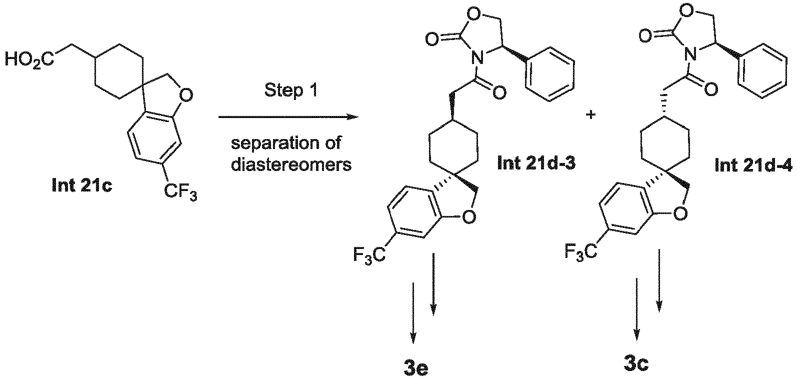

### Step 1: (R)-4-Phenyl-3-(2-((3R,4's)-6-(trifluoromethyl)-2H-spiro[benzofuran-3,1 '-cyclohexan]-4'-yl)acetyl)oxazolidin-2-one (Int 21d-3) and (R)-4-phenyl-3-(2-((3S,4'r)-6-(trifluoromethyl)-2H-spiro[benzofuran-3,1'-cyclohexan]-4'-yl)acetyl)oxazolidin-2-one (Int 21d-4)

(*R*)-4-Phenyl-3-(2-((3R,4's)-6-(trifluoromethyl)-2*H*-spiro[benzofuran-3,1'-cyclohexan]-4'-yl)acetyl)oxazolidin-2-one **(Int 13d-3)** and (R)-4-phenyl-3-(2-((3S,4'r)-6-(trifluoromethyl)-2*H*-spiro[benzofuran-3,1'-cyclohexan]-4'-yl)acetyl)oxazolidin-2-one **(Int 21d-4)** were obtained from 2-(6-(trifluoromethyl)-2*H*-spiro[benzofuran-3,1'-cyclohexan]-4'-yl)acetic acid **(Int 21c)** following the procedure described for (S)-4-phenyl-3-(2-((3R,4's)-6-(trifluoromethyl)-2*H*-spiro[benzofuran-3,1'-cyclohexan]-4'-yl)acetyl)oxazolidin-2-one **(Int 21d-1**) and (*S*)-4-phenyl-3-(2-((3*S*,4'*r*)-6-(trifluoromethyl)-2*H*-spiro[benzofuran-3,1'-cyclohexan]-4'-yl)acetyl)oxazolidin-2-one **(21d-2),** using (R)-4-phenyloxazolidin-2-one in place of (S)-4-phenyloxazolidin-2-one. (R)-4-Phenyl-3-(2-((3R,4's)-6-(trifluoromethyl)-2*H*-spiro[benzofuran-3,1'-cyclohexan]-4'-yl)acetyl)oxazolidin-2-one **(Int 21d-3)** was the second eluting isomer and (*R*)-4-phenyl-3-(2-((3*S*,4'*r*)-6-(trifluoromethyl)-2*H-*spiro[benzofuran-3,1'-cyclohexan]-4'-yl)acetyl)oxazolidin-2-one **(21d-4)** was the first eluting isomer. The structure of (*R*)-4-phenyl-3-(2-((3*S*,4'*r*)-6-(trifluoromethyl)-2*H-*spiro[benzofuran-3,1'-cyclohexan]-4'-yl)acetyl)oxazolidin-2-one **(21d-4)** was confirmed by X-ray diffraction analysis.

### Example 3e: (R)-N-(4-Chlorophenyl)-2-((3S,4's)-6-(trifluoromethyl)-2H-spiro[benzofuran-3,1'-cyclohexan]-4'-yl)propanamide (3e)

Starting from (*R*)-4-phenyl-3-(2-((3*S*,4'*s*)-6-(trifluoromethyl)-2*H*-spiro[benzofuran-3,1'-cyclohexan]-4'-yl)acetyl)oxazolidin-2-one **(21d-3),** (*R*)-*N*-(4-chlorophenyl)-2-((3*S*,4'*s*)-6-(trifluoromethyl)-2*H*-spiro[benzofuran-3,1'-cyclohexan]-4'-yl)propanamide **(Example 3e)** was obtained following a similar procedure as described for (*S*)-*N*-(4-chlorophenyl)-2-((3*R*,4'*s*)-6-(trifluoromethyl)-2*H*-spiro[benzofuran-3,1'-cyclohexan]-4'-yl)propanamide **(Example 3d).** ¹H NMR (400 MHz, DMSO-d₆): δ ppm 10.0 (s, 1H), 7.70-7.59 (m, 2H), 7.42-7.39 (m, 1H), 7.38-7.28 (m, 2H), 7.22-7.18 (m, 1H), 7.07 (s, 1H), 4.48-4.41 (m, 2H), 2.28-2.22 (m, 1H), 1.89-1.77 (m, 1H), 1.75-1.57 (m, 6H), 1.25-0.95 (m, 5H). MS (ESI): 438.2 [M+H]⁺.

### Example 3c: (R)-N-(4-Chlorophenyl)-2-((3R,4'r)-6-(trifluoromethyl)-2H-spiro[benzofuran-3,1'-cyclohexan]-4'-yl)propanamide (3c)

Starting from (*R*)-4-phenyl-3-(2-((3*R*,4'*r*)-6-(trifluoromethyl)-2*H*-spiro[benzofuran-3,1'-cyclohexan]-4'-yl)acetyl)oxazolidin-2-one **(21d-4),** (*R*)-*N*-(4-chlorophenyl)-2-((3*R*,4'*r*)-6-(trifluoromethyl)-2*H*-spiro[benzofuran-3,1'-cyclohexan]-4'-yl)propanamide **(Example 3c)** was obtained following a similar procedure as described for (*S*)-*N*-(4-chlorophenyl)-2-((3*R*,4'*s*)-6-(trifluoromethyl)-2*H*-spiro[benzofuran-3,1'-cyclohexan]-4'-yl)propanamide **(Example 3d).** ¹H NMR (400 MHz, DMSO-d₆): δ ppm 10.1 (s, 1H), 7.67-7.64 (m, 2H), 7.60-7.57 (m, 1H), 7.38-7.31 (m, 2H), 7.25-7.21 (m, 1H), 7.11 (s, 1H), 4.37-4.29 (m, 2H), 2.65-2.56 (m, 1H), 1.90-1.68 (m, 4H), 1.62-1.46 (m, 5H), 1.14 (d, J=6.8 Hz, 3H). MS (ESI): 438.1 [M+H]+.

### Examples 4b, 4c, 4d, 4e: Separate isomers of N-(4-chlorophenyl)-2-(5'-(trifluoromethyl)-2'H-spiro[cyclohexane-1,3'-furo[2,3-b]pyridin]-4-yl)propanamide (4a), (4b, 4c, 4d, 4e)

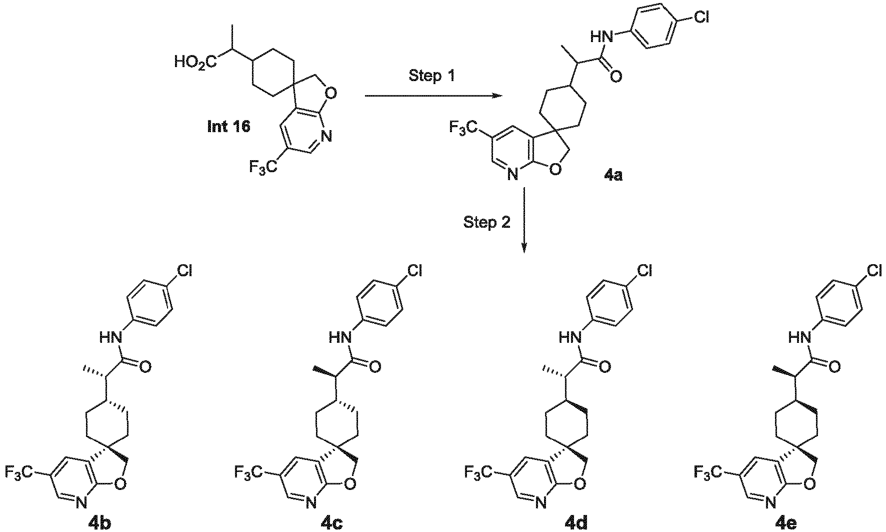

### Examples 4b, 4c, 4d, 4e, configurations not assigned

### Step 1: N-(4-chlorophenyl)-2-(5'-(trifluoromethyl)-2'H-spiro[cyclohexane-1,3'-furo[2,3-b]pyridin]-4-yl)propanamide (4a)

*N*-(4-chlorophenyl)-2-(5'-(trifluoromethyl)-2'*H*-spiro[cyclohexane-1,3'-furo[2,3-b]pyridin]-4-yl)propanamide **(4a)** was obtained from 2-(5'-(trifluoromethyl)-2'*H*-spiro[cyclohexane-1,3'-furo[2,3-b]pyridin]-4-yl)propanoic acid **(Int 16)** following a similar procedure as described for *N*-(4-chlorophenyl)-2-(6-(trifluoromethyl)-2*H*-spiro[benzofuran-3,1'-cyclohexan]-4'-yl)propanamide **(3a).**

### Step 2: Separate isomers of N-(4-chlorophenyl)-2-(5'-(trifluoromethyl)-2'H-spiro[cyclohexane-1,3'-furo[2,3-b]pyridin]-4-yl)propanamide (4a), (Examples 4a, 4b, 4c, 4d)

The separate isomers of *N*-(4-chlorophenyl)-2-(5'-(trifluoromethyl)-2'*H-*spiro[cyclohexane-1,3'-furo[2,3-b]pyridin]-4-yl)propanamide **(4a), (4b, 4c, 4d, 4e**) were prepared following a similar procedure as described for the separate isomers of *N*-(4-chlorophenyl)-2-(6-(trifluoromethyl)-2*H*-spiro[benzofuran-3,1'-cyclohexan]-4'-yl)propanamide **(3a), (3b, 3c, 3d, 3e**), using the following preparative chiral-SFC conditions: stationary phase CHIRALCEL OD (Daicel), mobile phase CO₂/MeOH (0.2% ammonia, 7N in MeOH), 80/20.

First eluting isomer of *N*-(4-chlorophenyl)-2-(5'-(trifluoromethyl)-2'*H*-spiro[cyclohexane-1,3'-furo[2,3-b]pyridin]-4-yl)propanamide **(Example 4b),** (RT of 3.38 min with the following analytical SFC-Conditions: column CHIRALPAK OD-H (4.6 x 100 mm, 5µm), mobile phase 90% CO₂/ 10% MeOH (0.2% ammonia, 7N in MeOH), flow rate 4 mL/min, column temperature 40°C): ¹H NMR (400 MHz, DMSO-d₆): δ ppm 10.04 (s, 1H), 8.40 (s, 1H), 7.96 (d, J = 2.0 Hz, 1H), 7.66 (d, J = 8.9 Hz, 2H), 7.35 (d, J = 8.9 Hz, 2H), 4.45-4.37 (m, 2H), 2.73-2.57 (m, 1H), 2.02-1.77 (m, 2H), 1.75-1.41 (m, 7H), 1.14 (d, J = 6.8 Hz, 3H).. MS (ESI): 439.2 [M+H]⁺.

Second eluting isomer of *N*-(4-chlorophenyl)-2-(5'-(trifluoromethyl)-2'*H-*spiro[cyclohexane-1,3'-furo[2,3-b]pyridin]-4-yl)propanamide **(Example 4c**), (RT of 4.20 min with the following analytical SFC-Conditions: column CHIRALPAK OD-H (4.6 x 100 mm, 5µm), mobile phase 90% CO₂/10% MeOH (0.2% ammonia, 7N in MeOH), flow rate 4 mL/min, column temperature 40°C): ¹H NMR (400 MHz, DMSO-d₆): δ ppm 10.04 (s, 1H), 8.40 (s, 1H), 7.96 (d, J = 2.0 Hz, 1H), 7.66 (d, J = 8.9 Hz, 2H), 7.35 (d, J = 8.9 Hz, 2H), 4.45-4.37 (m, 2H), 2.73-2.57 (m, 1H), 2.02-1.77 (m, 2H), 1.75-1.41 (m, 7H), 1.14 (d, J = 6.8 Hz, 3H). MS (ESI): 439.2 [M+H]⁺.

Third eluting isomer of *N*-(4-chlorophenyl)-2-(5'-(trifluoromethyl)-2'*H*-spiro[cyclohexane-1,3'-furo[2,3-b]pyridin]-4-yl)propanamide **(Example 4d),** (RT of 4.67 min with the following analytical SFC-Conditions: column CHIRALPAK OD-H (4.6 x 100 mm, 5µm), mobile phase 90% CO₂/ 10% MeOH (0.2% ammonia, 7N in MeOH), flow rate 4 mL/min, column temperature 40°C): ¹H NMR (400 MHz, DMSO-d₆): δ ppm 10.01 (s, 1H), 8.35 (s, 1H), 8.06 (d, J = 2.2 Hz, 1H), 7.65 (d, J=8.8 Hz, 2H), 7.34 (d, J=8.8 Hz, 2H), 4.55-4.49 (m, 2H), 2.29-2.17 (m, 1H), 1.90-1.51 (m, 7H), 1.19-0.99 (m, 5H). MS (ESI): 439.1 [M+H]+.

Fourth eluting isomer of *N*-(4-chlorophenyl)-2-(5'-(trifluoromethyl)-2'*H-*spiro[cyclohexane-1,3'-furo[2,3-b]pyridin]-4-yl)propanamide **(Example 4e),** (RT of 6.08 min with the following analytical SFC-Conditions: column CHIRALPAK OD-H (4.6 x 100 mm, 5µm), mobile phase 90% CO₂/10% MeOH (0.2% ammonia, 7N in MeOH), flow rate 4 mL/min, column temperature 40°C): ¹H NMR (400 MHz, DMSO-d₆): δ ppm 10.01 (s, 1H), 8.35 (s, 1H), 8.06 (d, J = 2.2 Hz, 1H), 7.65 (d, J=8.8 Hz, 2H), 7.34 (d, J=8.8 Hz, 2H), 4.55-4.49 (m, 2H), 2.29-2.17 (m, 1H), 1.90-1.51 (m, 7H), 1.19-0.99 (m, 5H). MS (ESI): 439.2 [M+H]⁺.

### Examples 5b, 5c, 5d, 5e: Separate isomers of N-(4-chlorophenyl)-2-(6'-(trifluoromethyl)-2'H-spiro[cyclohexane-1,3'-furo[2,3-b]pyridin]-4-yl)propanamide (5a), (5b, 5c, 5d, 5e)

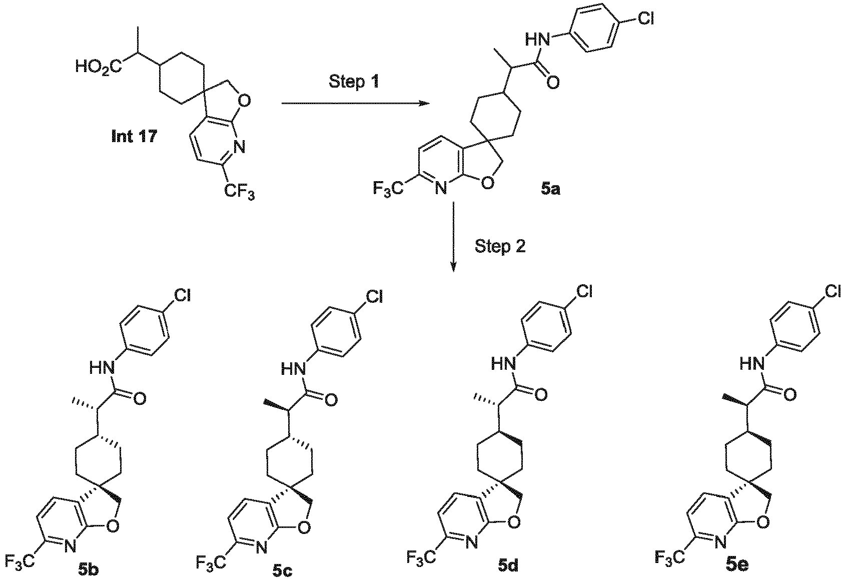

### Examples 5b, 5c, 5d, 5e, configurations not assigned

### Step 1: N-(4-chlorophenyl)-2-(6'-(trifluoromethyl)-2'H-spiro[cyclohexane-1,3'-furo[2,3-b]pyridin]-4-yl)propanamide (5a)

*N*-(4-chlorophenyl)-2-(6'-(trifluoromethyl)-2'*H*-spiro[cyclohexane-1,3'-furo[2,3-b]pyridin]-4-yl)propanamide **(5a)** was obtained from 2-(5'-(trifluoromethyl)-2'*H*-spiro[cyclohexane-1,3'-furo[2,3-b]pyridin]-4-yl)propanoic acid **(Int 17)** following a similar procedure as described for *N*-(4-chlorophenyl)-2-(6-(trifluoromethyl)-2H-spiro[benzofuran-3,1 '-cyclohexan]-4'-yl)propanamide **(3a).**

### Step 2: Separate isomers of N-(4-chlorophenyl)-2-(6'-(trifluoromethyl)-2'H-spiro[cyclohexane-1,3'-furo[2,3-b]pyridin]-4-yl)propanamide (5a), (Examples 5b, 5c, 5d, 5e)

*N*-(4-chlorophenyl)-2-(6'-(trifluoromethyl)-2'*H*-spiro[cyclohexane-1,3'-furo[2,3-b]pyridin]-4-yl)propanamide **(5a)** was subjected to a separation via chiral SFC, using the following preparative chiral-SFC conditions: stationary phase CHIRALCEL AD (Daicel), mobile phase CO₂/MeOH (0.2% ammonia, 7N in MeOH), 40/60. Two fractions were separated. The first eluted fraction (from prep chiral SFC) was concentrated to dryness and subjected to a second separation via chiral HPLC, using the following conditions: stationary phase (S, S) WHELK, mobile phase n-Hexane (0.1%DEA)/EtOH(0.1%DEA), 75:25, giving rise to two separate isomers of *N*-(4-chlorophenyl)-2-(6'-(trifluoromethyl)-2'*H*-spiro[cyclohexane-1,3'-furo[2,3-b]pyridin]-4-yl)propanamide **(Example 5b,** first eluting and **Example 5c,** second eluting from prep chiral HPLC). The second eluting fraction (from prep chiral SFC) was concentrated to dryness and subjected to a second separation via chiral SFC, using the following conditions: stationary phase CHIRALPAK AY (Daicel), mobile phase CO₂/EtOH (0.5% ammonia, 7N in MeOH), 70/30, giving rise to two separate isomers of *N*-(4-chlorophenyl)-2-(6'-(trifluoromethyl)-2'*H-*spiro[cyclohexane-1,3'-furo[2,3-b]pyridin]-4-yl)propanamide **(Example 5d,** first eluting and **Example 5e,** second eluting from prep chiral SFC).

*N*-(4-chlorophenyl)-2-(6'-(trifluoromethyl)-2'*H*-spiro[cyclohexane-1,3'-furo[2,3-b]pyridin]-4-yl)propanamide **(Example 5b),** (RT of 1.29 min with the following analytical SFC-Conditions: column CHIRALPAK AD-H (4.6 x 100 mm, 5µm), mobile phase 80% CO₂/ 20% MeOH (0.2% ammonia, 7N in MeOH), flow rate 4 mL/min, column temperature 40°C): ¹H NMR (400 MHz, DMSO-d₆): δ ppm 10.02 (s, 1H), 7.97 (d, J = 7.5 Hz, 1H), 7.65 (d, J = 8.9 Hz, 2H), 7.42 (d, J = 7.5 Hz, 1H), 7.35 (d, J = 8.9 Hz, 2H), 4.39.4.33 (m, 2H), 2.60-2.52 (m, 1H), 1.93-1.44 (m, 9H), 1.14 (d, J = 6.8 Hz, 3H). MS (ESI): 438.9 [M+H]⁺. *N*-(4-chlorophenyl)-2-(6'-(trifluoromethyl)-2'*H*-spiro[cyclohexane-1,3'-furo[2,3-b]pyridin]-4-yl)propanamide **(Example 5c),** (RT of 1.50 min with the following analytical SFC-Conditions: column CHIRALPAK AD-H (4.6 x 100 mm, 5µm), mobile phase 80% CO₂/ 20% MeOH (0.2% ammonia, 7N in MeOH), flow rate 4 mL/min, column temperature 40°C): ¹H NMR (400 MHz, DMSO-d₆): δ ppm 10.02 (s, 1H), 7.97 (d, J = 7.5 Hz, 1H), 7.65 (d, J = 8.9 Hz, 2H), 7.42 (d, J = 7.5 Hz, 1H), 7.35 (d, J = 8.9 Hz, 2H), 4.39.4.33 (m, 2H), 2.60-2.52 (m, 1H), 1.93-1.44 (m, 9H), 1.14 (d, J = 6.8 Hz, 3H). MS (ESI): 438.9 [M+H]⁺.

*N*-(4-chlorophenyl)-2-(6'-(trifluoromethyl)-2'*H*-spiro[cyclohexane-1,3'-furo[2,3-b]pyridin]-4-yl)propanamide **(Example 5d),** (RT of 2.28 min with the following analytical SFC-Conditions: column CHIRALPAK AD-H (4.6 x 100 mm, 5µm), mobile phase 80% CO₂/ 20% MeOH (0.2% ammonia, 7N in MeOH), flow rate 4 mL/min, column temperature 40°C): ¹H NMR (400 MHz, DMSO-d₆): δ ppm 10.00 (s, 1H), 7.86 (d, J = 7.5 Hz, 1H), 7.65 (d, J = 8.9 Hz, 2H), 7.39 (d, J = 7.5 Hz, 1H), 7.34 (d, J = 8.9 Hz, 2H), 4.55-4.47 (m, 2H), 2.27-2.22 (m, 1H), 1.92-1.53 (m, 7H), 1.22-0.96 (m, 5H). MS (ESI): 439.1 [M+H]⁺.

*N*-(4-chlorophenyl)-2-(6'-(trifluoromethyl)-2'*H*-spiro[cyclohexane-1,3'-furo[2,3-b]pyridin]-4-yl)propanamide **(Example 5e),** (RT of 2.48 min with the following analytical SFC-Conditions: column CHIRALPAK AD-H (4.6 x 100 mm, 5µm), mobile phase 80% CO₂/ 20% MeOH (0.2% ammonia, 7N in MeOH), flow rate 4 mL/min, column temperature 40°C): ¹H NMR (400 MHz, DMSO-d₆): δ ppm 10.00 (s, 1H), 7.86 (d, J = 7.5 Hz, 1H), 7.65 (d, J = 8.9 Hz, 2H), 7.39 (d, J = 7.5 Hz, 1H), 7.34 (d, J = 8.9 Hz, 2H), 4.55-4.47 (m, 2H), 2.27-2.22 (m, 1H), 1.92-1.53 (m, 7H), 1.22-0.96 (m, 5H). MS (ESI): 439.1 [M+H]⁺.

### Examples 6b, 6c, 6d, 6e: Separate isomers of N-(4-chlorophenyl)-2-(2'-methyl-3'-oxo-5'-(trifluoromethyl)spiro[cyclohexane-1,1'-isoindolin]-4-yl)propanamide (6a), (6b, 6c, 6d, 6e)

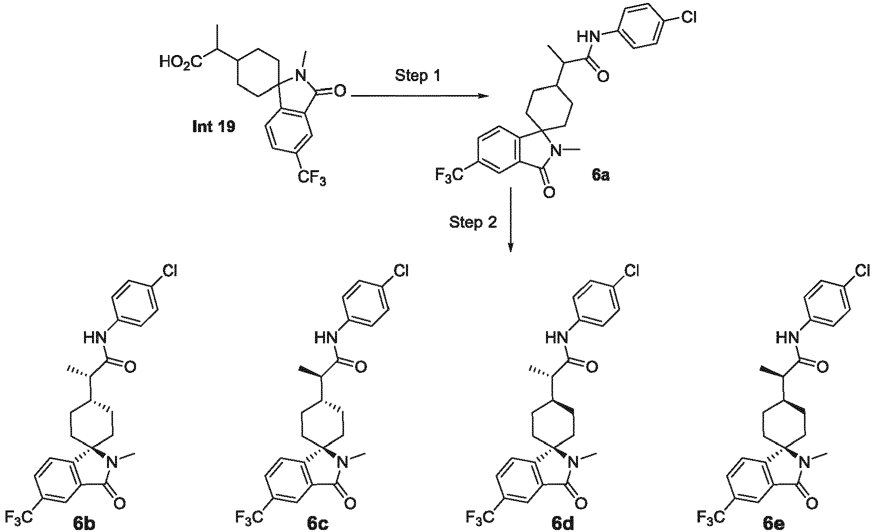

### Examples 6b, 6c, 6d, 6e, configurations not assigned

### Step 1: N-(4-chlorophenyl)-2-(2'-methyl-3'-oxo-5'-(trifluoromethyl)spiro[cyclohexane-1,1'-isoindolin]-4-yl)propanamide (6a)

*N*-(4-chlorophenyl)-2-(2'-methyl-3'-oxo-5'-(trifluoromethyl)spiro[cyclohexane-1,1'-isoindolin]-4-yl)propanamide **(6a)** was obtained from 2-(2'-methyl-3'-oxo-5'-(trifluoromethyl)spiro[cyclohexane-1,1'-isoindolin]-4-yl)propanoic acid **(Int 19)** following a similar procedure as described for *N*-(4-chlorophenyl)-2-(6-(trifluoromethyl)-2H-spiro[benzofuran-3,1'-cyclohexan]-4'-yl)propanamide **(3a).**

### Step 2: Separate isomers of N-(4-chlorophenyl)-2-(2'-methyl-3'-oxo-5'-(trifluoromethyl)spiro[cyclohexane-1,1'-isoindolin]-4-yl)propanamide (6a), (Examples 6a, 6b, 6c, 6d)

*N*-(4-Chlorophenyl)-2-(2'-methyl-3'-oxo-5'-(trifluoromethyl)spiro[cyclohexane-1,1'-isoindolin]-4-yl)propanamide **(6a)** was subjected to a separation via chiral SFC, using the following preparative chiral-SFC conditions: stationary phase CHIRALCEL AD-3 (Daicel), mobile phase CO₂/*i*-PrOH, 75/25. Three fractions were separated. The first eluted fraction (from prep chiral SFC) was concentrated to dryness and afforded the single isomer **6b**. The second eluted fraction was concentrated to dryness and afforded the single isomer **6c**. The third eluted fraction was concentrated to dryness and subjected to a second separation via chiral SFC, using the following conditions: stationary phase CHIRALCEL AD-3 (Daicel), mobile phase CO₂/MeOH, 60/40, giving rise to two separate isomers, **6d,** first eluting and **6e,** second eluting.

*N*-(4-Chlorophenyl)-2-(2'-methyl-3'-oxo-5'-(trifluoromethyl)spiro[cyclohexane-1,1'-isoindolin]-4-yl)propanamide **(6b),** (RT of 2.00 min with the following analytical SFC-Conditions: column CHIRALPAK AD-3 (4.6 x 100 mm, 3µm), mobile phase 75% CO₂/ 25% *i*-PrOH, flow rate 2 mL/min, column temperature 35°C): ¹H NMR (400 MHz, DMSO-d₆): δ ppm 10.08 (s, 1H), 8.06 (d, J = 7.9 Hz, 1H), 7.98-7.95 (m, 2H), 7.68 (d, J = 8.9 Hz, 2H), 7.36 (d, J = 8.9 Hz, 2H), 2.97 (s, 3H), 2.53-2.51 (m, 1H), 2.22-2.03 (m, 2H), 2.00-1.96 (m, 1H), 1.84-1.61 (m, 4H), 1.40-1.33 (m, 2H), 1.20 (d, J = 6.8 Hz, 3H). MS (ESI): 465.1 [M+H]⁺.

*N*-(4-Chlorophenyl)-2-(2'-methyl-3'-oxo-5'-(trifluoromethyl)spiro[cyclohexane-1,1'-isoindolin]-4-yl)propanamide **(6c),** (RT of 3.28 min with the following analytical SFC-Conditions: column CHIRALPAK AD-3 (4.6 x 100 mm, 3µm), mobile phase 75% CO₂/ 25% *i*-PrOH, flow rate 2 mL/min, column temperature 35°C): ¹H NMR (400 MHz, DMSO-d₆): δ ppm 10.14 (s, 1H), 8.13 (d, J = 7.9 Hz, 1H), 7.95 (d, J = 7.9 Hz, 1H), 7.91 (s, 1H), 7.66 (d, J = 8.9 Hz, 2H), 7.36 (d, J = 8.9 Hz, 2H), 3.19 (s, 3H), 2.69-2.65 (m, 1H), 2.11-1.81 (m, 5H), 1.76-1.68 (m, 4H), 1.17 (d, J = 6.7 Hz, 3H). MS (ESI): 465.2 [M+H]⁺.

*N*-(4-Chlorophenyl)-2-(2'-methyl-3'-oxo-5'-(trifluoromethyl)spiro[ cyclohexane-1,1'-isoindolin]-4-yl)propanamide **(6d),** (RT of 3.81 min with the following analytical SFC-Conditions: column CHIRALPAK AD-3 (4.6 x 100 mm, 3µm), mobile phase 75% CO₂/ 25% *i*-PrOH, flow rate 2 mL/min, column temperature 35°C): ¹H NMR (400 MHz, DMSO-d₆): δ ppm 10.07 (s, 1H), 8.06 (d, J = 7.9 Hz, 1H), 7.98-7.95 (m, 2H), 7.68 (d, J = 8.9 Hz, 2H), 7.36 (d, J = 8.9 Hz, 2H), 2.96 (s, 3H), 2.53-2.51 (m, 1H), 2.22-2.06 (m, 2H), 1.99-1.96 (m, 1H), 1.84-1.61 (m, 4H), 1.40-1.33 (m, 2H), 1.20 (d, J = 6.8 Hz, 3H). MS (ESI): 465.3 [M+H]⁺.

*N*-(4-Chlorophenyl)-2-(2'-methyl-3'-oxo-5'-(trifluoromethyl)spiro[ cyclohexane-1,1'-isoindolin]-4-yl)propanamide **(6e),** (RT of 4.29 min with the following analytical SFC-Conditions: column CHIRALPAK AD-3 (4.6 x 100 mm, 3µm), mobile phase 75% CO₂/ 25% *i*-PrOH, flow rate 2 mL/min, column temperature 35°C): ¹H NMR (400 MHz, DMSO-d₆): δ ppm 10.14 (s, 1H), 8.13 (d, J = 7.9 Hz, 1H), 7.95 (d, J = 7.9 Hz, 1H), 7.92 (s, 1H), 7.66 (d, J = 8.9 Hz, 2H), 7.36 (d, J = 8.9 Hz, 2H), 3.19 (s, 3H), 2.69-2.65 (m, 1H), 2.11-1.81 (m, 5H), 1.76-1.68 (m, 4H), 1.17 (d, J = 6.7 Hz, 3H). MS (ESI): 465.1 [M+H]⁺.

### Examples 7b, 7c, 7d, 7e: Separate isomers of N-(4-chloro-2-fluorophenyl)-2-(6-(trifluoromethyl)-2H-spiro[benzofuran-3,1'-cyclohexan]-4'-yl)propanamide (7a), (Examples 7b, 7c, 7d, 7e)

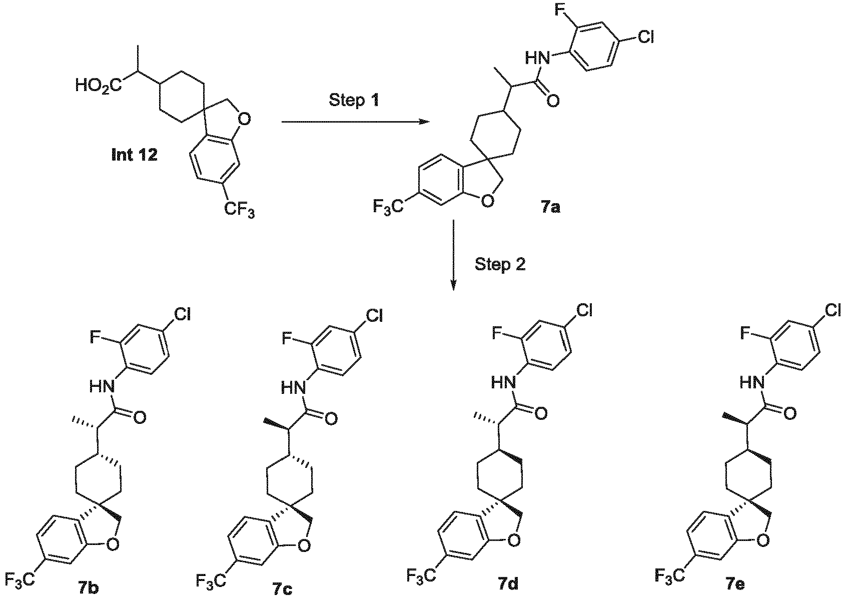

### Examples 7b, 7c, 7d, 7e, configurations not assigned

### Step 1: N-(4-Chloro-2-fluorophenyl)-2-(6-(trifluoromethyl)-2H-spiro[benzofuran-3,1'-cyclohexan]-4'-yl)propanamide (7a)

To a mixture of 2-(6-(trifluoromethyl)-2H-spiro[benzofuran-3,1'-cyclohexan]-4'-yl)propanoic acid **(Int 12)** (328 mg, 1.00 mmol) in DCM (10 mL) was added SOCl₂ (1 mL) and the mixture was stirred at rt for 2 h. The mixture was concentrated to give crude acyl chloride intermediate. The residue was redissolved in DCM (10 mL), 4-chloro-2-fluoroaniline (145 mg, 1.00 mmol) and TEA (303 mg, 3.00 mmol) were added and the mixture was stirred at rt for 2 h. The mixture was poured into water (30 mL) and the resulting mixture was extracted with DCM (3 x 30 mL). The combined organic phase was washed by brine (2 x 30 mL), dried over Na₂SO₄ and concentrated. The residue was purified by column chromatography on silica gel (PE:EtOAc = 5:1) to afford the title compound as a colorless oil.

### Step 2: Separate isomers of N-(4-chloro-2-fluorophenyl)-2-(6-(trifluoromethyl)-2H-spiro[benzofuran-3,1'-cyclohexan]-4'-yl)propanamide (7a), (Examples 7b, 7c, 7d, 7e)

*N*-(4-Chloro-2-fluorophenyl)-2-(6-(trifluoromethyl)-2*H*-spiro[benzofuran-3,1'-cyclohexan]-4'-yl)propanamide (7a) was subjected to a separation via chiral SFC, using the following conditions: stationary phase (S, S) WHELK (Daicel), mobile phase CO₂/MeOH (0.2% ammonia, 7N in MeOH), 75/25. Two fractions were separated. The first eluted fraction was concentrated to dryness and subjected to a second separation via chiral SFC, using the following conditions: stationary phase CHIRALPAK AD, mobile phase CO₂/MeOH (0.2% ammonia, 7N in MeOH), 65/35, giving rise to two separate isomers of *N*-(4-chloro-2-fluorophenyl)-2-(6-(trifluoromethyl)-2*H*-spiro[benzofuran-3,1'-cyclohexan]-4'-yl)propanamide: **Example 7b,** first eluting and **Example 7c,** second eluting. The second fraction of the first chiral SFC separation was also concentrated to dryness and subjected to a second separation via chiral SFC, using the following conditions: stationary phase CHIRALCEL AD, mobile phase CO₂/MeOH (0.2% ammonia, 7N in MeOH), 65/35, giving rise to two separate isomers of *N*-(4-chloro-2-fluorophenyl)-2-(6-(trifluoromethyl)-2*H*-spiro[benzofuran-3,1'-cyclohexan]-4'-yl)propanamide: **Example 7d,** first eluting and **Example 7e,** second eluting.

*N*-(4-Chloro-2-fluorophenyl)-2-(6-(trifluoromethyl)-2*H*-spiro[benzofuran-3,1'-cyclohexan]-4'-yl)propanamide **(Example 7b),** (RT of 0.92 min with the following analytical SFC-Conditions: column CHIRALPAK AD-H (4.6 x 100 mm, 5µm), mobile phase 75% CO₂/25% MeOH (0.2% ammonia, 7N in MeOH), flow rate 4 mL/min, column temperature 40°C): ¹H NMR (400 MHz, DMSO-d₆): δ ppm 9.81 (s, 1H), 7.89 (t, J = 8.6 Hz, 1H), 7.61 (d, J = 7.8 Hz, 1H), 7.49 (dd, J = 10.6, 2.3 Hz, 1H), 7.25 (t, J = 9.1 Hz, 2H), 7.11 (s, 1H), 4.38-4.31 (m, 2H), 2.91-2.78 (m, 1H), 1.96-1.86 (m, 1H), 1.84-1.63 (m, 3H), 1.62-1.47 (m, 5H), 1.13 (d, J = 6.8 Hz, 3H). MS (ESI): 456.1 [M+H]⁺.

*N*-(4-Chloro-2-fluorophenyl)-2-(6-(trifluoromethyl)-2*H*-spiro[benzofuran-3,1'-cyclohexan]-4'-yl)propanamide **(Example 7c),** (RT of 1.81 min with the following analytical SFC-Conditions: column CHIRALPAK AD-H (4.6 x 100 mm, 5µm), mobile phase 75% CO₂/25% MeOH (0.2% ammonia, 7N in MeOH), flow rate 4 mL/min, column temperature 40°C): ¹H NMR (400 MHz, DMSO-d₆): δ ppm 9.74 (s, 1H), 7.87 (t, J = 8.6 Hz, 1H), 7.48 (dd, J = 10.6, 2.3 Hz, 1H), 7.41 (d, J = 7.8 Hz, 1H), 7.25 (d, J = 8.6 Hz, 1H), 7.20 (d, J = 7.8 Hz, 1H), 7.08 (s, 1H), 4.48-4.42 (m, 2H), 2.47-2.40 (m, 1H), 1.92-1.50 (m, 7H), 1.28-0.93 (m, 5H). MS (ESI): 456.0 [M+H]⁺. *N*-(4-Chloro-2-fluorophenyl)-2-(6-(trifluoromethyl)-2*H*-spiro[benzofuran-3,1'-cyclohexan]-4'-yl)propanamide **(Example 7d),** (RT of 1.37 min with the following analytical SFC-Conditions: column CHIRALPAK AD-H (4.6 x 100 mm, 5µm), mobile phase 75% CO₂/25% MeOH (0.2% ammonia, 7N in MeOH), flow rate 4 mL/min, column temperature 40°C): ¹H NMR (400 MHz, DMSO-d₆): δ ppm 9.81 (s, 1H), 7.89 (t, J = 8.6 Hz, 1H), 7.61 (d, J = 7.8 Hz, 1H), 7.49 (dd, J = 10.6, 2.3 Hz, 1H), 7.25 (t, J = 9.1 Hz, 2H), 7.11 (s, 1H), 4.38-4.31 (m, 2H), 2.91-2.78 (m, 1H), 1.96-1.86 (m, 1H), 1.84-1.63 (m, 3H), 1.62-1.47 (m, 5H), 1.13 (d, J = 6.8 Hz, 3H). MS (ESI): 456.0 [M+H]⁺.

*N*-(4-Chloro-2-fluorophenyl)-2-(6-(trifluoromethyl)-2*H*-spiro[benzofuran-3,1'-cyclohexan]-4'-yl)propanamide **(Example 7e),** (RT of 1.83 min with the following analytical SFC-Conditions: column CHIRALPAK AD-H (4.6 x 100 mm, 5µm), mobile phase 75% CO₂/25% MeOH(0.2% ammonia, 7N in MeOH), flow rate 4 mL/min, column temperature 40°C): ¹H NMR (400 MHz, DMSO-d₆): δ ppm 9.74 (s, 1H), 7.87 (t, J = 8.6 Hz, 1H), 7.48 (dd, J = 10.6, 2.3 Hz, 1H), 7.41 (d, J = 7.8 Hz, 1H), 7.25 (d, J = 8.6 Hz, 1H), 7.20 (d, J = 7.8 Hz, 1H), 7.08 (s, 1H), 4.48-4.42 (m, 2H), 2.47-2.40 (m, 1H), 1.92-1.50 (m, 7H), 1.28-0.93 (m, 5H). MS (ESI): 456.1 [M+H]⁺.

### Examples 8b, 8c, 8d, 8e: Separate isomers of N-(p-tolyl)-2-(6-(trifluoromethyl)-2H-spiro[benzofuran-3,1'-cyclohexan]-4'-yl)propanamide (8a), (Examples 8b, 8c, 8d, 8e)

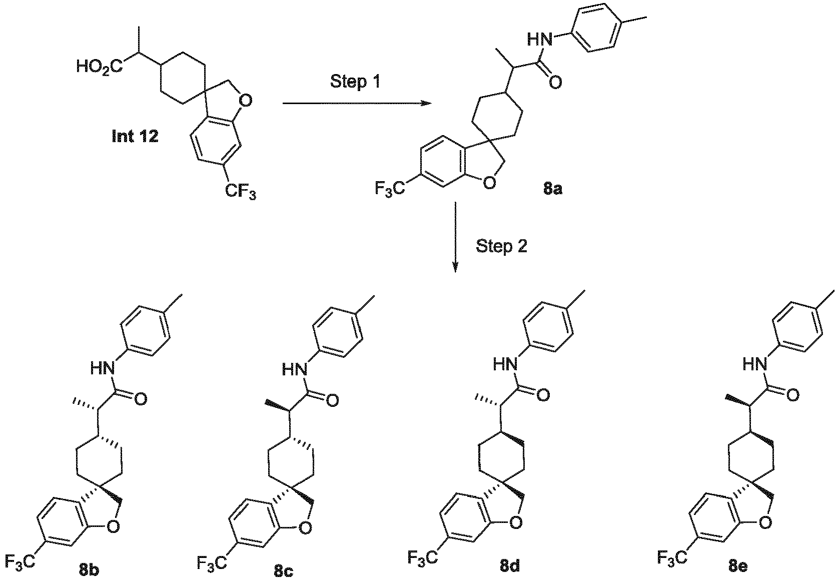

### Examples 8b, 8c, 8d, 8e, configurations not assigned

### Step 1: N-(p-Tolyl)-2-(6-(trifluoromethyl)-2H-spiro[benzofuran-3,1'-cyclohexan]-4'-yl)propanamide (8a)

*N*-(*p*-Tolyl)-2-(6-(trifluoromethyl)-2H-spiro[benzofuran-3,1'-cyclohexan]-4'-yl)propanamide **(8a)** was obtained from 2-(6-(trifluoromethyl)-2H-spiro[benzofuran-3,1'-cyclohexan]-4'-yl)propanoic acid **(Int 12)** following a similar procedure as described for *N*-(4-chloro-2-fluorophenyl)-2-(6-(trifluoromethyl)-2*H*-spiro[benzofuran-3,1'-cyclohexan]-4'-yl)propanamide **(7a).**

### Step 2: Separate isomers of N-(p-tolyl)-2-(6-(trifluoromethyl)-2H-spiro[benzofuran-3,1'-cyclohexan]-4'-yl)propanamide (8a), (Examples 8b, 8c, 8d, 8e)

*N*-(*p*-Tolyl)-2-(6-(trifluoromethyl)-2H-spiro[benzofuran-3,1'-cyclohexan]-4'-yl)propanamide **(8a)** was subjected to a separation via chiral SFC, using the following conditions: stationary phase (S, S) WHELK (Daicel), mobile phase CO₂/MeOH (0.2% ammonia, 7N in MeOH), 65/35. Two fractions were separated. The first eluted fraction was concentrated to dryness and subjected to a second separation via chiral SFC, using the following conditions: stationary phase CHIRALPAK AD, mobile phase CO₂/MeOH (0.2% ammonia, 7N in MeOH), 65/35, giving rise to two separate isomers of *N*-(*p*-tolyl)-2-(6-(trifluoromethyl)-2H-spiro[benzofuran-3,1'-cyclohexan]-4'-yl)propanamide: **Example 8b,** first eluting and **Example 8c,** second eluting isomer. The second fraction of the first chiral SFC separation was also concentrated to dryness and subjected to a second separation via chiral SFC, using the following conditions: stationary phase CHIRALCEL AD, mobile phase CO₂/MeOH (0.2% ammonia, 7N in MeOH), 65/35, giving rise to two separate isomers of *N*-(*p*-tolyl)-2-(6-(trifluoromethyl)-2H-spiro[benzofuran-3,1'-cyclohexan]-4'-yl)propanamide: **Example 8d,** first eluting and **Example 8e,** second eluting.

*N*-(*p*-Tolyl)-2-(6-(trifluoromethyl)-2H-spiro[benzofuran-3,1'-cyclohexan]-4'-yl)propanamide **(Example 8b):** (RT of 0.92 min with the following analytical SFC-Conditions: column CHIRALPAK AD-H (4.6 x 100 mm, 5µm), mobile phase 75% CO₂/ 25% MeOH(O.2% ammonia, 7N in MeOH), flow rate 4 mL/min, column temperature 40°C): ¹H NMR (500 MHz, DMSO-d₆): δ ppm 9.82 (s, 1H), 7.60 (d, J = 7.7 Hz, 1H), 7.50 (d, J = 8.6 Hz, 2H), 7.23 (d, J = 7.7 Hz, 1H), 7.10-7.07 (m, 3H), 4.36-4.30 (m, 2H), 2.63-2.58 (m, 1H), 2.24 (s, 3H), 1.93-1.84 (m, 1H), 1.83-1.63 (m, 3H), 1.62-1.46 (m, 5H), 1.13 (d, J = 6.8 Hz, 3H). MS (ESI): 418.1 [M+H]⁺.

*N*-(*p*-Tolyl)-2-(6-(trifluoromethyl)-2H-spiro[benzofuran-3,1'-cyclohexan]-4'-yl)propanamide **(Example 8c),** (RT of 1.69 min with the following analytical SFC-Conditions: column CHIRALPAK AD-H (4.6 x 100 mm, 5µm), mobile phase 75% CO₂/ 25% MeOH (0.2% ammonia, 7N in MeOH), flow rate 4 mL/min, column temperature 40°C): ¹H NMR (500 MHz, DMSO-d₆): δ ppm 9.75 (s, 1H), 7.49 (d, J = 8.4 Hz, 2H), 7.41 (d, J = 7.7 Hz, 1H), 7.20 (d, J = 8.4 Hz, 1H), 7.09-7.07 (m, 3H), 4.47-4.42 (m, 2H), 2.29-2.17 (m, 4H), 1.91-1.51 (m, 7H), 1.21-0.95 (m, 5H). MS (ESI): 418.1 [M+H]⁺.

*N*-(*p*-Tolyl)-2-(6-(trifluoromethyl)-2H-spiro[benzofuran-3,1'-cyclohexan]-4'-yl)propanamide **(Example 8d),** (RT of 1.04 min with the following analytical SFC-Conditions: column CHIRALPAK AD-H (4.6 x 100 mm, 5µm), mobile phase 75% CO₂/ 25% MeOH (0.2% ammonia, 7N in MeOH), flow rate 4 mL/min, column temperature 40°C): ¹H NMR (500 MHz, DMSO-d₆): δ ppm 9.83 (s, 1H), 7.60 (d, J = 7.7 Hz, 1H), 7.50 (d, J = 8.6 Hz, 2H), 7.23 (d, J = 7.7 Hz, 1H), 7.10-7.07 (m, 3H), 4.36-4.30 (m, 2H), 2.63-2.58 (m, 1H), 2.24 (s, 3H), 1.93-1.84 (m, 1H), 1.83-1.63 (m, 3H), 1.62-1.46 (m, 5H), 1.13 (d, J = 6.8 Hz, 3H).. MS (ESI): 418.1 [M+H]⁺. *N*-(*p*-Tolyl)-2-(6-(trifluoromethyl)-2H-spiro[benzofuran-3,1'-cyclohexan]-4'-yl)propanamide **(Example 8e),** (RT of 1.75 min with the following analytical SFC-Conditions: column CHIRALPAK AD-H (4.6 x 100 mm, 5µm), mobile phase 75% CO₂/ 25% MeOH (0.2% ammonia, 7N in MeOH), flow rate 4 mL/min, column temperature 40°C): ¹H NMR (500 MHz, DMSO-d₆): δ ppm 9.75 (s, 1H), 7.49 (d, J = 8.4 Hz, 2H), 7.41 (d, J = 7.7 Hz, 1H), 7.20 (d, J = 8.4 Hz, 1H), 7.09-7.07 (m, 3H), 4.47-4.42 (m, 2H), 2.29-2.17 (m, 4H), 1.91-1.51 (m, 7H), 1.21-0.95 (m, 5H). MS (ESI): 418.1 [M+H]⁺.

### Examples 9b, 9c, 9d, 9e: Separate isomers of N-((1r,4r)-4-methylcyclohexyl)-2-(6-(trifluoromethyl)-2H-spiro[benzofuran-3,1'-cyclohexan]-4'-yl)propanamide (9a), (Examples 9b, 9c, 9d, 9e)

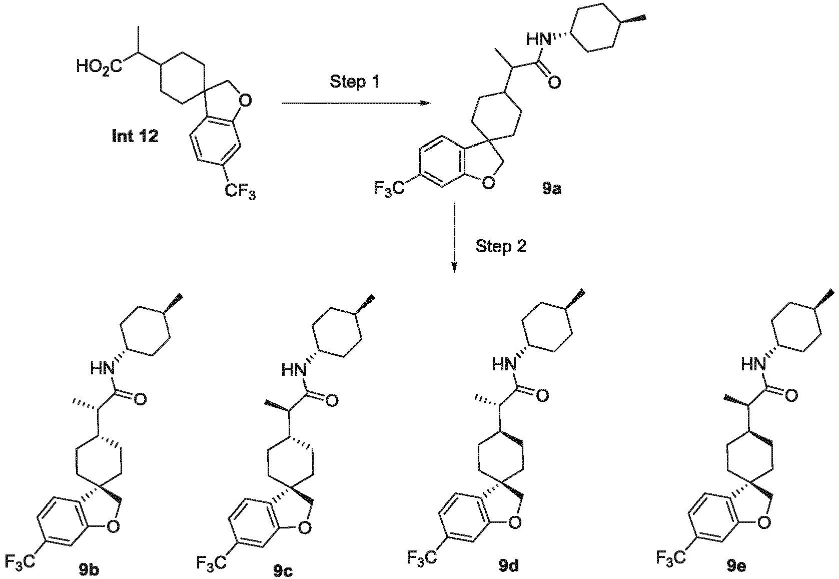

### Examples 9b, 9c, 9d, 9e, configurations not assigned

### Step 1: N-((1r,4r)-4-Methylcyclohexyl)-2-(6-(trifluoromethyl)-2H-spiro[benzofuran-3,1'-cyclohexan]-4'-yl)propanamide (9a)

*N*-((1*r*,4*r*)-4-Methylcyclohexyl)-2-(6-(trifluoromethyl)-2*H*-spiro[benzofuran-3,1'-cyclohexan]-4'-yl)propanamide **(9a)** was obtained from 2-(6-(trifluoromethyl)-2H-spiro[benzofuran-3,1'-cyclohexan]-4'-yl)propanoic acid **(Int 12)** following a similar procedure as described for *N*-(4-chloro-2-fluorophenyl)-2-(6-(trifluoromethyl)-2*H-*spiro[benzofuran-3,1'-cyclohexan]-4'-yl)propanamide **(7a).**

### Step 2: Separate isomers of N-((1r,4r)-4-Methylcyclohexyl)-2-(6-(trifluoromethyl)-2H-spiro[benzofuran-3,1'-cyclohexan]-4'-yl)propanamide (9a), (Examples 9b, 9c, 9d, 9e)

*N*-((1*r*,4*r*)-4-Methylcyclohexyl)-2-(6-(trifluoromethyl)-2*H*-spiro[benzofuran-3,1'-cyclohexan]-4'-yl)propanamide **(9a)** was subjected to a separation via chiral SFC, using the following conditions: stationary phase CHIRALPAK AD (Daicel), mobile phase CO₂/MeOH (1% ammonia, 7N in MeOH), 75/25. Three fractions were separated. The first eluted fraction was concentrated to afford the single isomer **Example 9b.** The second fraction was concentrated to dryness and subjected to a second separation via chiral HPLC, using the following conditions: stationary phase CHIRALCEL AD, mobile phase n-hexane(0.1% DEA):EtOH (0.1% DEA), 75/25, giving rise to two separate isomers of *N*-((1*r*,4*r*)-4-methylcyclohexyl)-2-(6-(trifluoromethyl)-2*H*-spiro[benzofuran-3,1'-cyclohexan]-4'-yl)propanamide: **Example 9c,** first eluting and **Example 9d,** second eluting isomer. The third fraction of the first chiral SFC separation was also concentrated to dryness to afford the single isomer of *N*-((1*r*,4*r*)-4-methylcyclohexyl)-2-(6-(trifluoromethyl)-2*H*-spiro[benzofuran-3,1'-cyclohexan]-4'-yl)propanamide: **Example 9e.**

*N*-((1*r*,4*r*)-4-Methylcyclohexyl)-2-(6-(trifluoromethyl)-2*H*-spiro[benzofuran-3,1'-cyclohexan]-4'-yl)propanamide **(Example 9b):** (RT of 1.34 min with the following analytical SFC-Conditions: column CHIRALPAK AD-H (4.6 x 100 mm, 5µm), mobile phase 75% CO₂/25% MeOH (0.2% ammonia, 7N in MeOH), flow rate 4 mL/min, column temperature 40°C): ¹H NMR (500 MHz, DMSO-d₆): δ ppm 7.66 (d, J = 7.9 Hz, 1H), 7.57 (d, J = 7.8 Hz, 1H), 7.22 (d, J = 7.9 Hz, 1H), 7.10 (s, 1H), 4.36-4.29 (m, 2H), 3.56-3.41 (m, 1H), 2.38-2.34 (m, 1H), 1.93-1.82 (m, 1H), 1.80-1.39 (m, 12H), 1.35-1.24 (m, 1H), 1.23-1.05 (m, 2H), 1.03-0.89 (m, 5H), 0.85 (d, J = 6.5 Hz, 3H). MS (ESI): 424.3 [M+H]⁺.

*N*-((1*r*,4*r*)-4-Methylcyclohexyl)-2-(6-(trifluoromethyl)-2*H*-spiro[benzofuran-3,1'-cyclohexan]-4'-yl)propanamide **(Example 9c),** (RT of 2.77 min with the following analytical SFC-Conditions: column CHIRALPAK AD-H (4.6 x 100 mm, 5µm), mobile phase 75% CO₂/25% MeOH (0.2% ammonia, 7N in MeOH), flow rate 4 mL/min, column temperature 40°C): ¹H NMR (500 MHz, DMSO-d₆): δ ppm 7.58 (d, J = 7.9 Hz, 1H), 7.40 (d, J = 7.7 Hz, 1H), 7.20 (d, J = 7.9 Hz, 1H), 7.07 (s, 1H), 4.45-4.38 (m, 2H), 3.51-3.39 (m, 1H), 2.00-1.89 (m, 1H), 1.84-1.40 (m, 11H), 1.35-0.89 (m, 10H), 0.85 (d, J = 6.5 Hz, 3H). MS (ESI): 424.3 [M+H]⁺.

*N*-((1*r*,4*r*)-4-Methylcyclohexyl)-2-(6-(trifluoromethyl)-2*H*-spiro[benzofuran-3,1'-cyclohexan]-4'-yl)propanamide **(Example 9d),** (RT of 2.92 min with the following analytical SFC-Conditions: column CHIRALPAK AD-H (4.6 x 100 mm, 5µm), mobile phase 75% CO₂/ 25% MeOH(0.2% ammonia, 7N in MeOH), flow rate 4 mL/min, column temperature 40°C): ¹H NMR (500 MHz, DMSO-d₆): δ ppm 7.58 (d, J = 7.9 Hz, 1H), 7.40 (d, J = 7.7 Hz, 1H), 7.20 (d, J = 7.9 Hz, 1H), 7.07 (s, 1H), 4.45-4.38 (m, 2H), 3.51-3.39 (m, 1H), 2.00-1.89 (m, 1H), 1.84-1.40 (m, 11H), 1.35-0.89 (m, 10H), 0.85 (d, J = 6.5 Hz, 3H). MS (ESI): 424.3 [M+H]⁺.

*N*-((1*r*,4*r*)-4-Methylcyclohexyl)-2-(6-(trifluoromethyl)-2*H*-spiro[benzofuran-3,1'-cyclohexan]-4'-yl)propanamide **(Example 9e),** (RT of 3.81 min with the following analytical SFC-Conditions: column CHIRALPAK AD-H (4.6 x 100 mm, 5µm), mobile phase 75% CO₂/ 25% MeOH(0.2% ammonia, 7N in MeOH), flow rate 4 mL/min, column temperature 40°C): ¹H NMR (500 MHz, DMSO-d₆): δ ppm 7.67 (d, J = 7.9 Hz, 1H), 7.57 (d, J = 7.8 Hz, 1H), 7.22 (d, J = 7.9 Hz, 1H), 7.10 (s, 1H), 4.36-4.29 (m, 2H), 3.56-3.41 (m, 1H), 2.38-2.34 (m, 1H), 1.93-1.82 (m, 1H), 1.80-1.39 (m, 12H), 1.35-1.24 (m, 1H), 1.23-1.05 (m, 2H), 1.03-0.89 (m, 5H), 0.85 (d, J = 6.5 Hz, 3H). MS (ESI): 424.3 [M+H]⁺.

### Examples 10b, 10c, 10d, 10e: N-(5-chloropyridin-2-yl)-2-(6-(trifluoromethyl)-2H-spiro[benzofuran-3,1'-cyclohexan]-4'-yl)butanamide (10a), (Examples 10b, 10c, 10d, 10e)

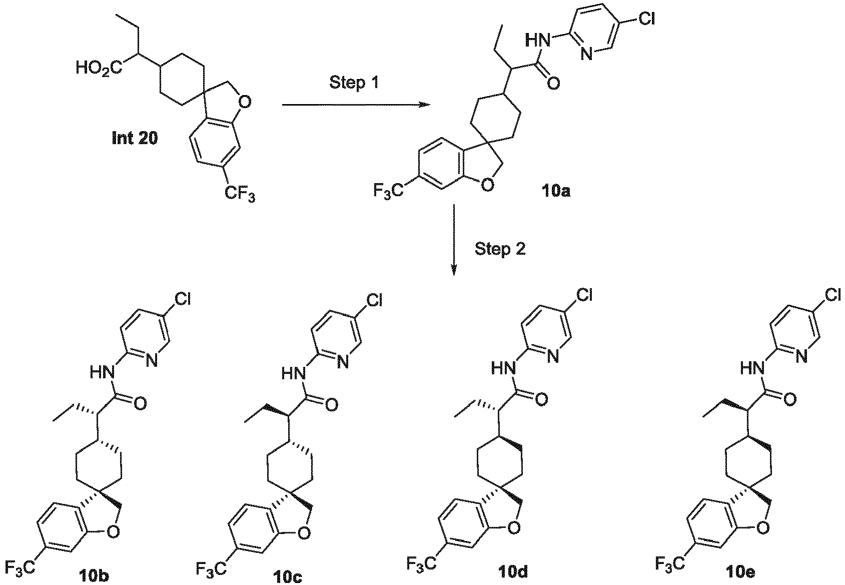

### Examples 10b, 10c, 10d, 10e, configurations not assigned

### Step 1: N-(5-chloropyridin-2-yl)-2-(6-(trifluoromethyl)-2H-spiro[benzofuran-3,1'-cyclohexan]-4'-yl)butanamide (10a)

*N*-(5-chloropyridin-2-yl)-2-(6-(trifluoromethyl)-2*H*-spiro[benzofuran-3,1'-cyclohexan]-4'-yl)butanamide **(10a)** was obtained from 2-(6-(trifluoromethyl)-2H-spiro[benzofuran-3,1'-cyclohexan]-4'-yl)butanoic acid (**Int 20**) following a similar procedure as described for N-(4-chloro-2-fluorophenyl)-2-(6-(trifluoromethyl)-2*H*-spiro[benzofuran-3,1'-cyclohexan]-4'-yl)propanamide **(7a).**

### Step 2: Separate isomers of N-(5-chloropyridin-2-yl)-2-(6-(trifluoromethyl)-2H-spiro[benzofuran-3,1'-cyclohexan]-4'-yl)butanamide (10a), (Examples 10b, 10c, 10d, 10e)

*N*-(5-chloropyridin-2-yl)-2-(6-(trifluoromethyl)-2*H*-spiro[benzofuran-3,1'-cyclohexan]-4'-yl)butanamide **(10a)** was subjected to a separation via column chromatography on silica gel (PE:EtOAc, 2:1). Two fractions were obtained. The first eluted fraction was concentrated to dryness and subjected to a separation via chiral SFC, using the following conditions: stationary phase CHIRALCEL AD, CO₂:MeOH, 70/30, giving rise to two separate isomers of *N*-(5-chloropyridin-2-yl)-2-(6-(trifluoromethyl)-2*H*-spiro[benzofuran-3,1'-cyclohexan]-4'-yl)butanamide: **Example 10b,** first eluting and **Example 10c,** second eluting isomer. The second fraction of the separation on silica gel was also concentrated to dryness and subjected to a separation via chiral SFC, using the following conditions: stationary phase CHIRALCEL AS, CO₂:MeOH, 90/210, giving rise to two separate isomers of *N*-(5-chloropyridin-2-yl)-2-(6-(trifluoromethyl)-2H-spiro[benzofuran-3,1'-cyclohexan]-4'-yl)butanamide: **Example 10d,** first eluting and **Example 10e,** second eluting isomer.

*N*-(5-chloropyridin-2-yl)-2-(6-(trifluoromethyl)-2*H*-spiro[benzofuran-3,1'-cyclohexan]-4'-yl)butanamide **(Example 10b):** (RT of 1.79 min with the following analytical SFC-Conditions: column CHIRALPAK AD-3 (4.6 x 100 mm, 3µm), mobile phase 65% CO₂/MeOH 35%, flow rate 2 mL/min, column temperature 35°C) ¹H NMR (400 MHz, DMSO-d₆): δ ppm 10.74 (s, 1H), 8.35 (s, 1H), 8.19 (d, J = 8.9 Hz, 1H), 7.88 (d, J = 8.9, 1H), 7.60 (d, J = 7.2 Hz, 1H), 7.21 (d, J = 7.2 Hz, 1H), 7.08 (s, 1H), 4.35-4.28 (m, 2H), 2.85-2.61 (m, 1H), 2.00-1.84 (m, 1H), 1.79-1.59 (m, 4H), 1.57-1.36 (m, 6H), 0.84 (t, J = 7.3 Hz, 3H). MS (ESI): 453.1 [M+H]⁺.

*N*-(5-chloropyridin-2-yl)-2-(6-(trifluoromethyl)-2*H*-spiro[benzofuran-3,1'-cyclohexan]-4'-yl)butanamide **(Example 10c):** (RT of 2.54 min with the following analytical SFC-Conditions: column CHIRALPAK AD-3 (4.6 x 100 mm, 3µm), mobile phase 65% CO₂/MeOH 35%, flow rate 2 mL/min, column temperature 35°C) ¹H NMR (400 MHz, DMSO-d₆): δ ppm 10.76 (s, 1H), 8.35 (s, 1H), 8.19 (d, J = 8.9 Hz, 1H), 7.88 (d, J = 8.9, 1H), 7.60 (d, J = 7.2 Hz, 1H), 7.21 (d, J = 7.2 Hz, 1H), 7.10 (s, 1H), 4.37-4.30 (m, 2H), 2.85-2.61 (m, 1H), 2.00-1.84 (m, 1H), 1.79-1.59 (m, 4H), 1.57-1.36 (m, 6H), 0.87 (t, J = 7.3 Hz, 3H). MS (ESI): 453.1 [M+H]⁺.

*N*-(5-chloropyridin-2-yl)-2-(6-(trifluoromethyl)-2*H*-spiro[benzofuran-3,1'-cyclohexan]-4'-yl)butanamide **(Example 10d):** (RT of 1.31 min with the following analytical SFC-Conditions: column CHIRALPAK AS-3 (4.6 x 100 mm, 3µm), mobile phase 90% CO₂/MeOH 10%, flow rate 2 mL/min, column temperature 35°C) ¹H NMR (400 MHz, DMSO-d₆): δ ppm 10.66 (s, 1H), 8.37 (s, 1H), 8.20 (d, J = 9.1 Hz, 1H), 7.88 (d, J = 9.1 Hz, 1H), 7.40 (d, J = 7.7 Hz, 1H), 7.20 (d, J = 7.7 Hz, 1H), 7.07 (s, 1H), 4.46-4.40 (m, 2H), 2.41-2.28 (m, 1H), 1.89-1.78 (m, 1H), 1.75-1.64 (m, 4H), 1.62-1.53 (m, 4H), 1.24-1.09 (m, 1H), 1.09-0.93 (m, 1H), 0.83 (t, J = 7.3 Hz, 3H). MS (ESI): 453.1 [M+H]⁺. *N*-(5-chloropyridin-2-yl)-2-(6-(trifluoromethyl)-2*H*-spiro[benzofuran-3,1'-cyclohexan]-4'-yl)butanamide **(Example 10e**): (RT of 1.77 min with the following analytical SFC-Conditions: column CHIRALPAK AS-3 (4.6 x 100 mm, 3µm), mobile phase 90% CO₂/MeOH 10%, flow rate 2 mL/min, column temperature 35°C) ¹H NMR (400 MHz, DMSO-d₆): δ ppm 10.66 (s, 1H), 8.37 (s, 1H), 8.20 (d, J = 9.1 Hz, 1H), 7.88 (d, J = 9.1 Hz, 1H), 7.40 (d, J = 7.7 Hz, 1H), 7.20 (d, J = 7.7 Hz, 1H), 7.07 (s, 1H), 4.46-4.40 (m, 2H), 2.41-2.28 (m, 1H), 1.89-1.78 (m, 1H), 1.75-1.64 (m, 4H), 1.62-1.53 (m, 4H), 1.24-1.09 (m, 1H), 1.09-0.93 (m, 1H), 0.83 (t, J = 7.3 Hz, 3H). MS (ESI): 453.1 [M+H]⁺.

### Examples 11b, 11c, 11d, 11e: 5-Chloro-2-(1-(6-(trifluoromethyl)-2H-spiro[benzofuran-3,1'-cyclohexan]-4'-yl)ethyl)-1H-benzo[d]imidazole (11a), (Examples 11b, 11c, 11d, 11e)

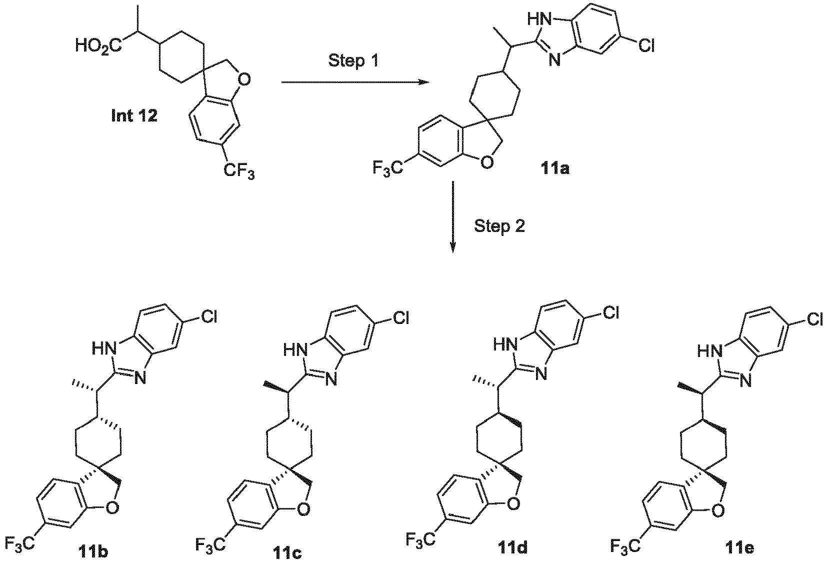

### Examples 11b, 11c, 11d, 11e, configurations not assigned

### Step 1: 5-Chloro-2-(1-(6-(trifluoromethyl)-2H-spiro[benzofuran-31'-cyclohexan]-4'-yl)ethyl)-1H-benzo[d]imidazole (11a)

A mixture of 2-(6-(trifluoromethyl)-2H-spiro[benzofuran-3,1'-cyclohexan]-4'-yl)propanoic acid **(Int 12, 328** mg, 1.00 mmol), 4-chloroaniline (142 mg, 1.00 mmol), HATU (380 mg, 1.00mmol) and DIPEA (387 mg, 3.00 mmol) in DMF (10 mL) was stirred at rt overnight. The mixture was poured into water (20 mL) and extracted with DCM (3 x 20 mL). The combined organic phase was concentrated and the residue was purified by column chromatography on silica gel (PE:EtOAc = 2:1) to give the intermediate anilide as a brown solid. The solid was dissolved in HOAc (20 mL) and was heated to reflux overnight. The mixture was poured into saturated NaHCO₃ solution (50 mL) and extracted with DCM (3 x 50 mL). The organic phase was concentrated and the residue was purified by column chromatography on silica gel (PE:EtOAc = 5:1) to give the title compound as a white solid.

5-Chloro-2-(1-(6-(trifluoromethyl)-2*H*-spiro[benzofuran-3,1'-cyclohexan]-4'-yl)ethyl)-1*H-*benzo[*d*]imidazole (**11a**) was subjected to a separation via preparative chiral SFC, using the following conditions: stationary phase CHIRALCEL OZ (Daicel), mobile phase CO₂:MeOH, 85/15. Three fractions were obtained. The first eluted fraction was concentrated to dryness and subjected to a second separation via chiral SFC, using the following conditions: stationary phase CHIRALCEL AD, CO₂:EtOH (0.3% ammonia), 85/15, giving rise to two separate isomers of 5-chloro-2-(1-(6-(trifluoromethyl)-2*H-*spiro[benzofuran-3,1'-cyclohexan]-4'-yl)ethyl)-1*H*-benzo[d]imidazole: **Example 11b,** first eluting and **Example 11c,** second eluting isomer. The second and the third fraction of the first separation on chiral SFC were concentrated to dryness to afford two isomers of 5-chloro-2-(1-(6-(trifluoromethyl)-2*H*-spiro[benzofuran-3,1'-cyclohexan]-4'-yl)ethyl)-1*H-*benzo[*d*]imidazole: **Example 11d,** second fraction and **Example 11e,** third fraction.

5-Chloro-2-(1-(6-(trifluoromethyl)-2*H*-spiro[benzofuran-3,1'-cyclohexan]-4'-yl)ethyl)-1*H-*benzo[*d*]imidazole **(Example 11b):** (RT of 7.20 min with the following analytical SFC-Conditions: column CHIRALCEL OZ-3 (4.6 x 100 mm, 3µm), mobile phase CO₂(95-90%)/MeOH (5-10%) ¹H NMR (400 MHz, DMSO-d₆): δ ppm 12.39 (d, J = 15.8 Hz, 1H), 7.64-7.51 (m, 2H), 7.49-7.43 (m, 1H), 7.21-7.04 (m, 3H), 4.34-4.27 (m, 2H), 3.21-3.17 (m, 1H), 1.88-1.81 (m, 3H), 1.78-1.29 (m, 9H). MS (ESI): 435.3 [M+H]⁺.

5-Chloro-2-(1-(6-(trifluoromethyl)-2*H*-spiro[benzofuran-3,1'-cyclohexan]-4'-yl)ethyl)-1*H-*benzo[*d*]imidazole **(Example 11c):** (RT of 7.69 min with the following analytical SFC-Conditions: column CHIRALCEL OZ-3 (4.6 x 100 mm, 3µm), mobile phase CO₂(95-90%)/MeOH (5-10%) ¹H NMR (400 MHz, DMSO-d₆): δ ppm 12.39 (d, J = 15.8 Hz, 1H), 7.64-7.51 (m, 2H), 7.49-7.43 (m, 1H), 7.21-7.04 (m, 3H), 4.34-4.27 (m, 2H), 3.21-3.17 (m, 1H), 1.88-1.81 (m, 3H), 1.78-1.29 (m, 9H). MS (ESI): 435.4 [M+H]⁺.

5-Chloro-2-(1-(6-(trifluoromethyl)-2*H*-spiro[benzofuran-3,1'-cyclohexan]-4'-yl)ethyl)-1*H-*benzo[*d*]imidazole **(Example 11d):** (RT of 8.38 min with the following analytical SFC-Conditions: column CHIRALCEL OZ-3 (4.6 x 100 mm, 3µm), mobile phase CO₂(95-90%)/MeOH (5-10%), flow rate 2 mL/min, column temperature 35°C) ¹H NMR (400 MHz, DMSO-d₆): δ ppm 12.36 (d, J = 14.9 Hz, 1H), 7.61-7.34 (m, 3H), 7.23-7.03 (m, 3H), 4.45-4.38 (m, 2H), 2.90-2.81 (m, 1H), 1.91-1.54 (m, 6H), 1.41-1.26 (m, 4H), 1.16-1.10 (m, 2H). MS (ESI): 435.3 [M+H]⁺.

5-Chloro-2-(1-(6-(trifluoromethyl)-2*H*-spiro[benzofuran-3,1'-cyclohexan]-4'-yl)ethyl)-1*H-*benzo[*d*]imidazole **(Example 11e):** (RT of 10.25 min with the following analytical SFC-Conditions: column CHIRALCEL OZ-3 (4.6 x 100 mm, 3µm), mobile phase CO₂(95-90%)/MeOH (5-10%), flow rate 2 mL/min, column temperature 35°C) ¹H NMR (400 MHz, DMSO-d₆): δ ppm 12.36 (d, J = 14.9 Hz, 1H), 7.61-7.34 (m, 3H), 7.23-7.03 (m, 3H), 4.45-4.38 (m, 2H), 2.90-2.81 (m, 1H), 1.91-1.54 (m, 6H), 1.41-1.26 (m, 4H), 1.16-1.10 (m, 2H). MS (ESI): 435.3 [M+H]⁺.

### Examples 12a and 12b: Separate isomers of (2S)-N-(4-fluorobicyclo[4.2.0]octa-1 (6),2,4-trien-7-yl)-2-((3R,4's)-6-(trifluoromethyl)-2H-spiro[benzofuran-3,1'-cyclohexan]-4'-yl)propanamide (Examples 12a, 12b)

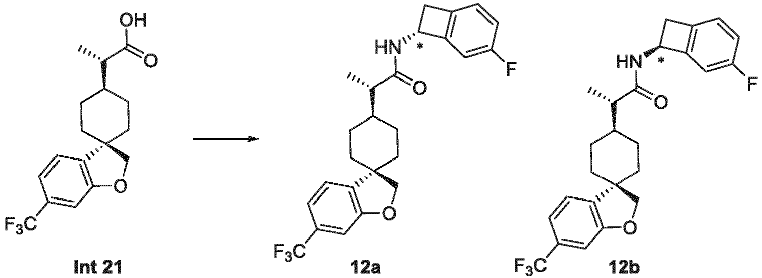

### Examples 12a, 12b * absolute configuration at this carbon not assigned

A mixture of (*S*)-2-((3R,4's)-6-(trifluoromethyl)-2*H*-spiro[benzofuran-3,1'-cyclohexan]-4'-yl)propanoic acid **(Int 21,** 38 mg, 0.115 mmol), 4-fluorobicyclo[4.2.0]octa-1,3,5-trien-7-aminium chloride (20.0 mg, 0.115 mmol), HATU (65.7 mg, 0.173 mmol) and DIPEA (0.080 mL, 0.461 mmol) in DMF (1.0 mL) were stirred overnight at rt. The mixture was diluted with EtOAc (20mL), washed with 1N aq. NH₄Cl and brine. The organic phase was dried over Na₂SO₄, filtered and concentrated to dryness. The residue was purified by silicagel flash chromatography (cyclohexane/EtOAc, gradient from 10-90% EtOAc in 10 column volumes). Two separate product fractions were obtained, pooled, concentrated to dryness and lyophilized from water/acetonitrile to obtain the separate title compounds: (2*S*)-*N*-(4-fluorobicyclo[4.2.0]octa-1(6),2,4-trien-7-yl)-2-((3*R*,4'*s*)-6-(trifluoromethyl)-2*H-*spiro[benzofuran-3,1'-cyclohexan]-4'-yl)propanamide **Example 12a,** first eluting on silica gel column, **Example 12b,** second eluting on silica gel column.

(2*S*)-*N*-(4-fluorobicyclo[4.2.0]octa-1(6),2,4-trien-7-yl)-2-((3R,4's)-6-(trifluoromethyl)-2*H-*spiro[benzofuran-3,1'-cyclohexan]-4'-yl)propanamide **(Example 12a):** ¹H-NMR (300 MHz, DMSO): δ ppm 8.54 (d, J=6.9 Hz, 1H), 7.44-7.39 (m, 1H), 7.23-7.00 (m, 5H), 5.24 (s, 1H), 4.48-4.38 (m, 2H), 3.53 (dd, J=4.8, 14.0 Hz, 1H), 2.91 (d, J=14.0 Hz, 1H), 2.11-1.97 (m, 1H), 1.87-145 (m, 7H), 1.17-0.89 (m, 5H). MS (ESI): 448.3 [M+H]⁺.

2*S*)-*N*-(4-fluorobicyclo[4.2.0]octa-1(6),2,4-trien-7-yl)-2-((3R,4's)-6-(trifluoromethyl)-2*H-*spiro[benzofuran-3,1'-cyclohexan]-4'-yl)propanamide **(Example 12b):** ¹H-NMR (300 MHz, DMSO): δ ppm 8.52 (d, J=7.3 Hz, 1H), 7.41 (d, J=7.6 Hz, 1H), 7.23-6.95 (m, 5H), 5.24 (s, br, 1H), 4.44-4.41 (m, 2H), 3.51 (dd, J=4.9, 13.6 Hz, 1H), 2.95 (d, J=14.0 Hz, 1H), 2.09-1.99 (m, 1H), 1.88-1.46 (m, 7H), 1.16-0.89 (m, 5H). MS (ESI): 448.3 [M+H]⁺.

### Example 13: (S)-N-(4-chlorophenyl)-2-((3R,4's)-6-(difluoromethyl)-2H-spiro[benzofuran-3,1'-cyclohexan]-4'-yl)propanamide (13)

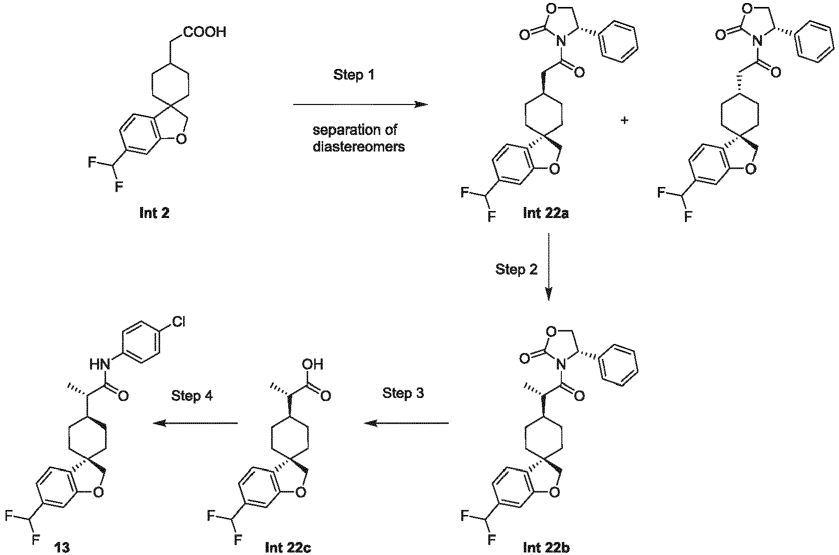

### Step 1: (S)-3-(2-((3R,4's)-6-(difluoromethyl)-2H-spiro[benzofuran-3,1'-cyclohexan]-4'-yl)acetyl)-4-phenyloxazolidin-2-one (Int 22a)

A solution of (S)-4-phenyloxazolidin-2-one (603 mg, 3.70 mmol) and LiCl (155 mg, 3.70 mmol) in EtOAc (20 mL) was heated at reflux overnight. The mixture was poured into HCl (50 mL, 1M.) and extracted by EtOAc (3 x 20 mL). The combined organic layers were concentrated to dryness. The residue was purified by column chromatography on silica gel (PE:EtOAc = 10:1) to give a white solid. The solid was submitted to chiral-HPLC separation to give the title compound which is the second eluting isomer of (S)-3-(2-(6-(difluoromethyl)-2H-spiro[benzofuran-3,1'-cyclohexan]-4'-yl)acetyl)-4-phenyloxazolidin-2-one **(Int 22a)** (RT of 2.92 min with the following SFC conditions: column OD (Chiralcel) (20 x 250 mm), mobile phase CO₂/0.2% NH₃ in Methanol 60:40, flow rate 45 mL/min).

### Step 2: (S)-3-((S)-2-((3R,4's)-6-(difluoromethyl)-2H-spiro[benzofuran-3,1'-cyclohexan]-4'-yl)propanoyl)-4-phenyloxazolidin-2-one (Int 22b)

To a solution of **Int 22a** (500 mg, 1.10 mmol) in THF (20 mL) was added LHMDS (1.6 mL, 1.60 mmol, 1M in THF) at -40 °C and the mixture was stirred for 0.5 h. Mel (315 mg, 2.20 mmol) was added and the mixture was stirred for 2 h at -40 °C. Saturated NH₄Cl solution (10 mL) was added at -40 °C and the mixture was concentrated. The residue was purified by column chromatography on silica gel (PE:EA = 10:1) to give the title compound as a white solid.

### Step 3: (S)-2-((3R,4's)-6-(difluoromethyl)-2H-spiro[benzofuran-3,1'-cyclohexan]-4'-yl)propanoic acid (Int 22c)

To a mixture of Int **22b** (200 mg, 0.44 mmol) and LiOH-H₂O (37 mg, 0.88 mmol) in THF (10 mL) was added H₂O₂ (1 mL, 30% in water). The mixture was stirred at 0 °C for 2 h. The mixture was poured into saturated NaHSO₃ solution. The mixture was extracted with EtOAc (3 x 20 mL). The combined organic layers were washed by brine, dried over anhydrous Na₂SO₄, filtered and concentrated to dryness. The residue was purified by column chromatography on silica gel (PE: EtOAc = 5: 1) to give the title compound as a white solid.

### Step 4: (S)-N-(4-chlorophenyl)-2-((3R,4's)-6-(difluoromethyl)-2H-spiro[benzofuran-3,1'-cyclohexan]-4'-yl)propanamide (13)

To a mixture of **Int 22c** (100 mg, 0.32 mmol) in DCM (10 mL) was added SOCl₂ (1 mL) and the mixture was stirred at rt for 2h. The mixture was concentrated to dryness to give a solid whisch was dissolved in DCM (10 mL). 4-Chloroaniline (61 mg, 0.48 mmol) and TEA (65 mg, 0.64 mmol) were added and the mixture was stirred at rt overnight. The mixture was poured into water (30 mL) and extracted with DCM (3 x 20 mL). The combined organic layers were concentrated to dryness and the residue was purified by preparative HPLC to give the title compound as a white solid. ¹H NMR (400 MHz, DMSO-d₆): δ ppm 9.99 (s, 1H), 7.71-7.60 (m, 2H), 7.42-7.26 (m, 3H), 7.07 - 6.77 (m, 3H), 4.44-4.36 (m, 2H), 2.28-2.20 (m, 1H), 1.88-1.55 (m, 7H), 1.20-0.94 (m, 5H). MS (ESI): m/z 420.1 [M+H]+.

### Examples 14a, 14b: Separate isomers of N-(4-chloro-2-fluorophenyl)-2-(6-(trifluoromethyl)-2H-spiro[benzofuran-3,1'-cyclohexan]-4'-yl)acetamide (14a, 14b)

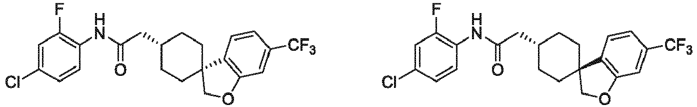

### Examples 14a, 14b, configurations not assigned

The title compounds were prepared simlar as described for Examples **1b** and **1c**, steps 1 and 2, using in step 1 2-(6-(trifluoromethyl)-2*H*-spiro[benzofuran-3,1'-cyclohexan]-4'-yl)acetic acid **(Int 21c,** step 3 in Examples **3b** and **3d**) in place of 2-(2',3'-dihydrospiro[cyclohexane-1,1'-inden]-4-yl)acetic acid **(Int 1).**

First eluting isomer of *N*-(4-chloro-2-fluorophenyl)-2-(6-(trifluoromethyl)-2*H-*spiro[benzofuran-3,1'-cyclohexan]-4'-yl)acetamide **(Example 14a)** (RT of 1.98 min with the following SFC conditions: column AD (Daicel) (20 x 250 mm), mobile phase CO₂/1% NH₃ in Ethanol 70:30, flow rate 80 mL/min): ¹H NMR (500 MHz, DMSO-d₆): δ ppm 9.80 (s, 1H), 7.93 (dd, J₁ = J₂ = 8.6 Hz, 1H), 7.61 (d, J = 7.8 Hz, 1H), 7.48 (dd, J₁ = 10.7, J₂ = 2.3 Hz, 1H), 7.28-7.21 (m, 2H), 7.11 (s, 1H), 4.36 (s, 2H), 2.54 (d, J = 7.4 Hz, 2H), 2.10-2.02 (m, 1H), 1.91-1.78 (m, 2H), 1.72-1.62 (m, 2H), 1.62-1.47 (m, 4H). MS (ESI): m/z 442.0 [M+H]⁺.

Second eluting isomer of *N*-(4-chloro-2-fluorophenyl)-2-(6-(trifluoromethyl)-2*H-*spiro[benzofuran-3,1'-cyclohexan]-4'-yl)acetamide **(Example 14b)** (RT of 2.58 min with the following SFC conditions: column AD (Daicel) (20 x 250 mm), mobile phase CO₂/1% NH₃ in Ethanol 70:30, flow rate 80 mL/min): ¹H NMR (500 MHz, DMSO-d₆): δ ppm 9.77 (s, 1H), 7.90 (dd, J₁ = J₂ = 8.6 Hz, 1H), 7.47 (dd, J₁ = 10.7, J₂ = 2.3 Hz, 1H), 7.43 (d, J = 7.7 Hz, 1H), 7.24 (d, J = 8.7 Hz, 1H), 7.21 (d, J = 7.4 Hz, 1H), 7.08 (s, 1H), 4.46 (s, 2H), 2.32 (d, J = 7.1 Hz, 2H), 1.91-1.84 (m, 1H), 1.81-1.62 (m, 6H), 1.18-1.05 (m, 2H). MS (ESI): m/z 442.0 [M+H]⁺.

If one were to use similar procedures as that desdribed for Example 13, steps 1-4 using in step 1 either (S)-4-phenyloxazolidin-2-one or (R)-4-phenyloxazolidin-2-one and in step 2 either the first or the second eluting diastereomer the following compounds, next to Example 13, would be obtained:

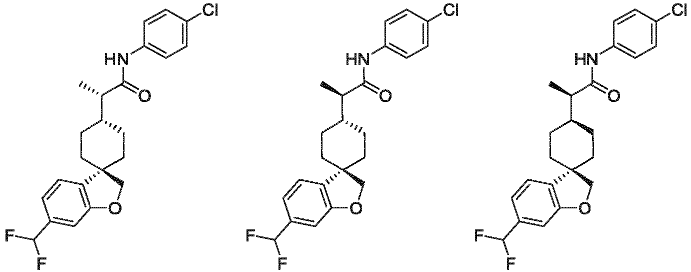

### Biological Assays

### SKOV-3 cellular Indoleamine 2,3-dioxygenase assay

SKOV-3 cells were obtained from the American Type Culture Collection (ATCC^{®} HTB-77^{™}) and maintained in McCoy's medium (Pan Biotech) supplemented with 10 % fetal bovine serum and 1 % Penicillin/Streptomycin. Cells were kept at 37° C in a humidified incubator with 5 % CO₂. For assay preparation, cells were seeded at a density of 2*10⁵/ml into black clear bottom 96 well plates in 100 µl medium/well supplemented with 50 ng/ml Interferon gamma (eBioscience, Thermo Fisher Scientific). After cells fully adhered to the plate, dilution series of compounds were added in medium containing additional L-Tryptophan to a final L-Tryptophan concentration of 100 µM. The cells were incubated for 24 hours. Detection of produced N-Formylkynurenin was performed by addition of 3-Methylpiperidine to a final concentration of 200 mM. The plates were sealed and heated to 65° C for 20 minutes in a water bath. After cooling the fluorescence of each well was recorded with a Victor^{™}X4 (PerkinElmer) plate reader at an emission wavelength of 535 nm and excitation at 405 nm (Tomek et al.; Anal Bioanal Chem (2013) 405:2515-2524., Tomek et al.; Biochim Biophys Acta. 2015 Sep;1850(9):1772-80).

The IC₅₀ values of the example compounds are shown in Table 1 below (A = IC₅₀ < 50 nM, B = 50 nM ≤ IC₅₀ ≤ 1 µM, C = IC₅₀ > 1 µM).

| **Example #** | **cell based activity** |
|---|---|
| **1b** | B |
| **1c** | A |
| **2a** | A |
| **2b** | A |
| **3b** | A |
| **3c** | A |
| **3d** | A |
| **3e** | B |
| **4b** | B |
| **4c** | B |
| **4d** | B |
| **4e** | A |
| **5b** | A |
| **5c** | A |
| **5d** | B |
| **5e** | A |
| **6b** | B |
| **6c** | A |
| **6d** | B |
| **6e** | B |
| **7b** | A |
| **7c** | A |
| **7d** | A |
| **7e** | B |
| **8b** | A |
| **8c** | A |
| **8d** | A |
| **8e** | B |
| **9b** | B |
| **9c** | B |
| **9d** | B |
| **9e** | A |
| **10b** | A |
| **10c** | A |
| **10d** | A |
| **10e** | B |
| **11b** | B |
| **11c** | A |
| **11d** | A |
| **11e** | A |
| **12a** | B |
| **12b** | B |
| **13** | A |
| **14a** | A |
| **14b** | B |

## Claims

1. A compound according to Formula (1) or (2) 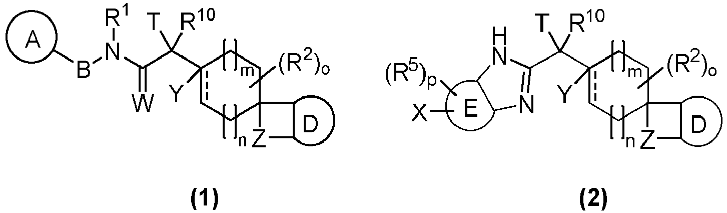
an enantiomer, diastereomer, tautomer or pharmaceutically acceptable salt thereof wherein
A represents C₃₋₁₀-cycloalkyl, which may be optionally fused with a phenyl ring being unsubstituted or substituted with 1 to 3 R^{a}, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl,
or
two substituents on the same carbon atom or on two different carbon atoms form together with the carbon atom to which they are attached a C₃₋₁₀-cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo;
R^{a} represents halogen, CN, C₁₋₆-alkyl, halo-C₁₋₆-alkyl, hydroxy-C₁₋₆-alkyl, C₃₋₆-cycloalkyl or halo-C₃₋₆-cycloalkyl;
R^{x} represents C₁₋₆-alkyl, C₃₋₆-cycloalkyl or 3- to 6-membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo-C₁₋₆-alkyl,OR¹ and CN;
B represents a bond or C₁₋₂-alkylene,
wherein alkylene is unsubstituted or substituted with one or two C₁₋₄-alkyl;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from the group consisting of halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S, and 6-membered aryl,
wherein aryl and heteroaryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl,
or
two substituents on the aryl or heteroaryl ring systems together with the carbon atoms to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl,CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
E represents a 6-membered aryl or 6-membered heteroaryl containing 1 to 2 nitrogen atoms;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl, or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
R¹⁰ represents hydrogen, halogen, C₁₋₆-alkyl, halo-C₁₋₆-alkyl; or
R¹⁰ and T form together with the carbon atom to which they are attached a 3- to 10-membered mono- or bicyclic ring system which is saturated or partially unsaturated and wherein the ring system may further contain 1, 2 or 3 heteroatoms independently selected from N, O and S,
wherein the ring system is unsubstituted or substituted with 1 to 5 substituents independently selected from the group consisting of halogen, CN, OH, OC₁₋₆-alkyl, oxo, C₁₋₆-alkyl and halo-C₁₋₆-alkyl;
W represents O, NOR⁴, NR¹, NCN, or NS(O)₂C₁₋₆-alkyl;
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl,OR¹ or CN;
Y is absent or represents hydrogen, OR⁴, halogen, C₁₋₆-alkyl or halo-C₁₋₆-alkyl;
Z represents -C₂₋₃-alkylene-, -O-C₁₋₂-alkylene-, -C₁₋₂-alkylene-O-, -NR³C(O)-C₀₋₁-alkylene-, -C(O)NR³-C₀₋₁-alkylene-, -C₀₋₁-alkylene-NR³C(O)-, -C₀₋₁-alkylene C(O)NR³-, -S(O)ₜ-C₁₋₂-alkylene-, -C₁₋₂-alkylene-S(O)ₜ-, -NR⁹-C₁₋₂-alkylene- or -C₁₋₂-alkylene-NR⁹-,
wherein alkylene is unsubstituted or substituted with 1 to 4 substituents independently selected from the group consisting of OR⁴, C₁₋₆-alkyl, halogen and halo-C₁₋₆-alkyl;
R⁹ is hydrogen, C₁₋₆-alkyl, halo-C₁₋₆-alkyl, C(O)-C₁₋₆-alkyl, or C(O)-halo-C₁₋₆-alkyl;
R¹ is hydrogen or C₁₋₆-alkyl;
R² is halogen, C₁₋₆-alkyl, C₃₋₆-cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, C(O)N(R¹)₂, CN, C(O)OR⁴ or oxo,
or
two R² on the same carbon atom form together with the carbon atom to which they are attached a C₃₋₁₀ cycloalkyl group,
or
two R² at different carbon atoms form together a -CH₂-, -CH(CH₃)-, -C(CH₃)₂-, CH₂-CH(CH₃)-, -CH₂-CH₂- or -CH₂-CH₂-CH₂- group;
R³ is hydrogen or C₁₋₆-alkyl;
R⁴ is hydrogen or C₁₋₆-alkyl;
m is 0, 1 or 2;
n is 0, 1 or 2;
o is 0, 1, 2, 3 or 4;
p is 0, 1, 2 or 3; and
t is 0, 1 or 2.

2. The compound of Formula (1) or (2) according to claim 1 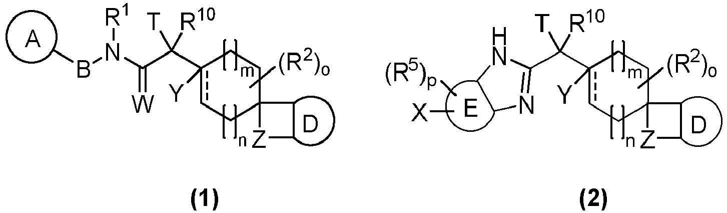
an enantiomer, diastereomer, tautomer or pharmaceutically acceptable salt thereof wherein
A represents C₃₋₁₀-cycloalkyl, which may be optionally fused with a phenyl ring, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl,
or
two substituents on the same carbon atom or on two different carbon atoms form together with the carbon atom to which they are attached a C₃₋₁₀-cycloalkyl group, wherein optionally one carbon atom in the cycloalkyl ring may be replaced by a heteroatom selected from O, N and S and wherein the (hetero)cyclic ring may be unsubstituted or substituted by 1 to 3 substituents independently selected from the group consisting of halogen, C₁₋₆-alkyl, halo-C₁₋₆-alkyl or oxo;
R^{x} represents C₁₋₆-alkyl, C₃₋₆-cycloalkyl or 3- to 6-membered heterocyclyl containing 1 to 2 heteroatoms independently selected from O, N and S,
wherein alkyl, cycloalkyl and heterocycloalkyl are unsubstituted or substituted with 1 or 6 substituents independently selected from the group consisting of halogen, C₁₋₆-alkyl, halo-C₁₋₆-alkyl, OR¹ and CN;
B represents a bond or C₁₋₂-alkylene,
wherein alkylene is unsubstituted or substituted with one or two C₁₋₄-alkyl;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from the group consisting of halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S, and 6-membered aryl,
wherein aryl and heteroaryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl,
or
two substituents on the aryl or heteroaryl ring systems together with the carbon atoms to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
E represents a 6-membered aryl or 6-membered heteroaryl containing 1 to 2 nitrogen atoms;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl, or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
R¹⁰ represents hydrogen, halogen, C₁₋₆-alkyl, halo-C₁₋₆-alkyl; or
R¹⁰ and T form together with the carbon atom to which they are attached a 3- to 10-membered mono- or bicyclic ring system which is saturated or partially unsaturated and wherein the ring system may further contain 1, 2 or 3 heteroatoms independently selected from N, O and S,
wherein the ring system is unsubstituted or substituted with 1 to 5 substituents independently selected from the group consisting of halogen, CN, OH, OC₁₋₆-alkyl, oxo, C₁₋₆-alkyl and halo-C₁₋₆-alkyl;
W represents O, NOR⁴, NR¹, NCN, or NS(O)₂C₁₋₆-alkyl-,
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
Y is absent or represents hydrogen, OR⁴, halogen, C₁₋₆-alkyl or halo-C₁₋₆-alkyl;
Z represents -C₂₋₃-alkylene-, -O-C₁₋₂-alkylene-, -C₁₋₂-alkylene-O-, -NR³C(O)-C₀₋₁-alkylene-, -C(O)NR³-C₀₋₁-alkylene-, -C₀₋₁-alkylene-NR³C(O)-, -C₀₋₁-alkylene C(O)NR³-, -S(O)ₜ-C₁₋₂-alkylene-, -C₁₋₂-alkylene-S(O)ₜ-, -NR⁹-C₁₋₂-alkylene- or -C₁₋₂-alkylene-NR⁹-, wherein alkylene is unsubstituted or substituted with 1 to 4 substituents independently selected from the group consisting of OR⁴, C₁₋₆-alkyl, halogen and halo-C₁₋₆-alkyl;
R⁹ is hydrogen, C₁₋₆-alkyl, halo-C₁₋₆-alkyl, C(O)-C₁₋₆-alkyl, or C(O)-halo-C₁₋₆-alkyl;
R¹ is hydrogen or C₁₋₆-alkyl;
R² is C₁₋₆-alkyl, C₃₋₆-cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, C(O)N(R¹)₂, CN, C(O)OR⁴ or oxo,
or
two R² on the same carbon atom form together with the carbon atom to which they are attached a C₃₋₁₀cycloalkyl group,
or
two R² at different carbon atoms form together a -CH₂-, -CH(CH₃)-, -C(CH₃)₂-, CH₂₋CH(CH3)-, -CH₂-CH₂- or -CH₂-CH₂-CH₂- group;
R³ is hydrogen or C₁₋₆-alkyl;
R⁴ is hydrogen or C₁₋₆-alkyl;
m is 0, 1 or 2;
n is 0, 1 or 2;
o is 0, 1, 2, 3 or 4;
p is 0, 1, 2 or 3; and
t is 0, 1 or 2.

3. The compound according to claim 1 which is represented by the following formulae (1-1) and (1-2) 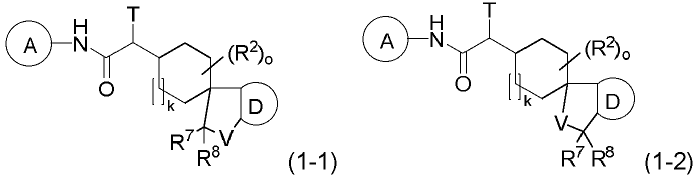 wherein
A represents C₃₋₁₀ cycloalkyl, which may be optionally fused with a phenyl ring being unsubstituted or substituted with 1 to 3 R^{a}, 3- to 10-membered heterocycloalkyl containing 1 to 4 heteroatoms independently selected from O, N and S, 6- to 10-membered mono or bicyclic aryl or 5- to 10-membered mono or bicyclic heteroaryl containing 1 to 4 heteroatoms independently selected from O, N and S,
wherein cycloalkyl, heterocycloalkyl, aryl and heteroaryl are unsubstituted or substituted with 1 to 5 substituents independently selected from the group consisting of halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- or 6-membered heteroaryl containing 1 to 4 heteroatoms independently selected from O, N and S and 6-membered aryl,
wherein heteroaryl and aryl are unsubstituted or substituted with 1 to 5 substituents independently selected from the group consisting of halogen, CN, OR¹, C₁₋₆-alkyl, C₃₋₆-cycloalkyl and halo-C₁₋₆-alkyl,
or
two substituents on the same carbon atom or on two different carbon atoms form together with the carbon atom to which they are attached a C₃₋₁₀ cycloalkyl group, wherein optionally one carbon atom in the cycloalkyl ring may be replaced by a heteroatom selected from O, N and S;
R^{a} represents halogen, CN, C₁₋₆-alkyl, halo-C₁₋₆-alkyl, or hydroxy-C₁₋₆-alkyl;
R^{x} represents C₁₋₆-alkyl, C₃₋₆-cycloalkyl or 3- to 6 membered heterocycloalkyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
or
two substituents on the aryl or heteroaryl ring systems together with the carbon atom to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl,CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
V is O or CR⁷R⁸;
R² is C₁₋₆ alkyl, C₃₋₆ cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, S(O)₂N(R¹)₂, C(O)N(R¹)₂, CN, C(O)OR⁴ or oxo,
or
two R² on the same carbon atom form together with the carbon atom to which they are attached a C₃₋₁₀ cycloalkyl group,
or
two R² at different carbon atoms form together a -CH₂-, -CH(CH₃)-, -C(CH₃)₂-, CH₂-CH(CH3)-, -CH₂-CH₂- or -CH₂-CH₂-CH₂- group;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S, and 6-membered aryl;
o is 0, 1, 2, 3 or 4; and
k is 1 or 2.

4. The compound according to any one of claims 1 to 3 which is represented by the following formulae (1-3) and (1-4) 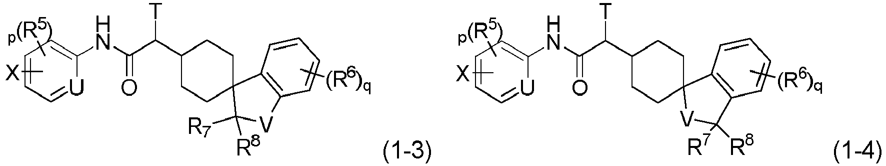 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
U is N or CR⁵;
V is O or CR⁷R⁸;
p is 0, 1, 2 or 3; and
q is 0, 1, 2, 3 or 4.

5. The compound according to any one of claims 1 to 4 which is represented by the following formula (1-5) 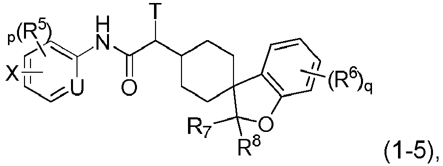 wherein X, U, T, R⁵ to R⁸, p and q are defined as in claim 4.

6. The compound according to any one of claims 1 to 3 which is represented by the following formula (1-6) 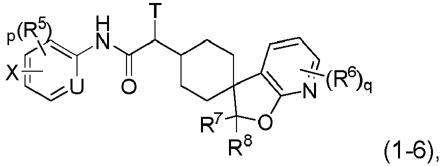 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
U is N or CR⁵;
p is 0, 1, 2 or 3; and
q is 0, 1, 2, 3 or 4.

7. The compound according to claim 1 or 2 which is represented by the following formula (1-7) 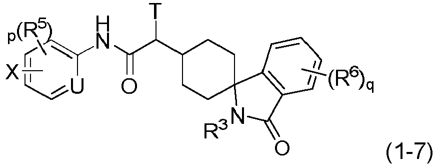 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R³ is hydrogen or C₁₋₆-alkyl;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
U is N or CR⁵;
p is 0, 1, 2 or 3; and
q is 0, 1, 2, 3 or 4.

8. The compound according to claim 1 or 2 which is represented by the following formulae (2-1) and (2-2) 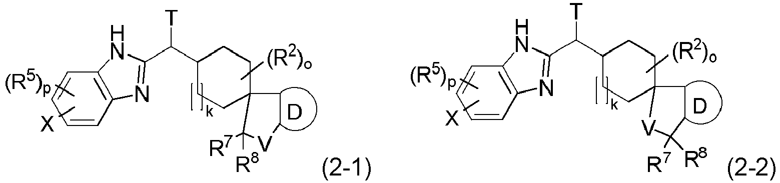 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
R^{x} represents C₁₋₆-alkyl, C₃₋₆-cycloalkyl or 3- to 6 membered heterocycloalkyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
D represents 6- to 10-membered mono- or bicyclic aryl or 5- to 10-membered mono- or bicyclic heteroaryl containing 1 to 4 heteroatoms independently selected from O, N and S,
wherein aryl and heteroaryl are unsubstituted or substituted with 1 to 5 substituents independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
or
two substituents on the aryl or heteroaryl ring systems together with the carbon atom to which they are attached form a 5- or 6-membered heterocyclic ring containing 1 or 2 heteroatoms independently selected from O, N and S,
wherein the heterocylic ring is unsubstituted or substituted with 1 or 2 substituents independently selected from the group consisting of halogen, C₁₋₆-alkyl, halo-C₁₋₆-alkyl, CN and oxo;
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
V is O or CR⁷R⁸;
R² is C₁₋₆ alkyl, C₃₋₆ cycloalkyl, halo-C₁₋₆-alkyl, OR⁴, S(O)₂N(R¹)₂, S(O)₂-C₁₋₆-alkyl, S(O)₂-C₃₋₆-cycloalkyl, S(O)₂-halo-C₁₋₆-alkyl, S(O)₂N(R¹)₂, C(O)N(R¹)₂, CN, C(O)OR⁴ or oxo,
or
two R² on the same carbon atom form together with the carbon atom to which they are attached a C₃₋₁₀ cycloalkyl group,
or
two R² at different carbon atoms form together a -CH₂-, -CH(CH₃)-, -C(CH₃)₂-, CH₂-CH(CH3)-, -CH₂-CH₂- or -CH₂-CH₂-CH₂- group;
R⁷ and R⁸ are independently selected from the group consisting of hydrogen, halogen, C₁₋₆ alkyl, halo-C₁₋₆-alkyl and OR⁴;
o is 0, 1, 2, 3 or 4; and
k is 1 or 2.

9. The compound according to any one of claims 1, 2 and 8, which is represented by the following formulae (2-3) and (2-4) 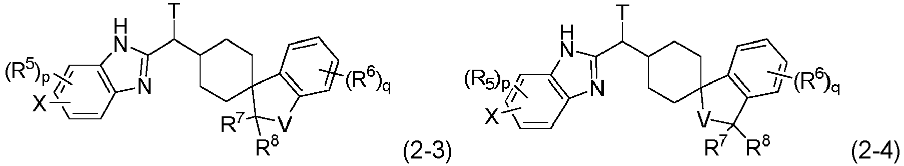 wherein
X is hydrogen, halogen, C₁₋₆-alkyl, C₃₋₆-cycloalkyl, O-C₁₋₆-alkyl, S-C₁₋₆-alkyl, CN or 5- or 6-membered heteroaryl containing 1 to 4 heteroatoms independently selected from O, N and S,
wherein alkyl and cycloalkyl are unsubstituted or substituted with halogen, C₁₋₆-alkyl, halo-C₁₋₆-alkyl, OR¹ or CN;
R⁵ is independently selected from hydrogen, halogen and C₁₋₆-alkyl;
R⁶ is independently selected from halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- or 6-membered heteroaryl containing 1 to 4 heteroatoms independently selected from O, N and S and 6-membered aryl,
wherein aryl and heteroaryl are unsubstituted or substituted with 1 to 5 substituents independently selected from the group consisting of halogen, CN, OR¹, C₁₋₆-alkyl; C₃₋₆-cycloalkyl, and halo-C₁₋₆-alkyl,
R^{z} represents C₁₋₆-alkyl, C₃₋₆-cycloalkyl or 3- to 6 membered heterocyclyl containing 1 to 2 heteroatoms independently selected from O, N and S,
wherein alkyl, cycloalkyl and heterocycloalkyl are unsubstituted or substituted with 1 to 6 substituents independently selected from the group consisting of halogen, C₁₋₆-alkyl, halo-C₁₋₆-alkyl, OR¹ and CN;
R⁷ and R⁸ are independently selected from the group consisting of hydrogen, halogen, C₁₋₆ alkyl, halo-C₁₋₆-alkyl and OR⁴;
T represents hydrogen, halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-alkyl, halo-C₁₋₆-alkyl, C₂₋₆-alkenyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-R^{x}, -C₀₋₆-alkylene-O-R^{x}, -C₀₋₆-alkylene-OC(O)-R^{x}, -C₀₋₆-alkylene-S-R^{x}, -C₀₋₆-alkylene-S(O)₂-R^{x}, -C₀₋₆-alkylene-S(O)₂N(R¹)₂, -C₀₋₆-alkylene-N(R¹)₂, -C₀₋₆-alkylene-NR¹C(O)R^{x}, -C₀₋₆-alkylene-C(O)N(R¹)₂, -C₀₋₆-alkylene-C(O)O-R^{x}, -C₀₋₆-alkylene-C(O)-R^{x}, -C₀₋₆-alkylene-CN, -C₀₋₆-alkylene-COOH, 5- or 6-membered heteroaryl containing 1 to 4 heteroatoms independently selected from O, N and S and 6-membered aryl;
V is O or CR⁷R⁸;
p is 0, 1, 2 or 3; and
q is 0, 1, 2, 3 or 4.

10. The compound according to any one of claims 1, 2, 8 and 9, which is represented by the following formula (2-5) 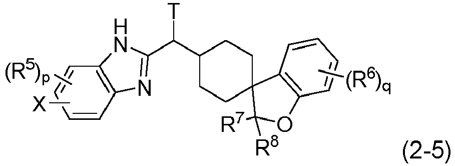 wherein X, T, R⁵ to R⁸, p and q are defined as in claim 8.

11. The compound according to any one of claims 1 to 10, wherein
T represents hydrogen, halogen, CN, C₁₋₆-alkyl, halo-C₁₋₆-alkyl, C₃₋₆-cycloalkyl, 6-membered aryl, 5- to 6-membered heteroaryl, 3- to 6-membered heterocyclyl, 6-membered aryl-C₁₋₆ alkyl, 5- to 6-membered heteroaryl-C₁₋₆-alkyl, 3- to 6-membered heterocyclyl-C₁₋₆-alkyl or 3- to 6-membered cycloalkyl-C₁₋₆-alkyl,
wherein alkyl, cycloalkyl, heterocycloalkyl, aryl and heteroaryl are unsubstituted or substituted with 1 to 5 substituents independently selected from the group consisting of halogen, -C₀₋₆-alkylene-OH, -C₀₋₆-alkylene-CN, -C₀₋₆-alkylene-R^{x} and -C₀₋₆-alkylene-O-R^{x}.

12. The compound according to any one of claims 1 to 11, wherein T represents hydrogen, C₁₋₆-alkyl, halo-C₁₋₆-alkyl, C₃₋₆-cycloalkyl, 3- to 6 membered cycloalkyl-C₁₋₆-alkyl,
wherein alkyl and cycloalkyl are unsubstituted or substituted with 1 to 5 substituents independently selected from the group consisting of C₁₋₆-alkyl, halo-C₁₋₆-alkyl, C₃₋₆-cycloalkyl, halogen, CN, OH and O-C₁₋₆-alkyl.

13. The compound according to any one of claims 1 to 7, 11 and 12, wherein the compound is selected from the group consisting of the following compounds 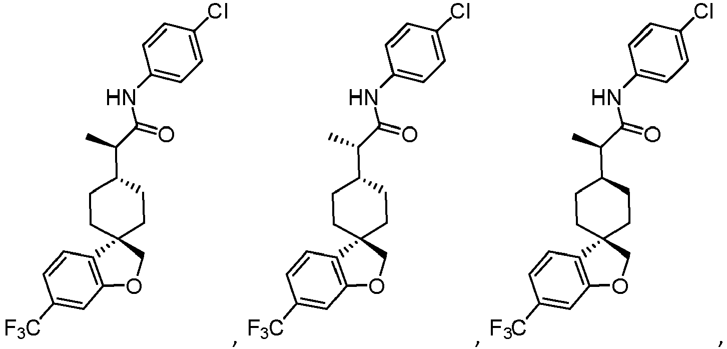 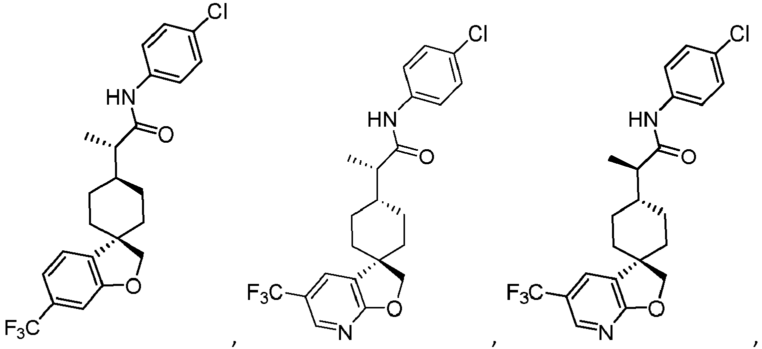 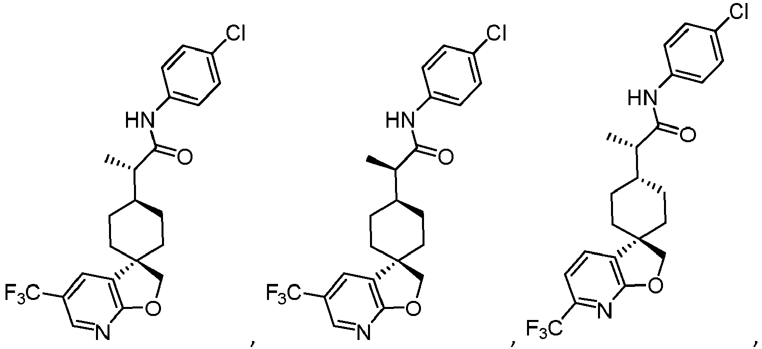 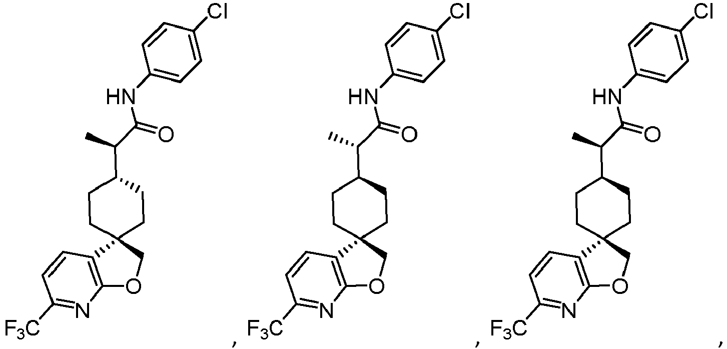 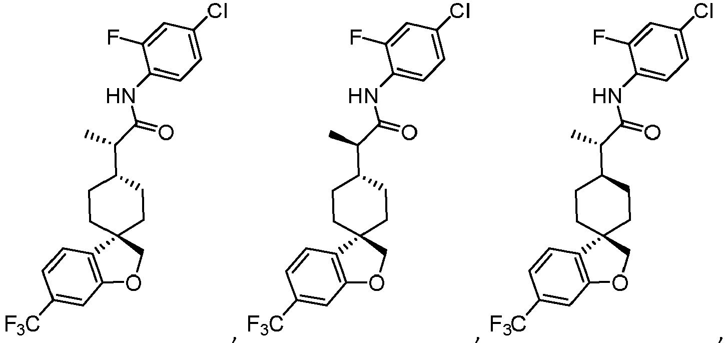 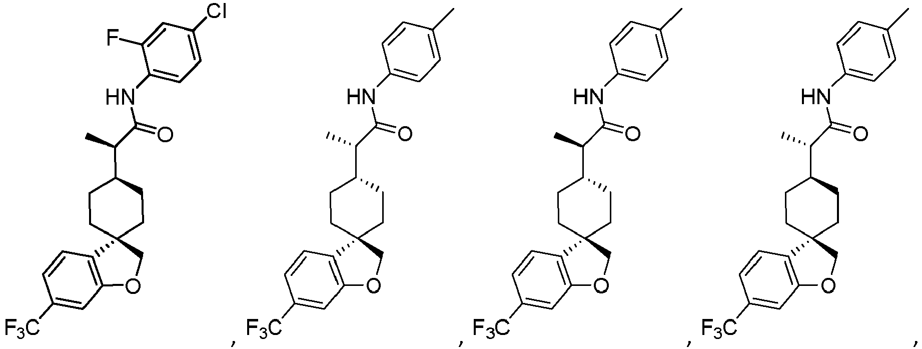 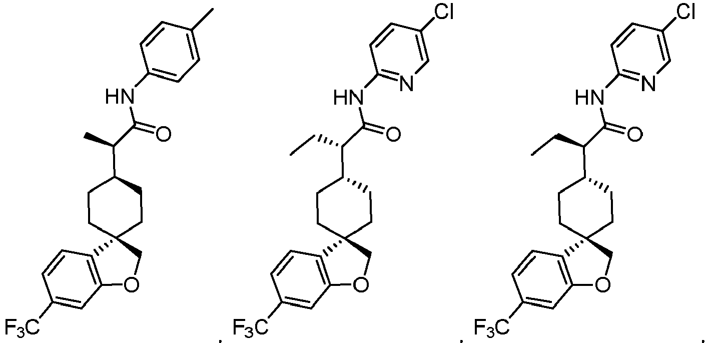 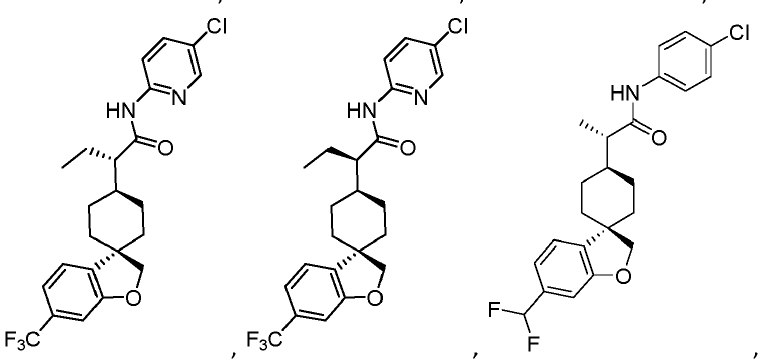 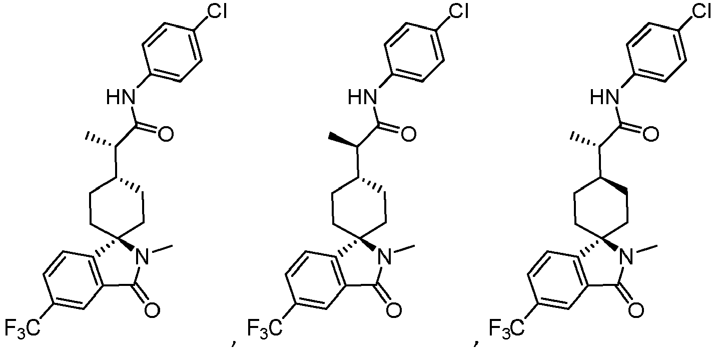 or 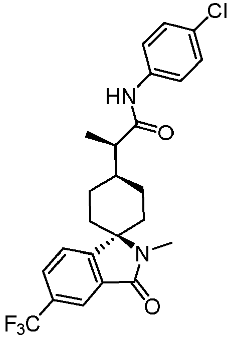 and their pharmaceutically acceptable salts thereof.

14. The compound according to any one of claims 1, 2, 8, 9, 10, 11 and 12 wherein the compound is selected from the group consisting of the following compounds 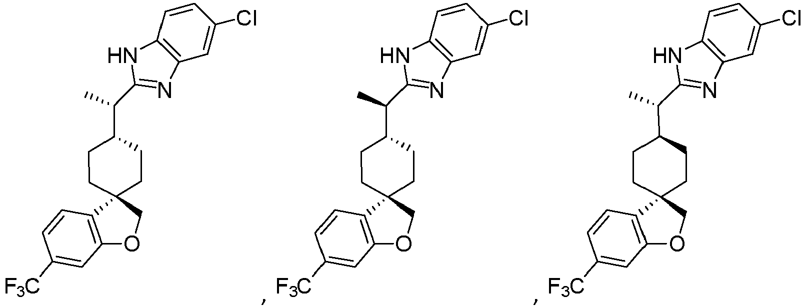 and 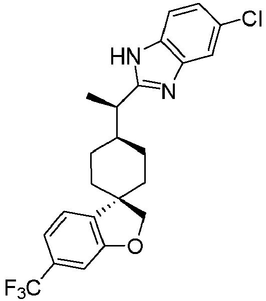 and their pharmaceutically acceptable salts thereof.

15. The compound according to any one of claims 1 to 14 for use as medicament.

16. The compound according to any one of claims 1 to 14 for use in the prophylaxis and/or treatment of a disease or condition mediated by indoleamine 2,3-dioxygenase, wherein the disease or condition is selected from the group consisting of cancer, viral and bacterial infections, depression, epilepsy, schizophrenia, neurodegenerative diseases, trauma, age-related cataracts, organ transplantation, cardiovascular disease, endometriosis, type-2 diabetic nephropathy, chronic obstructive pulmonary disease (COPD), osteoporosis, asthma, rheumatoid arthritis, multiple sclerosis, inflammatory bowel disease, psoriasis, and systemic lupus erythematosus.

17. The compound for use according to claim 16, wherein the viral and bacterial infection is selected from the group consisting of HIV infection, hanta virus infection, tuberculosis or leprae; or wherein the neurodegenerative disease is selected from the group consisting of Parkinson's disease, Alzheimer's disease and Hungtington's disease.

18. The compound for use according to claim 16, wherein the disease or condition is cancer.

19. The compound for use according to claim 18, wherein the compound is administered with one or more therapeutic agents for cancer selected from the group consisting of PD-1 agent, PD-L1 agent, CTLA-4 agent, AhR modulator, chemotherapeutic agent, anticancer vaccine, onolytic viruses, TLR agonists, STING agonists, and cytokine therapy as well as other immuno oncology, or wherein the compound is administered under irradiation therapy.

20. A pharmaceutical composition comprising a compound according to any one of claims 1 to 14 and pharmaceutically acceptable excipients.

## Patentansprüche

1. Verbindung gemäß Formel (1) oder (2) 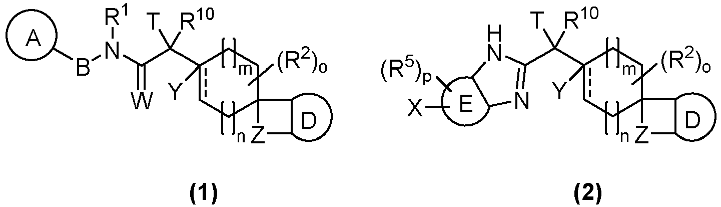 ein Enantiomer, Diastereomer, Tautomer oder pharmazeutisch akzeptables Salz davon
wobei
A C₃₋₁₀-Cycloalkyl, das gegebenenfalls mit einem Phenylring kondensiert sein kann, der unsubstituiert oder mit 1 bis 3 R^{a} substituiert ist, 3- bis 10-gliedriges Heterocycloalkyl, das 1 bis 4 Heteroatome, unabhängig ausgewählt aus O, N und S, enthält, 6- bis 10-gliedriges mono- oder bicyclisches Aryl oder 5- bis 10-gliedriges mono- oder bicyclisches Heteroaryl, das 1 bis 4 Heteroatome, unabhängig ausgewählt aus O, N und S, enthält, darstellt
wobei Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, OH, R^{x}, O-R^{x}, OC(O)-R^{x}, S-R^{x}, S(O)₂-Rx, S(O)₂N(R¹)₂, N(R¹)₂, NR¹C(O)R^{x}, C(O)N(R¹)₂, C(O)O-R^{x}, C(O)-R^{x}, CN, COOH, 5- oder 6-gliedrigem Heteroaryl, das 1 bis 4 Heteroatome enthält, die unabhängig voneinander aus O, N und S ausgewählt sind, und 6-gliedrigem Aryl,
wobei Heteroaryl und Aryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an demselben Kohlenstoffatom oder an zwei verschiedenen Kohlenstoffatomen zusammen mit dem Kohlenstoffatom, an das sie gebunden sind, eine C₃₋₁₀-Cycloalkylgruppe bilden, wobei gegebenenfalls ein Kohlenstoffatom in dem Cycloalkylring durch ein Heteroatom, ausgewählt aus O, N und S, ersetzt sein kann und wobei der (hetero)cyclische Ring unsubstituiert oder durch 1 bis 3 Substituenten, unabhängig ausgewählt aus der Gruppe bestehend aus Halogen, C₁₋₆-Alkyl, Halogen-C₁₋₆-alkyl oder Oxo, substituiert sein kann;
R^{a} Halogen, CN, C₁₋₆-Alkyl, Halogen-C₁₋₆-alkyl, Hydroxy-C₁₋₆-alkyl, C₃₋₆-Cycloalkyl oder Halogen-C₃₋₆-cycloalkyl darstellt;
R^{x} C₁₋₆-Alkyl, C₃₋₆-Cycloalkyl oder 3- bis 6-gliedriges Heterocyclyl mit 1 bis 2 Heteroatomen, unabhängig ausgewählt aus O, N und S, darstellt,
wobei Alkyl, Cycloalkyl und Heterocycloalkyl unsubstituiert oder mit 1 oder 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
B eine Bindung oder C₁₋₂-Alkylen darstellt,
wobei Alkylen unsubstituiert oder mit einem oder zwei C₁₋₄-Alkylgruppen substituiert ist;
D 6- bis 10-gliedriges mono- oder bicyclisches Aryl oder 5- bis 10-gliedriges mono- oder bicyclisches Heteroaryl, das 1 bis 4 Heteroatome enthält, die unabhängig voneinander aus O, N und S ausgewählt sind, darstellt,
wobei Aryl und Heteroaryl unsubstituiert oder mit 1 bis 5 Substituenten substituiert sind, die unabhängig voneinander aus der Gruppe bestehend aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig voneinander aus O, N und S ausgewählt sind, und 6-gliedrigem Aryl ausgewählt sind,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an den Aryl- oder Heteroaryl-Ringsystemen zusammen mit den Kohlenstoffatomen, an die sie gebunden sind, einen 5- oder 6-gliedrigen heterocyclischen Ring bilden, der 1 oder 2 Heteroatome enthält, die unabhängig voneinander aus O, N und S ausgewählt sind,
wobei der heterocyclische Ring unsubstituiert oder mit 1 oder 2 Substituenten substituiert ist, die unabhängig voneinander aus der Gruppe bestehend aus Halogen, C₁₋₆-Alkyl, Halogen-C₁₋₆-alkyl, CN und Oxo ausgewählt sind;
R^{z} C₁₋₆-Alkyl, C₃₋₆-Cycloalkyl oder 3- bis 6-gliedriges Heterocyclyl, das 1 bis 2 Heteroatome enthält, die unabhängig voneinander aus O, N und S ausgewählt sind, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
E ein 6-gliedriges Aryl oder 6-gliedriges Heteroaryl mit 1 bis 2 Stickstoffatomen darstellt;
R⁵ unabhängig ausgewählt ist aus Wasserstoff, Halogen und C₁₋₆-Alkyl;
T Wasserstoff, Halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl, oder 3- bis 6-gliedriges Cycloalkyl-C₁₋₆-alkyl darstellt,
worin Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-Rx, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl mit 1 bis 4 Heteroatomen, die unabhängig voneinander aus O, N und S ausgewählt sind, und 6-gliedrigem Aryl ausgewählt sind;
R¹⁰ Wasserstoff, Halogen, C₁₋₆-Alkyl, Halogen-C₁₋₆-alkyl darstellt; oder
R¹⁰ und T zusammen mit dem Kohlenstoffatom, an das sie gebunden sind, ein 3- bis 10-gliedriges mono- oder bicyclisches Ringsystem bilden, das gesättigt oder teilweise ungesättigt ist und wobei das Ringsystem weiterhin 1, 2 oder 3 Heteroatome enthalten kann, die unabhängig voneinander aus N, O und S ausgewählt sind,
wobei das Ringsystem unsubstituiert oder mit 1 bis 5 Substituenten substituiert ist, die unabhängig voneinander aus der Gruppe bestehend aus Halogen, CN, OH, OC₁₋₆-Alkyl, Oxo, C₁₋₆-Alkyl und Halogen-C₁₋₆-alkyl ausgewählt sind;
W O, NOR⁴, NR¹, NCN oder NS(O)₂-C₁₋₆-Alkyl darstellt;
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, ist,
wobei Alkyl und Cycloalkyl unsubstituiert oder mit Halogen, C₁₋₆-Alkyl, Halogen-C₁₋₆-alkyl, OR¹ oder CN substituiert sind;
Y abwesend ist oder Wasserstoff, OR⁴, Halogen, C₁₋₆-Alkyl oder Halogen-C₁₋₆-alkyl darstellt; Z -C₂₋₃-Alkylen-, -O-C₁₋₂-Alkylen-, -C₁₋₂-Alkylen-O-, -NR³C(O)-C₀₋₁-Alkylen-, -C(O)NR³-C₀₋₁-Alkylen-, -C₀₋₁-Alkylen-NR³C(O)-, -C₀₋₁-Alkylen-C(O)NR³-, -S(O)ₜ-C₁₋₂-Alkylen-, -C₁₋₂-Alkylen-S(O)ₜ-, -NR⁹-C₁₋₂-Alkylen- oder -C₁₋₂-Alkylen-NR⁹- darstellt,
wobei Alkylen unsubstituiert oder mit 1 bis 4 Substituenten substituiert ist, die unabhängig voneinander aus der Gruppe bestehend aus OR⁴, C₁₋₆-Alkyl, Halogen und Halogen-C₁₋₆-alkyl ausgewählt sind;
R⁹ Wasserstoff, C₁₋₆-Alkyl, Halogen-C₁₋₆-alkyl, C(O)-C₁₋₆-alkyl oder C(O)-Halogen-C₁₋₆-alkyl ist;
R¹ Wasserstoff oder C₁₋₆-Alkyl ist;
R² Halogen, C₁₋₆-Alkyl, C₃₋₆-Cycloalkyl, Halogen-C₁₋₆-alkyl, OR⁴, S(O)₂N(R¹)₂, S(O)₂-C₁₋₆-Alkyl, S(O)₂-C₃₋₆-Cycloalkyl, S(O)₂-Halogen-C₁₋₆-alkyl, C(O)N(R¹)₂, CN, C(O)OR⁴ oder Oxo darstellt,
oder
zwei R² an demselben Kohlenstoffatom zusammen mit dem Kohlenstoffatom, an das sie gebunden sind, eine C₃₋₁₀-Cycloalkylgruppe bilden,
oder
zwei R² an verschiedenen Kohlenstoffatomen zusammen eine -CH₂-, -CH(CH₃)-, -C(CH₃)₂-, CH₂-CH(CH₃)-, -CH₂-CH₂- oder -CH₂-CH₂-CH₂-Gruppe bilden;
R³ Wasserstoff oder C₁₋₆-Alkyl ist;
R⁴ Wasserstoff oder C₁₋₆-Alkyl ist;
m 0, 1 oder 2 ist;
n 0, 1 oder 2 ist;
o 0, 1, 2, 3 oder 4 ist;
p 0, 1, 2 oder 3 ist; und
t 0, 1 oder 2 ist.

2. Verbindung der Formel (1) oder (2) nach Anspruch 1 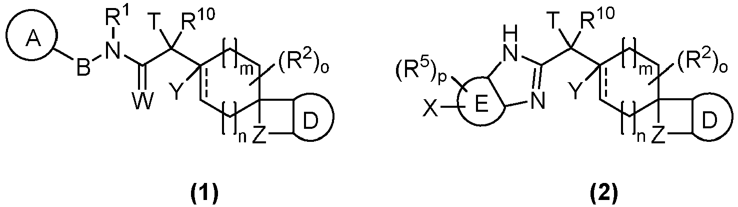 ein Enantiomer, Diastereomer, Tautomer oder pharmazeutisch akzeptables Salz davon wobei
A C₃₋₁₀-Cycloalkyl, das gegebenenfalls mit einem Phenylring kondensiert sein kann, 3- bis 10-gliedriges Heterocycloalkyl, das 1 bis 4 Heteroatome, unabhängig ausgewählt aus O, N und S, enthält, 6- bis 10-gliedriges mono- oder bicyclisches Aryl oder 5- bis 10-gliedriges mono- oder bicyclisches Heteroaryl, das 1 bis 4 Heteroatome, unabhängig ausgewählt aus O, N und S, enthält, darstellt
wobei Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- oder 6-gliedrigem Heteroaryl, das 1 bis 4 Heteroatome enthält, die unabhängig voneinander aus O, N und S ausgewählt sind, und 6-gliedrigem Aryl,
wobei Heteroaryl und Aryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an demselben Kohlenstoffatom oder an zwei verschiedenen Kohlenstoffatomen zusammen mit dem Kohlenstoffatom, an das sie gebunden sind, eine C₃₋₁₀-Cycloalkylgruppe bilden, wobei gegebenenfalls ein Kohlenstoffatom in dem Cycloalkylring durch ein Heteroatom, ausgewählt aus O, N und S, ersetzt sein kann und wobei der (hetero)cyclische Ring unsubstituiert oder durch 1 bis 3 Substituenten, unabhängig ausgewählt aus der Gruppe bestehend aus Halogen, C₁₋₆-Alkyl, Halogen-C₁₋₆-alkyl oder Oxo, substituiert sein kann;
R^{x} C₁₋₆-Alkyl, C₃₋₆-Cycloalkyl oder 3- bis 6-gliedriges Heterocyclyl darstellt, das 1 bis 2 Heteroatome enthält, die unabhängig voneinander aus O, N und S ausgewählt sind,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
B eine Bindung oder C₁₋₂-Alkylen darstellt,
wobei Alkylen unsubstituiert oder mit einem oder zwei C₁₋₄-Alkylgruppen substituiert ist;
D 6- bis 10-gliedriges mono- oder bicyclisches Aryl oder 5- bis 10-gliedriges mono- oder bicyclisches Heteroaryl, das 1 bis 4 Heteroatome enthält, die unabhängig voneinander aus O, N und S ausgewählt sind, darstellt,
wobei Aryl und Heteroaryl unsubstituiert oder mit 1 bis 5 Substituenten substituiert sind, die unabhängig voneinander aus der Gruppe bestehend aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig voneinander aus O, N und S ausgewählt sind, und 6-gliedrigem Aryl ausgewählt sind,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an den Aryl- oder Heteroaryl-Ringsystemen zusammen mit den Kohlenstoffatomen, an die sie gebunden sind, einen 5- oder 6-gliedrigen heterocyclischen Ring bilden, der 1 oder 2 Heteroatome enthält, die unabhängig voneinander aus O, N und S ausgewählt sind,
wobei der heterocyclische Ring unsubstituiert oder mit 1 oder 2 Substituenten substituiert ist, die unabhängig voneinander aus der Gruppe bestehend aus Halogen, C₁₋₆-Alkyl, Halogen-C₁₋₆-alkyl, CN und Oxo ausgewählt sind;
R^{z} C₁₋₆-Alkyl, C₃₋₆-Cycloalkyl oder 3- bis 6-gliedriges Heterocyclyl, das 1 bis 2 Heteroatome enthält, die unabhängig voneinander aus O, N und S ausgewählt sind, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
E ein 6-gliedriges Aryl oder 6-gliedriges Heteroaryl mit 1 bis 2 Stickstoffatomen darstellt;
R⁵ unabhängig ausgewählt ist aus Wasserstoff, Halogen und C₁₋₆-Alkyl;
T Wasserstoff, Halogen, CN, OH, OR^{x}, OC(O)-R^{x}, S-R^{x}, S(O)₂-R^{x}, S(O)₂N(R¹)₂, N(R¹)₂, NR¹C(O)Rₓ, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl mit 1 bis 4 Heteroatomen, die unabhängig voneinander aus O, N und S ausgewählt sind, und 6-gliedrigem Aryl ausgewählt sind;
R¹⁰ Wasserstoff, Halogen, C₁₋₆-Alkyl, Halogen-C₁₋₆-alkyl darstellt; oder
R¹⁰ und T zusammen mit dem Kohlenstoffatom, an das sie gebunden sind, ein 3- bis 10-gliedriges mono- oder bicyclisches Ringsystem bilden, das gesättigt oder teilweise ungesättigt ist und wobei das Ringsystem weiterhin 1, 2 oder 3 Heteroatome enthalten kann, die unabhängig voneinander aus N, O und S ausgewählt sind,
wobei das Ringsystem unsubstituiert oder mit 1 bis 5 Substituenten substituiert ist, die unabhängig voneinander aus der Gruppe bestehend aus Halogen, CN, OH, OC₁₋₆-Alkyl, Oxo, C₁₋₆-Alkyl und Halogen-C₁₋₆-alkyl ausgewählt sind;
W O, NOR⁴, NR¹, NCN oder NS(O)₂-C₁₋₆-Alkyl darstellt;
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, darstellt,
wobei Alkyl und Cycloalkyl unsubstituiert oder mit Halogen, C₁₋₆-Alkyl, Halogen-C₁₋₆-alkyl, OR¹ oder CN substituiert sind;
Y abwesend ist oder Wasserstoff, OR⁴, Halogen, C₁₋₆-Alkyl oder Halogen-C₁₋₆-alkyl darstellt;
Z -C₂₋₃-Alkylen-, -O-C₁₋₂-Alkylen-, -C₁₋₂-Alkylen-O-, -NR³C(O)-C₀₋₁-Alkylen-, -C(O)NR³-C₀₋₁-Alkylen-, -C₀₋₁-Alkylen-NR³C(O)-, -C₀₋₁-Alkylen-C(O)NR³-, -S(O)ₜ-C₁₋₂-Alkylen-, -C₁₋₂-Alkylen-S(O)ₜ-, -NR⁹-C₁₋₂-Alkylen- oder -C₁₋₂-Alkylen-NR⁹- darstellt,
wobei Alkylen unsubstituiert oder mit 1 bis 4 Substituenten substituiert ist, die unabhängig voneinander aus der Gruppe bestehend aus OR⁴, C₁₋₆-Alkyl, Halogen und Halogen-C₁₋₆-alkyl ausgewählt sind;
R⁹ Wasserstoff, C₁₋₆-Alkyl, Halogen-C₁₋₆-alkyl, C(O)-C₁₋₆-alkyl oder C(O)-Halogen-C₁₋₆-alkyl ist;
R¹ Wasserstoff oder C₁₋₆-Alkyl ist;
R² C₁₋₆-Alkyl, C₃₋₆-Cycloalkyl, Halogen-C₁₋₆-alkyl, OR⁴, S(O)₂N(R¹)₂, S(O)₂-C₁₋₆-Alkyl, S(O)₂-C₃₋₆-Cycloalkyl, S(O)₂-Halogen-C₁₋₆-alkyl, C(O)N(R¹)₂, CN, C(O)OR⁴ oder Oxo ist,
oder
zwei R² an demselben Kohlenstoffatom zusammen mit dem Kohlenstoffatom, an das sie gebunden sind, eine C₃₋₁₀-Cycloalkylgruppe bilden,
oder
zwei R² an verschiedenen Kohlenstoffatomen zusammen eine -CH₂-, -CH(CH₃)-, -C(CH₃)₂-, CH₂-CH(CH₃)-, -CH₂-CH₂- oder -CH₂-CH₂-CH₂-Gruppe bilden;
R³ Wasserstoff oder C₁₋₆-Alkyl ist;
R⁴ ist ein Wasserstoffatom oder ein C₁₋₆-Alkylrest;
m ist 0, 1 oder 2;
n ist 0, 1 oder 2;
o 0, 1, 2, 3 oder 4 ist;
p ist 0, 1, 2 oder 3; und
t ist 0, 1 oder 2.

3. Verbindung nach Anspruch 1, die durch die folgenden Formeln (1-1) und (1-2) dargestellt wird 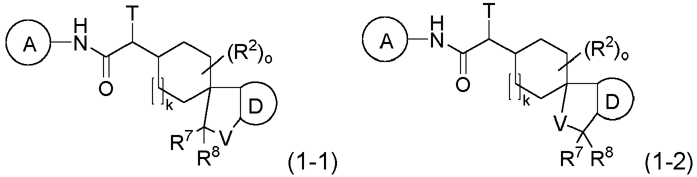 wobei
A C₃₋₁₀-Cycloalkyl, das gegebenenfalls mit einem Phenylring kondensiert sein kann, der unsubstituiert oder mit 1 bis 3 R^{a} substituiert ist, 3- bis 10-gliedriges Heterocycloalkyl, das 1 bis 4 Heteroatome enthält, die unabhängig aus O, N und S ausgewählt sind, 6- bis 10-gliedriges mono- oder bicyclisches Aryl oder 5- bis 10-gliedriges mono- oder bicyclisches Heteroaryl, das 1 bis 4 Heteroatome enthält, die unabhängig aus O, N und S ausgewählt sind, darstellt,
wobei Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, 5- oder 6-gliedrigem Heteroaryl, das 1 bis 4 Heteroatome enthält, die unabhängig voneinander aus O, N und S ausgewählt sind, und 6-gliedrigem Aryl ausgewählt sind,
wobei Heteroaryl und Aryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an demselben Kohlenstoffatom oder an zwei verschiedenen Kohlenstoffatomen zusammen mit dem Kohlenstoffatom, an das sie gebunden sind, eine C₃₋₁₀-Cycloalkylgruppe bilden, wobei gegebenenfalls ein Kohlenstoffatom im Cycloalkylring durch ein Heteroatom, ausgewählt aus O, N und S, ersetzt sein kann;
R^{a} Halogen, CN, C₁₋₆-Alkyl, Halogen-C₁₋₆-alkyl oder Hydroxy-C₁₋₆-alkyl darstellt;
R^{x} C₁₋₆-Alkyl, C₃₋₆-Cycloalkyl oder 3- bis 6-gliedriges Heterocycloalkyl mit 1 bis 2 Heteroatomen, unabhängig ausgewählt aus O, N und S, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
D 6- bis 10-gliedriges mono- oder bicyclisches Aryl oder 5- bis 10-gliedriges mono- oder bicyclisches Heteroaryl, das 1 bis 4 Heteroatome enthält, die unabhängig voneinander aus O, N und S ausgewählt sind, darstellt,
wobei Aryl und Heteroaryl unsubstituiert oder substituiert sind mit 1 bis 5 Substituenten, unabhängig ausgewählt aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedriges Heteroaryl, das 1 bis 4 Heteroatome enthält, die unabhängig aus O, N und S ausgewählt sind, und 6-gliedriges Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an den Aryl- oder Heteroaryl-Ringsystemen zusammen mit dem Kohlenstoffatom, an das sie gebunden sind, einen 5- oder 6-gliedrigen heterocyclischen Ring bilden, der 1 oder 2 Heteroatome enthält, die unabhängig voneinander aus O, N und S ausgewählt sind,
wobei der heterocyclische Ring unsubstituiert oder mit 1 oder 2 Substituenten substituiert ist, die unabhängig voneinander aus der Gruppe bestehend aus Halogen, C₁₋₆-Alkyl, Halogen-C₁₋₆-alkyl, CN und Oxo ausgewählt sind;
R^{z} C₁₋₆-Alkyl, C₃₋₆-Cycloalkyl oder 3- bis 6-gliedriges Heterocyclyl, das 1 bis 2 Heteroatome enthält, die unabhängig voneinander aus O, N und S ausgewählt sind, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
V O oder CR⁷R⁸ ist;
R² C₁₋₆-Alkyl, C₃₋₆-Cycloalkyl, Halogen-C₁₋₆-alkyl, OR⁴, S(O)₂N(R¹)₂, S(O)₂-C₁₋₆-Alkyl, S(O)₂-C₃₋₆-Cycloalkyl, S(O)₂-Halogen-C₁₋₆-alkyl, S(O)₂N(R¹)₂, C(O)N(R¹)₂, CN, C(O)OR⁴ oder Oxo ist,
oder
zwei R² an demselben Kohlenstoffatom zusammen mit dem Kohlenstoffatom, an das sie gebunden sind, eine C₃₋₁₀-Cycloalkylgruppe bilden,
oder
zwei R² an verschiedenen Kohlenstoffatomen zusammen eine -CH₂-, -CH(CH₃)-, -C(CH₃)₂-, CH₂-CH(CH₃)-, -CH₂-CH₂- oder -CH₂-CH₂-CH₂-Gruppe bilden;
R⁷ und R⁸ unabhängig voneinander ausgewählt sind aus der Gruppe bestehend aus Wasserstoff, Halogen, C₁₋₆-Alkyl, Halogen-C₁₋₆-alkyl und OR⁴;
T Wasserstoff, Halogen, CN, OH, ORₓ, OC(O)-R^{x}, S-R^{x}, S(O)₂-R^{x}, S(O)₂N(R¹)₂, N(R¹)₂, NR¹C(O)R^{x}, C(O)N(R₁)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl mit 1 bis 4 Heteroatomen, die unabhängig voneinander aus O, N und S ausgewählt sind, und 6-gliedrigem Aryl ausgewählt sind;
o 0, 1, 2, 3 oder 4 ist; und
k 1 oder 2 ist.

4. Verbindung nach einem der Ansprüche 1 bis 3, die durch die folgenden Formeln (1-3) und (1-4) dargestellt ist 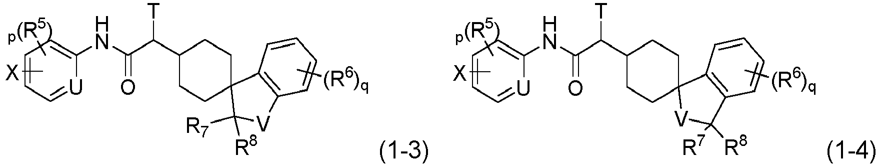 wobei
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, ist,
wobei Alkyl und Cycloalkyl unsubstituiert oder mit Halogen, C₁₋₆-Alkyl, Halogen-C₁₋₆-alkyl, OR¹ oder CN substituiert sind;
R⁵ unabhängig ausgewählt ist aus Wasserstoff, Halogen und C₁₋₆-Alkyl;
R⁶ unabhängig ausgewählt ist aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig ausgewählt sind aus O, N und S, und 6-gliedrigem Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
R^{z} C₁₋₆-Alkyl, C₃₋₆-Cycloalkyl oder 3- bis 6-gliedriges Heterocyclyl mit 1 bis 2 Heteroatomen, unabhängig ausgewählt aus O, N und S, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
R⁷ und R⁸ unabhängig voneinander aus der Gruppe bestehend aus Wasserstoff, Halogen, C₁₋₆-Alkyl, Halogen-C₁₋₆-alkyl und OR⁴ ausgewählt sind;
T Wasserstoff, Halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-Rx, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl, das 1 bis 4 Heteroatome enthält, die unabhängig aus O, N und S ausgewählt sind, und 6-gliedrigem Aryl ausgewählt sind;
U N oder CR⁵ ist;
V O oder CR⁷R⁸ ist;
p 0, 1, 2 oder 3 ist; und
q 0, 1, 2, 3 oder 4 ist.

5. Verbindung nach einem der Ansprüche 1 bis 4, die durch die folgende Formel (1-5) dargestellt wird 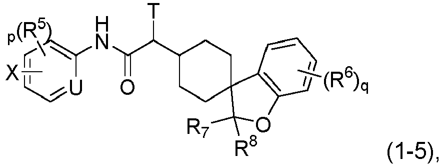 wobei X, U, T, R⁵ bis R⁸, p und q wie in Anspruch 4 definiert sind.

6. Verbindung nach einem der Ansprüche 1 bis 3, die durch die folgende Formel (1-6) dargestellt wird 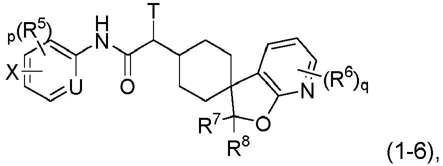 wobei
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, ist,
wobei Alkyl und Cycloalkyl unsubstituiert oder mit Halogen, C₁₋₆-Alkyl, Halogen-C₁₋₆-alkyl, OR¹ oder CN substituiert sind;
R⁵ unabhängig ausgewählt ist aus Wasserstoff, Halogen und C₁₋₆-Alkyl;
R⁶ unabhängig ausgewählt ist aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig ausgewählt sind aus O, N und S, und 6-gliedrigem Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
R^{z} C₁₋₆-Alkyl, C₃₋₆-Cycloalkyl oder 3- bis 6-gliedriges Heterocyclyl mit 1 bis 2 Heteroatomen, unabhängig ausgewählt aus O, N und S, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
R⁷ und R⁸ unabhängig voneinander aus der Gruppe bestehend aus Wasserstoff, Halogen, C₁₋₆-Alkyl, Halogen-C₁₋₆-alkyl und OR⁴ ausgewählt sind;
T Wasserstoff, Halogen, CN, OH, OR^{x}, OC(O)-R^{x}, S-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, - C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl, das 1 bis 4 Heteroatome enthält, die unabhängig aus O, N und S ausgewählt sind, und 6-gliedrigem Aryl ausgewählt sind;
U N oder CR⁵ ist;
p 0, 1, 2 oder 3 ist; und
q 0, 1, 2, 3 oder 4 ist.

7. Verbindung nach Anspruch 1 oder 2, die durch die folgende Formel (1-7) dargestellt wird 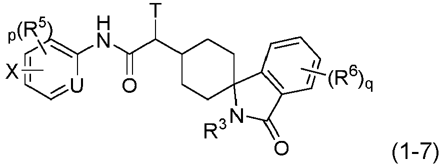 wobei
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl, das 1 bis 4 Heteroatome enthält, die unabhängig aus O, N und S ausgewählt sind, ist,
wobei Alkyl und Cycloalkyl unsubstituiert oder mit Halogen, C₁₋₆-Alkyl, Halogen-C₁₋₆-alkyl, OR¹ oder CN substituiert sind;
R³ Wasserstoff oder C₁₋₆-Alkyl ist;
R⁵ unabhängig ausgewählt ist aus Wasserstoff, Halogen und C₁₋₆-Alkyl;
R⁶ unabhängig ausgewählt ist aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig ausgewählt sind aus O, N und S, und 6-gliedrigem Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
R^{z} C₁₋₆-Alkyl, C₃₋₆-Cycloalkyl oder 3- bis 6-gliedriges Heterocyclyl mit 1 bis 2 Heteroatomen, unabhängig ausgewählt aus O, N und S, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
R⁷ und R⁸ unabhängig voneinander aus der Gruppe bestehend aus Wasserstoff, Halogen, C₁₋₆-Alkyl, Halogen-C₁₋₆-alkyl und OR⁴ ausgewählt sind;
T Wasserstoff, Halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl, das 1 bis 4 Heteroatome enthält, die unabhängig aus O, N und S ausgewählt sind, und 6-gliedrigem Aryl ausgewählt sind;
U N oder CR⁵ ist;
p 0, 1, 2 oder 3 ist; und
q 0, 1, 2, 3 oder 4 ist.

8. Verbindung nach Anspruch 1 oder 2, die durch die folgenden Formeln (2-1) und (2-2) dargestellt wird 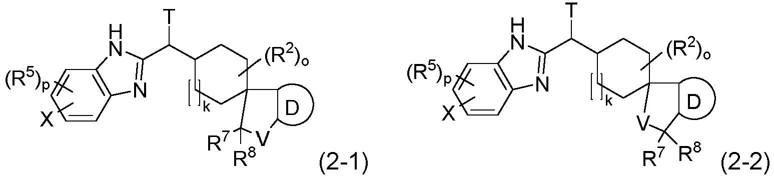 wobei
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, ist,
wobei Alkyl und Cycloalkyl unsubstituiert oder mit Halogen, C₁₋₆-Alkyl, Halogen-C₁₋₆-alkyl, OR¹ oder CN substituiert sind;
R⁵ unabhängig ausgewählt ist aus Wasserstoff, Halogen und C₁₋₆-Alkyl;
T Wasserstoff, Halogen, CN, OH, OR^{x}, OC(O)-R^{x}, S-Rx, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl mit 1 bis 4 Heteroatomen, die unabhängig voneinander aus O, N und S ausgewählt sind, und 6-gliedrigem Aryl ausgewählt sind;
R^{x} C₁₋₆-Alkyl, C₃₋₆-Cycloalkyl oder 3- bis 6-gliedriges Heterocycloalkyl, das 1 bis 2 Heteroatome enthält, die unabhängig voneinander aus O, N und S ausgewählt sind, darstellt, wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
D 6- bis 10-gliedriges mono- oder bicyclisches Aryl oder 5- bis 10-gliedriges mono- oder bicyclisches Heteroaryl, das 1 bis 4 Heteroatome enthält, die unabhängig voneinander aus O, N und S ausgewählt sind, darstellt,
wobei Aryl und Heteroaryl unsubstituiert oder substituiert sind mit 1 bis 5 Substituenten, unabhängig ausgewählt aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig aus O, N und S ausgewählt sind, und 6-gliedrigem Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
oder
zwei Substituenten an den Aryl- oder Heteroaryl-Ringsystemen zusammen mit dem Kohlenstoffatom, an das sie gebunden sind, einen 5- oder 6-gliedrigen heterocyclischen Ring bilden, der 1 oder 2 Heteroatome enthält, die unabhängig voneinander aus O, N und S ausgewählt sind,
wobei der heterocyclische Ring unsubstituiert oder mit 1 oder 2 Substituenten substituiert ist, die unabhängig voneinander aus der Gruppe ausgewählt sind, die aus Halogen, C₁₋₆-Alkyl, Halogen-C₁₋₆-alkyl, CN und Oxo besteht;
R^{z} C₁₋₆-Alkyl, C₃₋₆-Cycloalkyl oder 3- bis 6-gliedriges Heterocyclyl, das 1 bis 2 Heteroatome enthält, die unabhängig voneinander aus O, N und S ausgewählt sind, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
V O oder CR⁷R⁸ ist;
R² C₁₋₆-Alkyl, C₃₋₆-Cycloalkyl, Halogen-C₁₋₆-alkyl, OR⁴, S(O)₂N(R¹)₂, S(O)₂-C₁₋₆-Alkyl, S(O)₂-C₃₋₆-Cycloalkyl, S(O)₂-Halogen-C₁₋₆-alkyl, S(O)₂N(R¹)₂, C(O)N(R¹)₂, CN, C(O)OR⁴ oder Oxo ist, oder
zwei R² an demselben Kohlenstoffatom zusammen mit dem Kohlenstoffatom, an das sie gebunden sind, eine C₃₋₁₀-Cycloalkylgruppe bilden,
oder
zwei R² an verschiedenen Kohlenstoffatomen zusammen eine -CH₂-, -CH(CH₃)-, -C(CH₃)₂-, CH₂-CH(CH₃)-, -CH₂-CH₂- oder -CH₂-CH₂-CH₂-Gruppe bilden;
R⁷ und R⁸ unabhängig voneinander ausgewählt sind aus der Gruppe bestehend aus Wasserstoff, Halogen, C₁₋₆-Alkyl, Halogen-C₁₋₆-alkyl und OR⁴;
o 0, 1, 2, 3 oder 4 ist; und
k 1 oder 2 ist.

9. Verbindung nach einem der Ansprüche 1, 2 und 8, die durch die folgenden Formeln (2-3) und (2-4) dargestellt wird 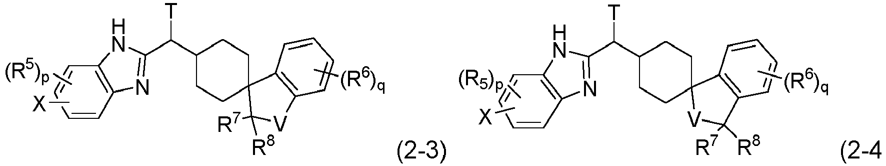 wobei
X Wasserstoff, Halogen, C₁₋₆-Alkyl, C₃₋₆-Cycloalkyl, O-C₁₋₆-Alkyl, S-C₁₋₆-Alkyl, CN oder 5- oder 6-gliedriges Heteroaryl mit 1 bis 4 Heteroatomen, unabhängig ausgewählt aus O, N und S, ist,
wobei Alkyl und Cycloalkyl unsubstituiert oder mit Halogen, C₁₋₆-Alkyl, Halogen-C₁₋₆-alkyl, OR¹ oder CN substituiert sind;
R⁵ unabhängig ausgewählt ist aus Wasserstoff, Halogen und C₁₋₆-Alkyl;
R⁶ unabhängig ausgewählt ist aus Halogen, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, 5- oder 6-gliedrigem Heteroaryl, das 1 bis 4 Heteroatome enthält, die unabhängig ausgewählt sind aus O, N und S, und 6-gliedrigem Aryl,
wobei Aryl und Heteroaryl unsubstituiert oder mit 1 bis 5 Substituenten substituiert sind, die unabhängig voneinander aus der Gruppe bestehend aus Halogen, CN, OR¹, C₁₋₆-Alkyl, C₃₋₆-Cycloalkyl und Halogen-C₁₋₆-alkyl ausgewählt sind,
R^{z} C₁₋₆-Alkyl, C₃₋₆-Cycloalkyl oder 3- bis 6-gliedriges Heterocyclyl mit 1 bis 2 Heteroatomen, unabhängig ausgewählt aus O, N und S, darstellt,
wobei Alkyl, Cycloalkyl und Heterocycloalkyl unsubstituiert oder mit 1 bis 6 Substituenten substituiert sind, die unabhängig voneinander aus der Gruppe bestehend aus Halogen, C₁₋₆-Alkyl, Halogen-C₁₋₆-alkyl, OR¹ und CN ausgewählt sind;
R⁷ und R⁸ unabhängig voneinander aus der Gruppe bestehend aus Wasserstoff, Halogen, C₁₋₆-Alkyl, Halogen-C₁₋₆-alkyl und OR⁴ ausgewählt sind;
T Wasserstoff, Halogen, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, C₁₋₆-Alkyl, Halogen-C₁₋₆-alkyl, C₂₋₆-Alkenyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5- bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander aus der Gruppe bestehend aus Halogen, -C₀₋₆-Alkylen-OH, -C₀₋₆-Alkylen-R^{x}, -C₀₋₆-Alkylen-O-R^{x}, -C₀₋₆-Alkylen-OC(O)-R^{x}, -C₀₋₆-Alkylen-S-R^{x}, -C₀₋₆-Alkylen-S(O)₂-R^{x}, -C₀₋₆-Alkylen-S(O)₂N(R¹)₂, -C₀₋₆-Alkylen-N(R¹)₂, -C₀₋₆-Alkylen-NR¹C(O)R^{x}, -C₀₋₆-Alkylen-C(O)N(R¹)₂, -C₀₋₆-Alkylen-C(O)O-R^{x}, -C₀₋₆-Alkylen-C(O)-R^{x}, -C₀₋₆-Alkylen-CN, -C₀₋₆-Alkylen-COOH, 5- oder 6-gliedrigem Heteroaryl mit 1 bis 4 Heteroatomen, die unabhängig voneinander aus O, N und S ausgewählt sind, und 6-gliedrigem Aryl ausgewählt sind;
V O oder CR⁷R⁸ ist;
p 0, 1, 2 oder 3 ist; und
q 0, 1, 2, 3 oder 4 ist.

10. Verbindung nach einem der Ansprüche 1, 2, 8 und 9, die durch die folgende Formel (2-5) dargestellt wird 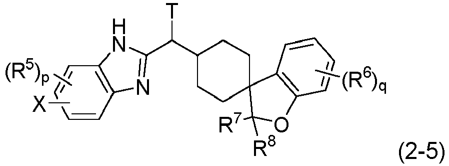 wobei X, T, R⁵ bis R⁸, p und q wie in Anspruch 8 definiert sind.

11. Verbindung nach einem der Ansprüche 1 bis 10, wobei T Wasserstoff, Halogen, CN, C₁₋₆-Alkyl, Halogen-C₁₋₆-alkyl, C₃₋₆-Cycloalkyl, 6-gliedriges Aryl, 5- bis 6-gliedriges Heteroaryl, 3- bis 6-gliedriges Heterocyclyl, 6-gliedriges Aryl-C₁₋₆-alkyl, 5-bis 6-gliedriges Heteroaryl-C₁₋₆-alkyl, 3- bis 6-gliedriges Heterocyclyl-C₁₋₆-alkyl oder 3- bis 6-gliedriges Cycloalkyl-C₁₋₆-alkyl darstellt,
wobei Alkyl, Cycloalkyl, Heterocycloalkyl, Aryl und Heteroaryl unsubstituiert oder mit 1 bis 5 Substituenten substituiert sind, die unabhängig voneinander ausgewählt sind aus der Gruppe bestehend aus Halogen, -C₀₋₆-Alkylen-OH, -C₀₋₆-Alkylen-CN, -C₀₋₆-Alkylen-R^{x} und -C₀₋₆-Alkylen-O-R^{x}.

12. Verbindung nach einem der Ansprüche 1 bis 11, wobei T Wasserstoff, C₁₋₆-Alkyl, Halogen-C₁₋₆-alkyl, C₃₋₆-Cycloalkyl, 3- bis 6-gliedriges Cycloalkyl-C₁₋₆-alkyl darstellt,
wobei Alkyl und Cycloalkyl unsubstituiert oder mit 1 bis 5 Substituenten substituiert sind, die unabhängig voneinander ausgewählt sind aus der Gruppe bestehend aus C₁₋₆-Alkyl, Halogen-C₁₋₆-alkyl, C₃₋₆-Cycloalkyl, Halogen, CN, OH und O-C₁₋₆-Alkyl.

13. Verbindung nach einem der Ansprüche 1 bis 7, 11 und 12, wobei die Verbindung ausgewählt ist aus der Gruppe bestehend aus den folgenden Verbindungen 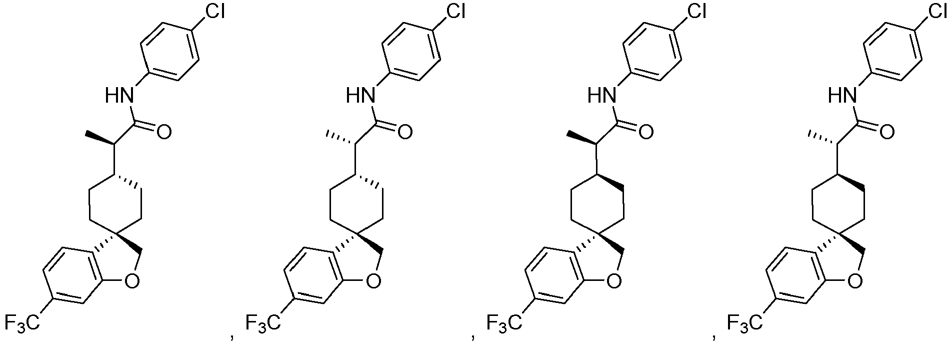 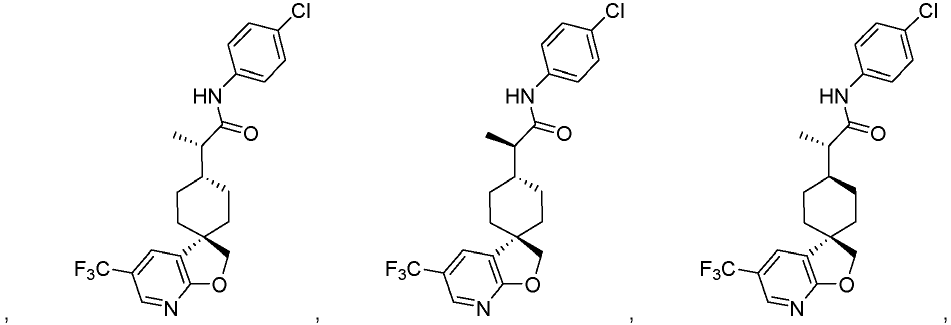 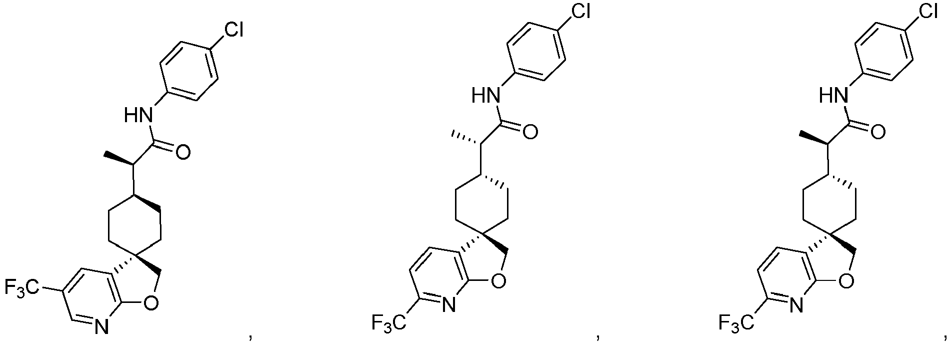 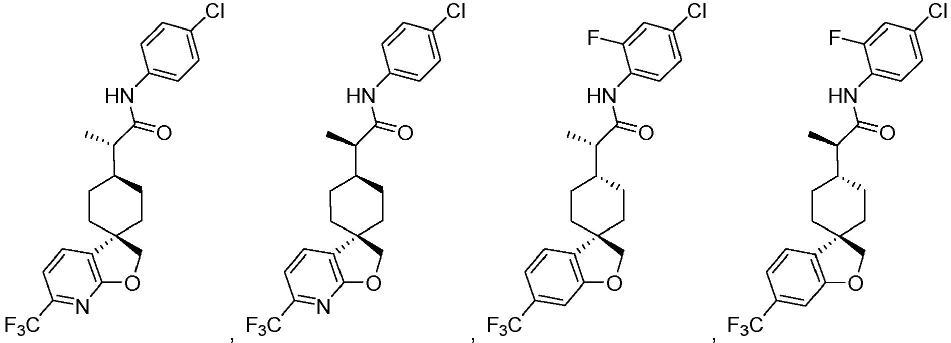 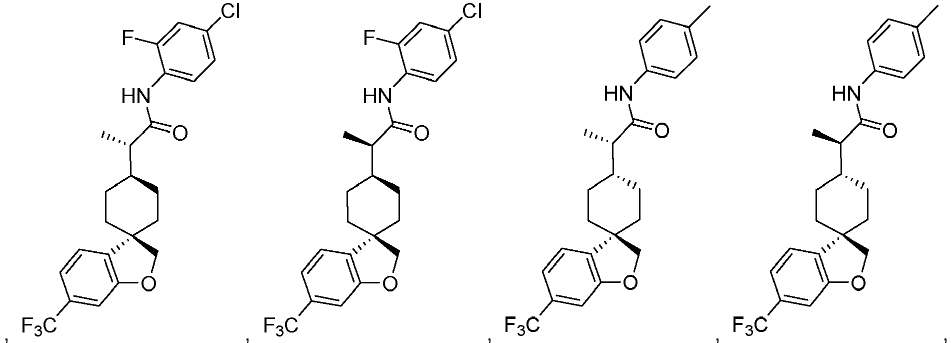 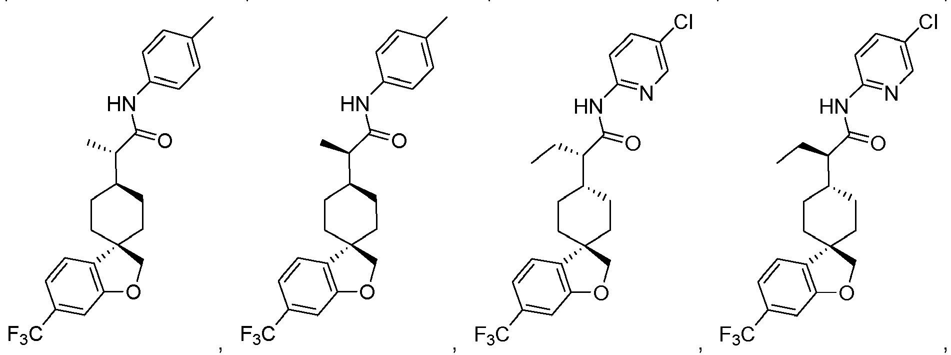 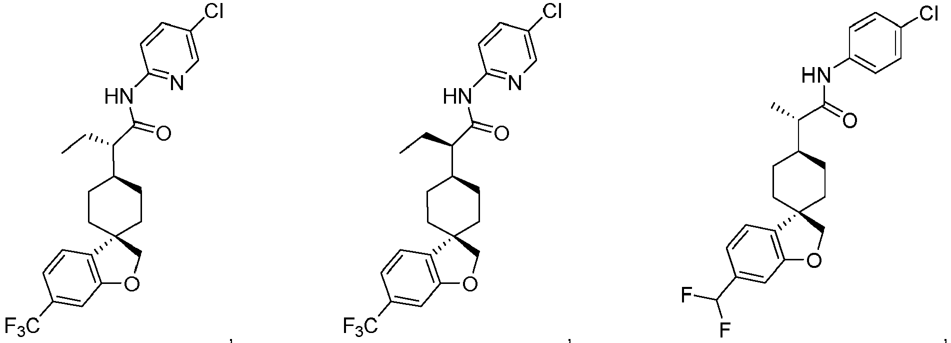 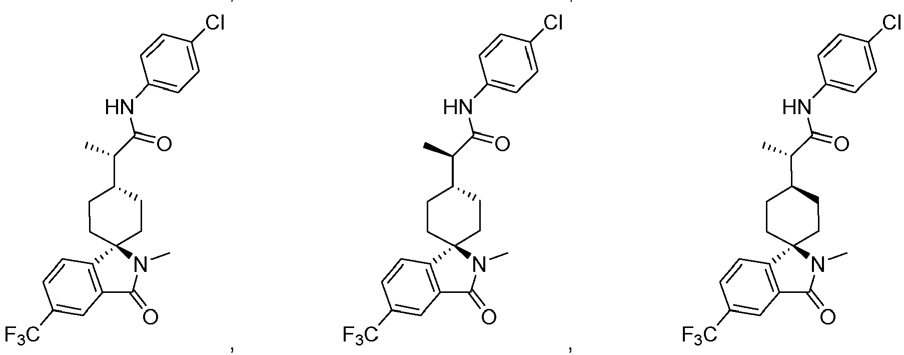 oder 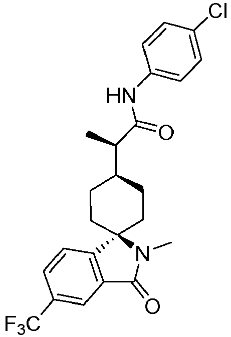 und ihre pharmazeutisch akzeptablen Salze davon.

14. Verbindung nach einem der Ansprüche 1, 2, 8, 9, 10, 11 und 12, wobei die Verbindung ausgewählt ist aus der Gruppe bestehend aus den folgenden Verbindungen 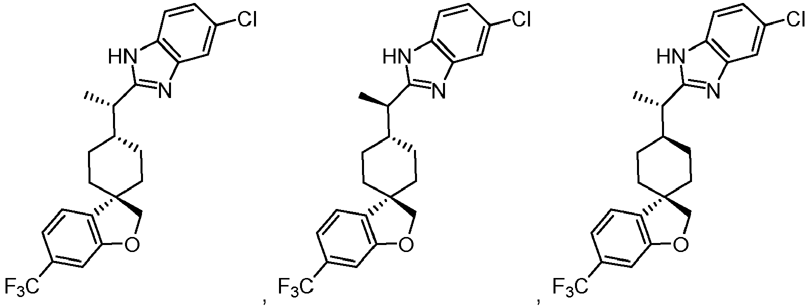 und 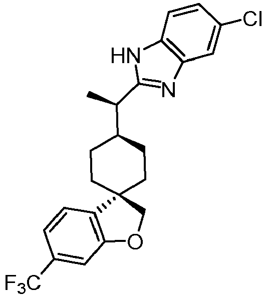 und ihre pharmazeutisch akzeptablen Salze davon.

15. Verbindung nach einem der Ansprüche 1 bis 14 zur Verwendung als Arzneimittel.

16. Verbindung nach einem der Ansprüche 1 bis 14 zur Verwendung bei der Prophylaxe und/oder Behandlung einer Krankheit oder eines Zustands, die/der durch Indolamin-2,3-dioxygenase vermittelt wird, wobei die Krankheit oder der Zustand ausgewählt ist aus der Gruppe bestehend aus Krebs, viralen und bakteriellen Infektionen, Depression, Epilepsie, Schizophrenie, neurodegenerativen Krankheiten, Trauma, altersbedingter grauer Star, Organtransplantation, Herz-Kreislauf-Erkrankungen, Endometriose, Typ-2 diabetische Nephropathie , chronisch obstruktive Lungenerkrankung (COPD), Osteoporose, Asthma, rheumatoide Arthritis, multiple Sklerose, entzündliche Darmerkrankungen, Psoriasis und systemischer Lupus erythematodes.

17. Verbindung zur Verwendung nach Anspruch 16, wobei die virale und bakterielle Infektion ausgewählt ist aus der Gruppe bestehend aus HIV-Infektion, Hantavirus-Infektion, Tuberkulose oder Lepra; oder wobei die neurodegenerative Erkrankung ausgewählt ist aus der Gruppe bestehend aus Parkinson-Krankheit, Alzheimer-Krankheit und Hungtington-Krankheit.

18. Verbindung zur Verwendung nach Anspruch 17, wobei die Krankheit oder der Zustand Krebs ist.

19. Verbindung zur Verwendung nach Anspruch 18, wobei die Verbindung mit einem oder mehreren therapeutischen Wirkstoffen für Krebs verabreicht wird, die aus der Gruppe bestehend aus PD-1-Wirkstoff, PD-L1-Wirkstoff, CTLA-4-Wirkstoff, AhR-Modulator, chemotherapeutischem Wirkstoff, Antikrebs-Impfstoff, onolytischen Viren, TLR-Agonisten, STING-Agonisten und Zytokintherapie sowie weiterer Immuno-Onkologie ausgewählt sind, oder wobei die Verbindung unter Bestrahlungstherapie verabreicht wird.

20. Pharmazeutische Zusammensetzung, umfassend eine Verbindung nach einem der Ansprüche 1 bis 14 und pharmazeutisch annehmbare Hilfsstoffe.

## Revendications

1. Composé selon la Formule (1) ou (2) 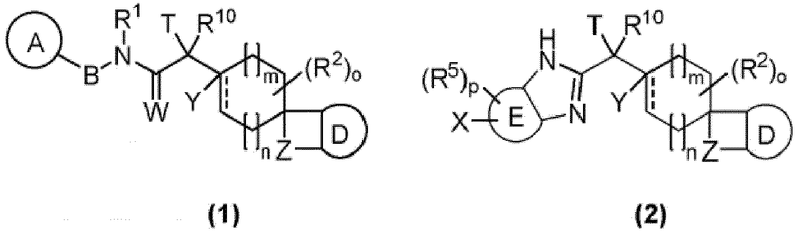
énantiomère, diastéréoisomère, tautomère ou sel pharmaceutiquement acceptable de celui-ci
dans lequel
A représente un cycloalkyle en C₃₋₁₀, qui peut être éventuellement fusionné avec un noyau phényle qui est non substitué, ou substitué par 1 à 3 R^{a}, un hétérocy-cloalkyle de 3 à 10 chaînons contenant 1 à 4 hétéroatomes choisis indépendamment parmi O, N et S, un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
le cycloalkyle, l'hétérocycloalkyle, l'aryle et l'hétéroaryle étant non substitués, ou substitués par 1 à 5 substituants choisis indépendamment dans le groupe constitué par un halogène, OH, R^{x}, O-R^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, CN, COOH, un hétéroaryle à 5 ou 6 chaînons contenant 1 à 4 hétéroatomes choisis indépendamment parmi O, N et S et un aryle à 6 chaînons,
l'hétéroaryle et l'aryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur le même atome de carbone ou sur deux atomes de carbone différents forment ensemble, avec l'atome de carbone auquel ils sont liés, un groupe cycloalkyle en C₃₋₁₀, un atome de carbone dans le noyau cycloalkyle pouvant éventuellement être remplacé par un hétéroa-tome choisi parmi O, N et S et le noyau (hétéro)cyclique pouvant être non substitué ou substitué par 1 à 3 substituants choisis indépendamment dans le groupe constitué par un halogène, un halo-alkyle en C₁₋₆ ou un oxo ;
R^{a} représente un halogène, CN, un alkyle en C₁₋₆, un halo-alkyle en C₁₋₆, un hydroxy-alkyle en C₁₋₆, un cycloalkyle en C₃₋₆ ou un halo-cycloalkyle en C₃₋₆ ;
R^{x} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 ou 6 substituants choisis indépendamment dans le groupe constitué par un halogène, un alkyle en C₁₋₆, un halo-alkyle en C₁₋₆, OR¹ et CN ;
B représente une liaison ou un alkylène en C₁₋₂,
l'alkylène étant non substitué, ou substitué par un ou deux alkyles en C₁₋₄ ;
D représente un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
l'aryle et l'hétéroaryle étant non substitués, ou substitués par 1 à 5 substituants choisis indépendamment dans le groupe constitué par un halogène, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{z}, C(O)-R^{z}, CN, COOH, un hétéroaryle à 5 ou 6 chaînons contenant 1 à 4 hétéroatomes choisis indépendamment parmi O, N et S, et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur les systèmes de noyau aryle ou hétéroaryle forment ensemble, avec les atomes de carbone auxquels ils sont fixés, un noyau hétérocyclique à 5 ou 6 chaînons contenant 1 ou 2 hétéroatomes choisis indépendamment parmi O, N et S,
le noyau hétérocyclique étant non substitué, ou substitué par 1 ou 2 substituants choisis indépendamment dans le groupe constitué par un halogène, un alkyle en C₁₋₆, un halo-alkyle en C₁₋₆, CN et un oxo ;
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN ;
E représente un aryle à 6 chaînons ou un hétéroaryle à 6 chaînons contenant 1 à 2 atomes d'azote ;
R⁵ est choisi indépendamment parmi un hydrogène, un halogène et un alkyle en C₁₋₆ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆-S(O)₂-R^{x}, -alkylène en C₀₋₆-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
R¹⁰ représente un hydrogène, un halogène, un alkyle en C₁₋₆, un halo-alkyle en C₁₋₆ ; ou
R¹⁰ et T forment ensemble, avec l'atome de carbone auquel ils sont liés, un système de noyau mono ou bicyclique de 3 à 10 chaînons qui est saturé ou partiellement insaturé et le système de noyau pouvant contenir, en outre, 1, 2 ou 3 hétéroatomes choisis indépendamment parmi N, O et S,
le système de noyau étant non substitué, ou substitué par 1 à 5 substituants choisis indépendamment dans le groupe constitué par un halogène, CN, OH, un O-alkyle en C₁₋₆, un oxo, un alkyle en C₁₋₆ et un halo-alkyle en C₁₋₆ ;
W représente O, NOR⁴, NR¹, NCN, ou NS(O)₂-alkyle en C₁₋₆ ;
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
Y est absent ou représente un hydrogène, OR⁴, un halogène, un alkyle en C₁₋₆ ou un halo-alkyle en C₁₋₆ ;
Z représente un alkylène en C₂₋₃, -O-alkylène en C₁₋₂, -alkylène en C₁₋₂-O-, - NR³C(O)-alkylène en C₀₋₁, -C(O)NR³-alkylène en C₀₋₁, -alkylène en C₀₋₁-C(O)NR³C(O)-, -alkylène en C₀₋₁ C(O)NR³-, -S(O)ₜ-alkylène en C₁₋₂, -alkylène en C₁₋₂-S(O)ₜ-, -NR⁹-alkylène en C₁₋₂- ou -alkylène en C₁₋₂-NR⁹-,
l'alkylène étant non substitué, ou substitué par 1 à 4 substituants choisis indépendamment dans le groupe constitué par OR⁴, un alkyle en C₁₋₆, un halogène et un halo-alkyle en C₁₋₆ ;
R⁹ est un hydrogène, un alkyle en C₁₋₆, un halo-alkyle en C₁₋₆, un (O)-alkyle en C₁₋₆, ou un C(O)-halo-alkyle en C₁₋₆ ;
R¹ est un hydrogène ou un alkyle en C₁₋₆ ;
R² est un halogène, un alkyle en C₁₋₆, un cycloalkyle en C₃₋₆, un halo-alkyle en C₁₋₆, OR⁴, S(O)₂N(R¹)₂, S(O)₂-alkyle en C₁₋₆, S(O)₂-cycloalkyle en C₃₋₆, S(O)₂-halo-alkyle en C₁₋₆, C(O)N(R¹)₂, CN, C(O)OR ⁴ ou oxo,
ou
deux R² sur le même atome de carbone forment ensemble, avec l'atome de carbone auquel ils sont liés, un groupe cycloalkyle en C₃₋₁₀,
ou
deux R² sur des atomes de carbone différents forment, ensemble, un groupe - CH₂-, -CH(CH₃)-, -C(CH₃)₂-, CH₂-CH(CH₃)-, -CH₂-CH₂- ou -CH₂-CH₂-CH₂- ;
R³ est un hydrogène ou un alkyle en C₁₋₆ ;
R⁴ est un hydrogène ou un alkyle en C₁₋₆ ;
m vaut 0, 1 ou 2 ;
n vaut 0, 1 ou 2 ;
o vaut 0, 1, 2, 3 ou 4 ;
p vaut 0, 1, 2 ou 3 ; et
t vaut 0, 1 ou 2.

2. Composé de Formule (1) ou (2) selon la revendication 1 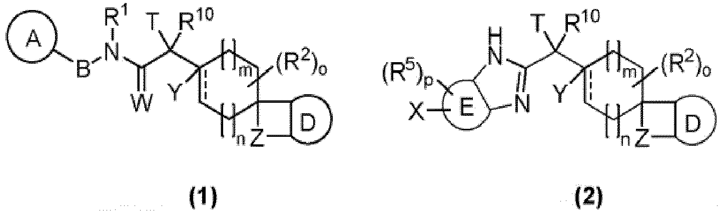
énantiomère, diastéréoisomère, tautomère ou sel pharmaceutiquement acceptable de celui-ci
dans lequel
A représente un cycloalkyle en C₃₋₁₀, qui peut être éventuellement fusionné avec un noyau phényle, un hétéocycloalkyle de 3 à 10 chaînons contenant 1 à 4 hétéroatomes choisis indépendamment parmi O, N et S, un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
le cycloalkyle, l'hétérocycloalkyle, l'aryle et l'hétéroaryle étant non substitués, ou substitués par 1 à 5 substituants choisis indépendamment dans le groupe constitué par un halogène, OH, R^{x}, O-R^{x}, OC(O)-R^{x}, S-R^{x}, S(O)₂-R^{x}, S(O)2N(R¹)₂, N(R¹)₂, NR¹C(O)R^{x}, C(O)N(R¹)₂, C(O)O-R^{x}, C(O)-R^{X}, CN, COOH, un hétéroaryle à 5 ou 6 chaînons contenant 1 à 4 hétéroatomes choisis indépendamment parmi O, N et S et un aryle à 6 chaînons,
l'hétéroaryle et l'aryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur le même atome de carbone ou sur deux atomes de carbone différents forment ensemble, avec l'atome de carbone auquel ils sont fixés, un groupe cycloalkyle en C₃₋₁₀, un atome de carbone dans le noyau cycloalkyle pouvant être éventuellement remplacé par un hétéroatome choisi parmi O, N et S et le noyau (hétéro)cyclique pouvant être non substitué ou substitué par 1 à 3 substituants choisis indépendamment dans le groupe constitué par un halogène, un halo-alkyle en C₁₋₆ ou un oxo ;
R^{x} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 ou 6 substituants choisis indépendamment dans le groupe constitué par un halogène, un alkyle en C₁₋₆, un halo-alkyle en C₁₋₆, OR¹ et CN;
B représente une liaison ou un alkylène en C₁₋₂,
l'alkylène étant non substitué, ou substitué par un ou deux alkyles en C1-4;
D représente un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
l'aryle et l'hétéroaryle étant non substitués, ou substitués par 1 à 5 substituants choisis indépendamment dans le groupe constitué par un halogène, OH, R^{z}, O-R^{z}, OC(O)-R^{z}, S-R^{z}, S(O)₂-R^{z}, S(O)₂N(R¹)₂, N(R¹)₂, NR¹C(O)R^{z}, C(O)N(R¹)₂, C(O)O-R^{Z}, C(O)-R^{Z}, CN, COOH, un hétéroaryle à 5 ou 6 chaînons contenant 1 à 4 hétéroatomes choisis indépendamment parmi O, N et S, et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur les systèmes de noyau aryle ou hétéroaryle forment ensemble, avec les atomes de carbone auxquels ils sont fixés, un noyau hétérocyclique à 5 ou 6 chaînons contenant 1 ou 2 hétéroatomes choisis indépendamment parmi O, N et S,
le noyau hétérocyclique étant non substitué, ou substitué par 1 ou 2 substituants choisis indépendamment dans le groupe constitué par un halogène, un alkyle en C₁₋₆, un halo-alkyle en C₁₋₆, CN et un oxo ;
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
E représente un aryle à 6 chaînons ou un hétéroaryle à 6 chaînons contenant 1 à 2 atomes d'azote ;
R⁵ est choisi indépendamment parmi un hydrogène, un halogène et un alkyle en C₁₋₆ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{X}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
R¹⁰ représente un hydrogène, un halogène, un alkyle en C₁₋₆, un halo-alkyle en C₁₋₆ ; ou
R¹⁰ et T forment ensemble, avec l'atome de carbone auquel ils sont liés, un système de noyau mono ou bicyclique de 3 à 10 chaînons qui est saturé ou partiellement insaturé et le système de noyau pouvant contenir, en outre, 1, 2 ou 3 hétéroatomes choisis indépendamment parmi N, O et S,
le système de noyau étant non substitué, ou substitué par 1 à 5 substituants choisis indépendamment dans le groupe constitué par un halogène, CN, OH, un O-alkyle en C₁₋₆, un oxo, un alkyle en C₁₋₆ et un halo-alkyle en C₁₋₆ ;
W représente O, NOR⁴, NR¹, NCN, ou NS(O)₂-alkyle en C₁₋₆ ;
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
Y est absent ou représente un hydrogène, OR⁴, un halogène, un alkyle en C₁₋₆ ou un halo-alkyle en C₁₋₆ ;
Z représente un alkylène en C₂₋₃, -O-alkylène en C₁₋₂, -alkylène en C₁₋₂-O-, - NR³C(O)-alkylène en C₀₋₁, -C(O)NR³-alkylène en C₀₋₁, -alkylène en C₀₋₁-C(O)NR³C(O)-, -alkylène en C₀₋₁ C(O)NR³-, -S(O)ₜ-alkylène en C₁₋₂, -alkylène en C₁₋₂-S(O)ₜ-, -NR⁹-alkylène en C₁₋₂- ou -alkylène en C₁₋₂-NR⁹-,
l'alkylène étant non substitué, ou substitué par 1 à 4 substituants choisis indépendamment dans le groupe constitué par OR⁴, un alkyle en C₁₋₆, un halogène et un halo-alkyle en C₁₋₆ ;
R⁹ est un hydrogène, un alkyle en C₁₋₆, un halo-alkyle en C₁₋₆, un C(O)-alkyle en C₁₋₆, ou un C(O)-halo-alkyle en C₁₋₆ ;
R¹ est un hydrogène ou un alkyle en C₁₋₆ ;
R² est un alkyle en C₁₋₆, un cycloalkyle en C₃₋₆, un halo-alkyle en C₁₋₆, OR⁴, S(O)₂N(R¹)₂, S(O)₂-alkyle en C₁₋₆, S(O)₂-cycloalkyle en C₃₋₆, S(O)₂-halo-alkyle en C₁₋₆, C(O)N(R¹)₂, CN, C(O)OR ⁴ ou oxo,
ou
deux R² sur le même atome de carbone forment ensemble, avec l'atome de carbone auquel ils sont liés, un groupe cycloalkyle en C₃₋₁₀,
ou
deux R² sur des atomes de carbone différents forment, ensemble, un groupe - CH₂-, -CH(CH₃)-, -C(CH₃)₂-, CH₂-CH(CH₃)-, -CH₂-CH₂- ou -CH₂-CH₂-CH₂- ;
R³ est un hydrogène ou un alkyle en C₁₋₆ ;
R⁴ est un hydrogène ou un alkyle en C₁₋₆ ;
m vaut 0, 1 ou 2 ;
n vaut 0, 1 ou 2 ;
o vaut 0, 1, 2, 3 ou 4 ;
p vaut 0, 1, 2 ou 3 ; et
t vaut 0, 1 ou 2.

3. Composé selon la revendication 1 qui est représenté par les formules (1-1) et (1-2) suivantes 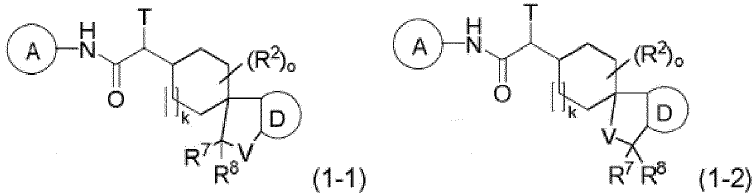 dans lesquelles
A représente un cycloalkyle en C₃₋₁₀, qui peut être éventuellement fusionné avec un noyau phényle qui est non substitué, ou substitué par 1 à 3 R^{a}, un hétérocycloalkyle de 3 à 10 chaînons contenant 1 à 4 hétéroatomes choisis indépendamment parmi O, N et S, un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
le cycloalkyle, l'hétérocycloalkyle, l'aryle et l'hétéroaryle étant non substitués, ou substitués par 1 à 5 substituants choisis indépendamment dans le groupe constitué par un halogène, OH, R^{x}, O-R^{x}, OC(O)-R^{x}, S-R^{x}, S(O)₂-R^{x}, S(O)2N(R¹)₂, N(R¹)₂, NR¹C(O)R^{x}, C(O)N(R¹)₂, C(O)O-R^{x}, C(O)-R^{X}, CN, COOH, un hétéroaryle à 5 ou 6 chaînons contenant 1 à 4 hétéroatomes choisis indépendamment parmi O, N et S et un aryle à 6 chaînons,
l'hétéroaryle et l'aryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur le même atome de carbone ou sur deux atomes de carbone différents forment ensemble, avec l'atome de carbone auquel ils sont liés, un groupe cycloalkyle en C₃₋₁₀, un atome de carbone dans le noyau cycloalkyle pouvant éventuellement être remplacé par un hétéroatome choisi parmi O, N et S ;
R^{a} représente un halogène, CN, un alkyle en C₁₋₆, un halo- alkyle en C₁₋₆, ou un hydroxy- alkyle en C₁₋₆ ;
R^{x} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 ou 6 substituants choisis indépendamment dans le groupe constitué par un halogène, un alkyle en C₁₋₆, un halo-alkyle en C₁₋₆, OR¹ et CN;
D représente un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
l'aryle et l'hétéroaryle étant non substitués, ou substitués par 1 à 5 substituants choisis indépendamment parmi un halogène, OH, R^{z}, O-R^{z}, OC(O)-R^{z}, S-R^{z}, S(O)₂-R^{z}, S(O)₂N(R¹)₂, N(R¹)₂, NR¹C(O)R_{z}, C(O)N(R¹)₂, C(O)OR^{z}, C(O)-R^{Z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur les systèmes de noyau aryle ou hétéroaryle forment ensemble, avec l'atome de carbone auquel ils sont fixés, un noyau hétérocyclique à 5 ou 6 chaînons contenant 1 ou 2 hétéroatomes choisis indépendamment parmi O, N et S,
le noyau hétérocyclique étant non substitué, ou substitué par 1 ou 2 substituants choisis indépendamment dans le groupe constitué par un halogène, un alkyle en C₁₋₆, un halo-alkyle en C₁₋₆, CN et un oxo ;
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
V est O ou CR⁷R⁸ ;
R² est un alkyle en C₁₋₆, un cycloalkyle en C₃₋₆, un halo-alkyle en C₁₋₆, OR⁴, S(O)₂N(R¹)₂, S(O)₂-alkyle en C₁₋₆, S(O)₂-cycloalkyle en C₃₋₆, S(O)₂-halo-alkyle en C₁₋₆, S(O)₂N(R¹)₂, C(O)N(R¹)₂, CN, C(O)OR ⁴ ou oxo,
ou
deux R² sur le même atome de carbone forment ensemble, avec l'atome de carbone auquel ils sont liés, un groupe cycloalkyle en C₃₋₁₀,
ou
deux R² sur des atomes de carbone différents forment, ensemble un groupe -CH₂- , -CH(CH₃)-, -C(CH₃)₂-, CH₂-CH(CH₃)-, -CH₂-CH₂- ou -CH₂-CH₂-CH₂- ;
R⁷ et R⁸ sont choisis indépendamment dans le groupe constitué par un hydrogène, un halogène, un alkyle en C₁₋₆, un halo-alkyle en C₁₋₆ et OR⁴ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
o vaut 0, 1, 2, 3 ou 4 ; et
k vaut 1 ou 2.

4. Composé selon l'une quelconque des revendications 1 à 3 qui est représenté par les formules (1-3) et (1-4) suivantes 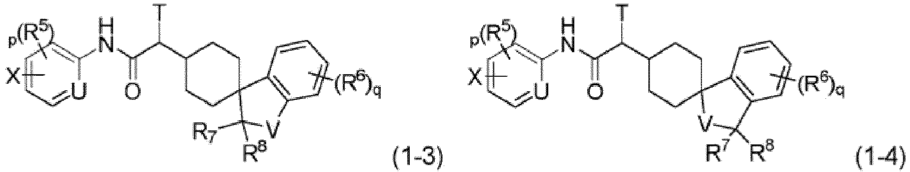 dans lequelles
X est un hydrogène, un halogène, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
R⁵ est choisi indépendamment parmi un hydrogène, un halogène et un alkyle en C₁₋₆ ;
R⁶ est choisi indépendamment parmi un halogène, OH, R^{z}, O-R^{z}, OC(O)-R^{Z}, S-R^{z}, S(O)2-R^{z}, S(O)2N(R¹)₂, N(R¹)₂, NR¹C(O)R^{z}, C(O)N(R¹)₂, C(O)O-R^{Z}, C(O)-R^{z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆, et un halo-alkyle en C₁₋6,
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
R⁷ et R⁸ sont choisis indépendamment dans le groupe constitué par un hydrogène, un halogène, un alkyle en C₁₋₆, un halo-alkyle en C₁₋₆ et OR⁴ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
U est N ou CR⁵ ;
V est O ou CR⁷R⁸ ;
p vaut 0, 1, 2 ou 3 ; et
q vaut 0, 1, 2, 3 ou 4.

5. Composé selon l'une quelconque des revendications 1 à 4 qui est représenté par la formule (1-5) suivante 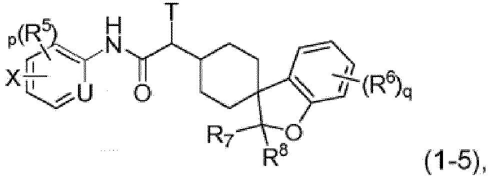 dans laquelle X, U, T, R⁵ à R⁸, p et q sont définis comme dans la revendication 4.

6. Composé selon l'une quelconque des revendications 1 à 3 qui est représenté par la formule (1-6) suivante 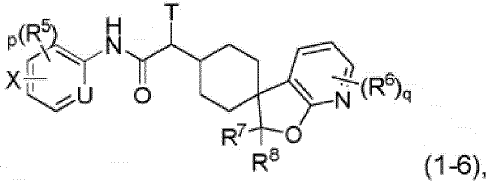 dans laquelle
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
R⁵ est choisi indépendamment parmi un hydrogène, un halogène et un alkyle en C₁₋₆ ;
R⁶ est choisi indépendamment parmi un halogène, OH, R^{z}, O-R^{z}, OC(O)-R^{Z}, S-R^{z}, S(O)2-R^{z}, S(O)2N(R¹)₂, N(R¹)₂, NR¹C(O)R^{z}, C(O)N(R¹)₂, C(O)O-R^{Z}, C(O)-R^{Z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆, et un halo-alkyle en C₁₋₆,
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
R⁷ et R⁸ sont choisis indépendamment dans le groupe constitué par un hydrogène, un halogène, un alkyle en C₁₋₆, un halo-alkyle en C₁₋₆ et OR⁴ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{X}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
U est N ou CR⁵ ;
p vaut 0, 1, 2 ou 3 ; et
q est 0, 1, 2, 3 ou 4.

7. Composé selon la revendication 1 ou 2 qui est representé par la formule (1-7) suivante 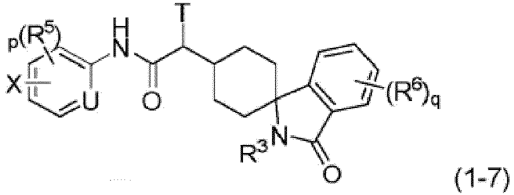 dans laquelle
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
R³ est un hydrogène ou un alkyle en C₁₋₆ ;
R⁵ est choisi indépendamment parmi un hydrogène, un halogène et un alkyle en C₁₋₆ ;
R⁶ est choisi indépendamment parmi un halogène, OH, R^{z}, O-R^{z}, OC(O)-R^{Z}, S-R^{z}, S(O)₂-R^{z}, S(O)2N(R¹)₂, N(R¹)₂, NR¹C(O)R^{z}, C(O)N(R¹)₂, C(O)O-R^{Z}, C(O)-R^{z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
R⁷ et R⁸ sont choisis indépendamment dans le groupe constitué par un hydrogène, un halogène, un alkyle en C₁₋₆, un halo-alkyle en C₁₋₆ et OR⁴ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S et un aryle à 6 chaînons ;
U est N ou CR⁵ ;
p vaut 0, 1, 2 ou 3; et
q vaut 0, 1, 2, 3 ou 4.

8. Composé selon la revendication 1 ou 2 qui est représenté par les formules (2-1) et (2-2) suivantes 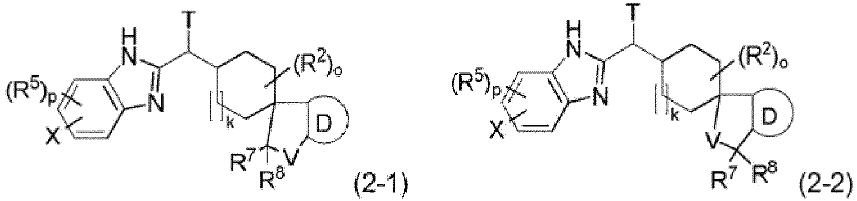 dans lesquelles
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
R⁵ est choisi indépendamment parmi un hydrogène, un halogène et un alkyle en C₁₋₆ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{x}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆,-S(O)₂-R^{x}, -alkylène en C_{0-6,}-S(O)₂N(R¹)₂, - alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S et un aryle à 6 chaînons ;
R^{x} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 ou 6 substituants choisis indépendamment dans le groupe constitué par un halogène, un alkyle en C₁₋₆, un halo-alkyle en C₁₋₆, OR¹ et CN;
D représente un aryle mono ou bicyclique de 6 à 10 chaînons ou un hétéroaryle mono ou bicyclique de 5 à 10 chaînons contenant 1 à 4 hétéroatomes choisis indépendamment parmi O, N et S,
l'aryle et l'hétéroaryle étant non substitués, ou substitués par 1 à 5 substituants choisis indépendamment parmi un halogène, OH, R^{z}, O-R^{z}, OC(O)-R^{z}, S-R^{z}, S(O)₂-R^{z}, S(O)2N(R¹)₂, N(R¹)₂, NR¹C(O)R^{z}, C(O)N(R¹)₂, C(O)OR^{z}, C(O)-R^{Z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆ et un halo-alkyle en C₁₋₆,
ou
deux substituants sur les systèmes de noyau aryle ou hétéroaryle forment ensemble, avec l'atome de carbone auquel ils sont fixés, un noyau hétérocyclique à 5 ou 6 chaînons contenant 1 ou 2 hétéroatomes choisis indépendamment parmi O, N et S,
le noyau hétérocyclique étant non substitué, ou substitué par 1 ou 2 substituants choisis indépendamment dans le groupe constitué par un halogène, un alkyle en C₁₋₆, un halo-alkyle en C₁₋₆, CN et un oxo ;
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
V est O ou CR⁷R⁸ ;
R² est un alkyle en C₁₋₆, un cycloalkyle en C₃₋₆, un halo-alkyle en C₁₋₆, OR⁴, S(O)₂N(R¹)₂, S(O)₂-alkyle en C₁₋₆, S(O)₂-cycloalkyle en C₃₋₆, S(O)₂-halo-alkyle en C₁₋₆, S(O)₂N(R¹)₂, C(O)N(R¹)₂, CN, C(O)OR ⁴ ou un oxo,
ou
deux R² sur le même atome de carbone forment ensemble, avec l'atome de carbone auquel ils sont liés, un groupe cycloalkyle en C₃₋₁₀,
ou
deux R² sur des atomes de carbone différents forment, ensemble, un groupe - CH₂-, -CH(CH₃)-, -C(CH₃)₂-, CH₂-CH(CH₃)-, -CH₂-CH₂- ou -CH₂-CH₂-CH₂- ;
R⁷ et R⁸ sont choisis indépendamment dans le groupe constitué par un hydrogène, un halogène, un alkyle en C₁₋₆, un halo-alkyle en C₁₋₆ et OR⁴ ;
o vaut 0, 1, 2, 3 ou 4 ; et
k vaut 1 ou 2.

9. Composé selon l'une quelconque des revendications 1, 2 et 8, qui est représenté par les formules (2-3) et (2-4) suivantes 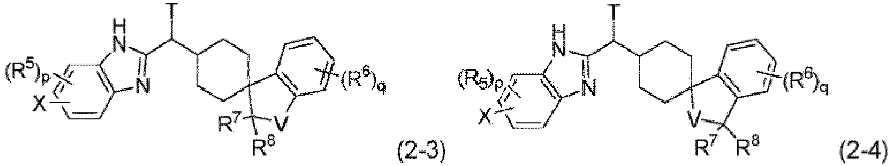 dans lesquelles
X est un hydrogène, un halogène, un alkyle en C₁₋₆, un cycloalkyle en C₃₋₆, un O-alkyle en C₁₋₆, un S-alkyle en C₁₋₆, CN ou un hétéroaryle à 5 ou 6 chaînons contenant 1 à 4 hétéroatomes choisis indépendamment parmi O, N et S,
l'alkyle et le cycloalkyle étant non substitués, ou substitués par un halogène, un alkyle en C₁₋₆, un halo-alkyle en C₁₋₆, OR¹ ou CN ;
R⁵ est choisi indépendamment parmi un hydrogène, un halogène et un alkyle en C₁₋₆ ;
R⁶ est choisi indépendamment parmi un halogène, OH, R^{z}, O-R^{z}, OC(O)-R^{Z}, S-R^{z}, S(O)2-R^{z}, S(O)₂N(R¹)₂, N(R¹)₂, NR¹C(O)R^{z}, C(O)N(R¹)₂, C(O)O-R^{z}, C(O)-R^{Z}, CN, COOH, un hétéroaryle à 5 ou 6 chaînons contenant 1 à 4 hétéroatomes choisis indépendamment parmi O, N et S et un aryle à 6 chaînons,
l'aryle et l'hétéroaryle étant non substitués, ou substitués par 1 à 5 substituants choisis indépendamment dans le groupe constitué par un halogène, CN, OR¹, un alkyle en C₁₋₆, un cycloalkyle en C₃₋₆, et un halo-alkyle en C₁₋6,
R^{z} représente un alkyle en C₁₋₆, un cycloalkyle en C₃₋₆ ou un hétérocyclyle de 3 à 6 chaînons contenant 1 à 2 hétéroatomes choisis indépendamment parmi O, N et S,
l'alkyle, le cycloalkyle et l'hétéocycloalkyle étant non substitués, ou substitués par 1 à 6 substituants choisis indépendamment dans le groupe constitué par un halogène, un alkyle en C₁₋₆, un halo-alkyle en C₁₋₆, OR¹ et CN;
R⁷ et R⁸ sont choisis indépendamment dans le groupe constitué par un hydrogène, un halogène, un alkyle en C₁₋₆, un halo-alkyle en C₁₋₆ et OR⁴ ;
T représente un hydrogène, un halogène, CN, OH, OR^{x}, OC(O)-R^{x}, S-R^{x}, S(O)₂-R^{x}, S(O)₂N(R¹)₂, N(R¹)₂, NR¹C(O)R^{x}, C(O)N(R¹)₂, C(O)O-R^{x}, C(O)-R^{X}, COOH, un alkyle en C₁₋₆, un halo-alkyle en C₁₋₆, un alcényle en C₂₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-R^{x}, -alkylène en C₀₋₆-O-R^{x}, -alkylène en C₀₋₆-OC(O)-R^{x}, -alkylène en C₀₋₆-S-R^{x}, -alkylène en C₀₋₆-S(O)₂-R^{x}, -alkylène en C₀₋₆-S(O)₂N(R¹)₂,-alkylène en C₀₋₆-N(R¹)₂, -alkylène en C₀₋₆-NR¹C(O)R^{x}, -alkylène en C₀₋₆-C(O)N(R¹)₂, -alkylène en C₀₋₆-C(O)O-R^{x}, -alkylène en C₀₋₆-C(O)-R^{x}, -alkylène en C₀₋₆-CN, -alkylène en C₀₋₆-COOH, un hétéroaryle à 5 ou 6 chaînons contenant 1 à 4 hétéroatomes choisis indépendamment parmi O, N et S, et un aryle à 6 chaînons ;
V est O ou CR⁷R⁸ ;
p vaut 0, 1, 2 ou 3 ; et
q vaut 0, 1, 2, 3 ou 4.

10. Composé selon l'une quelconque des revendications 1, 2, 8 et 9, qui est représenté par la formule (2-5) suivante 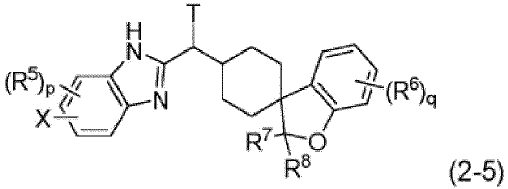 dans laquelle X, T, R⁵ à R⁸, p et q sont définis comme dans la revendication 8.

11. Composé selon l'une quelconque des revendications 1 à 10, dans lequel T représente un hydrogène, un halogène, CN, un alkyle en C₁₋₆, un halo-alkyle en C₁₋₆, un cycloalkyle en C₃₋₆, un aryle à 6 chaînons, un hétéroaryle de 5 à 6 chaînons, un hétérocyclyle de 3 à 6 chaînons, un aryle à 6 chaînons-alkyle en C₁₋₆, un hétéroaryle de 5 à 6 chaînons-alkyle en C₁₋₆, un hétérocyclyle de 3 à 6 chaînons-alkyle en C₁₋₆, ou un cycloalkyle de 3 à 6 chaînons-alkyle en C₁₋₆,
l'alkyle, le cycloalkyle, l'hétérocycloalkyle, l'aryle et l'hétéroaryle étant non substitués, ou substitués par 1 à 5 substituants choisis indépendamment dans le groupe constitué par un halogène, -alkylène en C₀₋₆-OH, -alkylène en C₀₋₆-CN, -alkylène en C₀₋₆-R^{x}, et -alkylène en C₀₋₆-O-R^{x}.

12. Composé selon l'une quelconque des revendications 1 à 11, dans lequel T représente un hydrogène, un alkyle en C₁₋₆, un halo-alkyle en C₁₋₆, un cycloalkyle en C₃₋₆, un cycloalkyle de 3 à 6 chaînons-alkyle en C₁₋₆,
l'alkyle et le cycloalkyle étant non substitués, ou substitués par 1 à 5 substituants choisis indépendamment dans le groupe constitué par un alkyle en C₁₋₆, un halo-alkyle en C₁₋₆, un cycloalkyle en C₃₋₆, un halogène, CN, OH et un O-alkyle en C₁₋₆.

13. Composé selon l'une quelconque des revendications 1 à 7, 11 et 12, le composé étant choisi dans le groupe constitué par les composés suivants 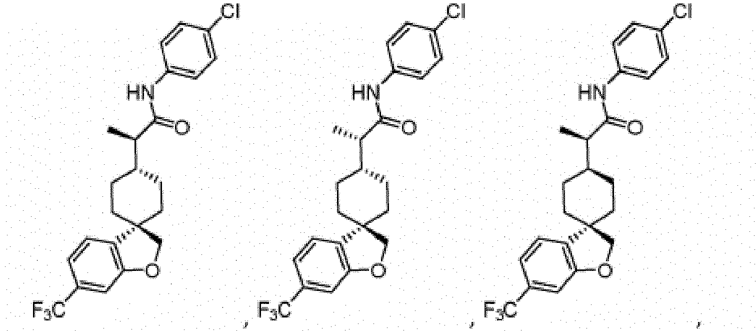 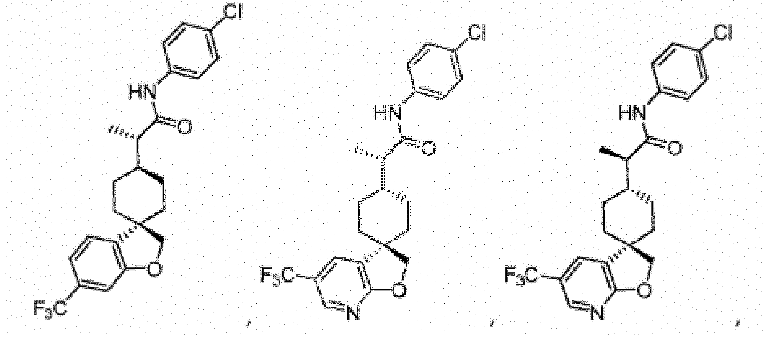 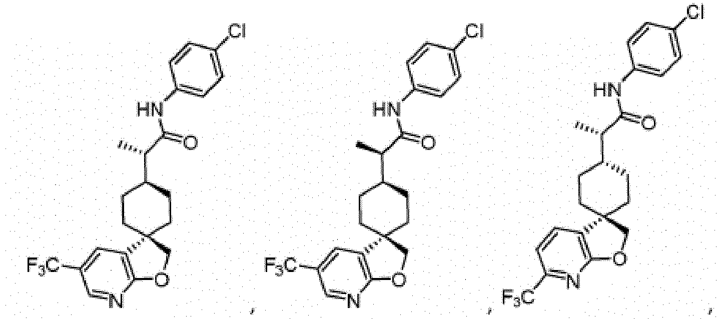 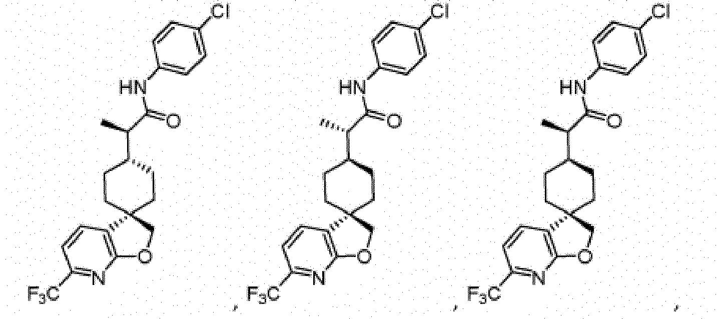 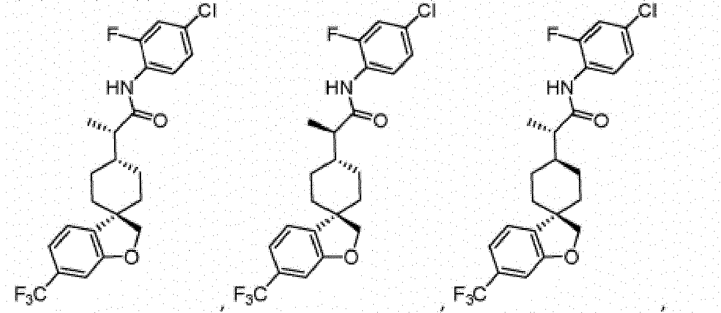 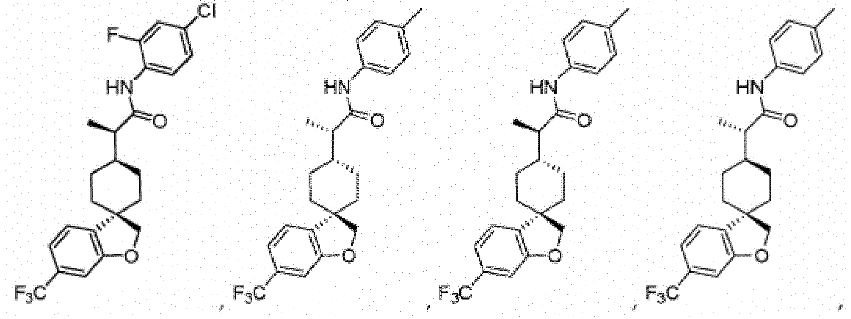 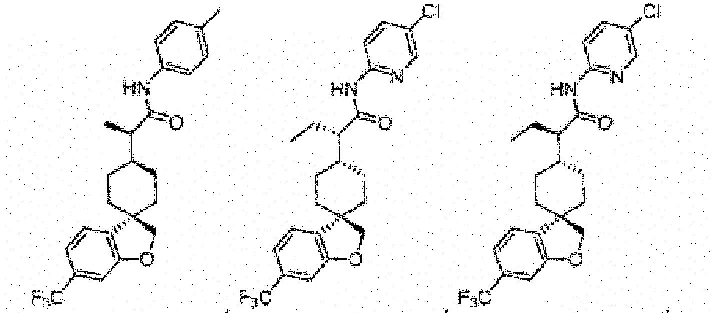 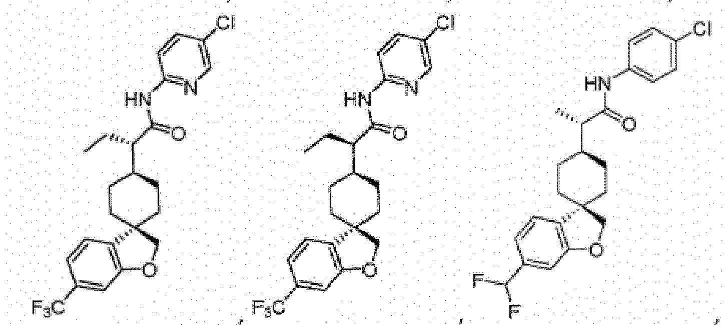 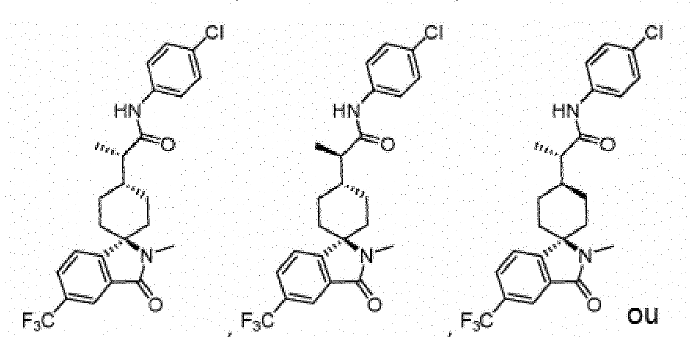 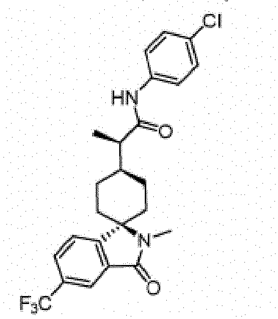 et leurs sels pharmaceutiquement acceptables.

14. Composé selon l'une quelconque des revendications 1, 2, 8, 9, 10, 11 et 12, le composé étant choisi dans le groupe constitué par les composés suivants 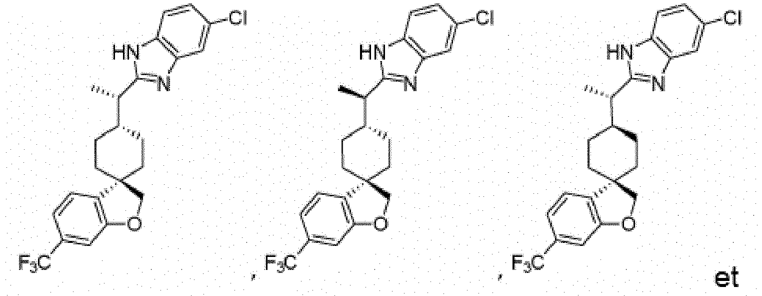 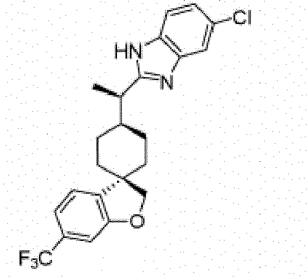 et leurs sels pharmaceutiquement acceptables.

15. Composé selon l'une quelconque des revendications 1 à 14 destiné à être utilisé comme médicament.

16. Composé selon l'une quelconque des revendications 1 à 14 destiné à être utilisé dans la prophylaxie et/ou le traitement d'une maladie ou d'une affection médiée par l'indoléamine 2,3-dioxygénase, la maladie ou l'affection étant choisie dans le groupe constitué par un cancer, des infections virales et bactériennes, une dépression, l'épilepsie, une schizophrénie, des maladies neurodégénératives, un traumatisme, des cataractes liées à l'âge, une greffe d'organe, une maladie cardiovasculaire, l'endométriose, une néphropathie liée au diabète de type 2, une broncopneumopathie chronique obstructive (BPCO), l'ostéoporose, l'asthme, la polyarthrite rhumatoïde, la sclérose en plaques, une maladie inflammatoire de l'intestin, le psoriasis et le lupus érythémateux disséminé.

17. Composé destiné à être utilisé selon la revendication 16, l'infection virale et bactérienne étant choisie dans le groupe constitué par une infection à VIH, une infection à hantavirus, la tuberculose ou la lèpre ; ou la maladie neurodégénérative étant choisie dans le groupe constitué par la maladie de Parkinson, la maladie d'Alzheimer et la maladie de Huntington.

18. Composé destiné à être utilisé selon la revendication 16, la maladie ou l'affection étant un cancer.

19. Composé destiné à être utilisé selon la revendication 18, le composé étant administré avec un ou plusieurs agents thérapeutiques destinés à un cancer choisis dans le groupe constitué par un agent PD-1, un agent PD-L1, un agent CTLA-4, un modulateur d'AhR, un agent chimiothérapeutique, un vaccin anti-cancer, des virus onolytiques, des agonistes de TLR, des agonistes de STING et une thérapie utilisant des cytokines ainsi qu'une autre immuno-oncologie, ou le composé étant administré sous radiothérapie.

20. Composition pharmaceutique comprenant un composé selon l'une quelconque des revendications 1 à 14 et des excipients pharmaceutiquement acceptables.
